# EUROPEAN PATENT APPLICATION

(11) **EP 3 330 266 A1**
(43) Date of publication of application: **06.06.2018**
(21) Application number: 16830600.9
(22) Date of filing: 28.07.2016
(51) Int. Cl.: C07D 405/12, A61K 31/443, A61K 31/4439, A61K 31/444, A61K 31/497, A61K 31/501

(54) **CYCLOPROPANE DERIVATIVE AND DRUG CONTAINING SAME**

(30) Priority: 29.07.2015 JP 2015150058
(71) Applicant: EA PHARMA CO., LTD., Chuo-ku Tokyo 104-0042 (JP)
(72) Inventor: KOBAYASHI, Kaori, Kawasaki-shi Kanagawa 210-8681 (JP); SUZUKI, Tamotsu, Kawasaki-shi Kanagawa 210-8681 (JP); OKUZUMI, Tatsuya, Tokyo 104-8315 (JP)
(74) Representative: Dunne, Paul David
(86) International application number: PCT/JP2016/072232
(87) International publication number: WO 2017/018495

(57) **Abstract**

A compound represented by the formula (I): 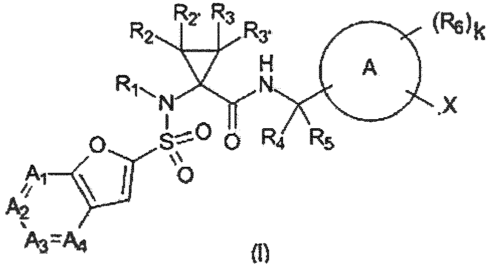 wherein each symbol is as defined in the DESCRIPTION,
or a pharmaceutically acceptable salt thereof has superior TRPA1 antagonist activity, and the compound or a pharmaceutically acceptable salt thereof is useful for the prophylaxis or treatment of diseases involving TRPA1 antagonist and TRPA1.

SEQUENCE LISTING

<110>  EA PHARMA CO., LTD.

<120>  Cyclopropane derivatives and pharmaceutical agents containing them

<130>  092505

<150>  JP 2015-150058
<151>  2015-7-29

<160>  2

<170>  PatentIn version 3.4

<210>  1
<211>  29
<212>  DNA
<213>  Artificial

<220>
<223>  primer 1

<400>  1
aactttagta agcttcgatc gccatgaag                                          29

<210>  2
<211>  34
<212>  DNA
<213>  Artificial

<220>
<223>  primer 2

<400>  2
gtaccgatct agaattcgtt tactaaggct caag                                    34

## Description

### [Technical Field]

The present invention relates to a novel cyclopropane compound having a Transient Receptor Potential Ankyrin 1 (TRPA1) antagonist activity and a pharmaceutical composition containing the compound, as well as a medicament possibly utilizable for the prophylaxis or treatment of a disease involving TRPA1.

### [Background Art]

Transient Receptor Potential Ankyrin 1 (TRPA1) is a non-selective cation channel belonging to the Transient Receptor Potential (TRP) channel superfamily. Like other TRP channel family, it has 6 transmembrane domains and forms a tetramer consisting of 4 subunits. TRPA1 is a ligand dependent ion channel, which changes structure by the binding of ligand. As a result, the channel opens to allow intracellular flow of cations such as calcium ion, sodium ion and the like, thereby controlling the membrane potential of the cells. As the TRPA1 ligand, stimulant natural substances (e.g., allylisothiocyanate (AITC), cinnamaldehyde and the like), environmental stimulants (e.g., formalin, acrolein and the like), endogenous substances (e.g., 4-hydroxynonenal and the like) and the like are known (non-patent documents 1 - 3). It is known that TRPA1 is also activated by cold stimulation, intracellular Ca²⁺ and the like (non-patent document 1). Many ligands such as AITC, cinnamaldehyde and the like form a covalent bond with the cysteine residue and the lysine residue at the N-terminal in the cytoplasm, and activate the channel (non-patent document 2). In addition, intracellular Ca²⁺ is considered to bind to the N-terminal EF hand domain and opens the channel (non-patent document 4). TRPA1 has been reported to be highly expressed in the sensory nerves such as spinal cord nerve, vagus nerve, trigeminal nerve and the like. TRPA1 has been reported to be co-expressed with perception·pain-related markers such as TRPV1, calcitonin gene related peptide (CGRP), substance P and the like (non-patent documents 5 - 7). Therefore, it is considered that, once TRPA1 present in the sensory nerve is activated by various stimulations, channel opening and depolarization of the cellular membrane occur, neuropeptides (CGRP, substance P) are liberated from the nerve ending, and perception such as nociception and the like is transmitted.

In fact, it has been reported that TRPA1 gene knockdown by the gene specific antisense method improves hyperalgesia induced by inflammation and nerve damage in pain model (non-patent document 8). Also, it has been reported that a pain behavior induced by formalin disappears in TRPA1 gene knockout mouse (non-patent document 9). From the above, TRPA1 is considered to play an important role in the nociceptive transmission. There are reports suggesting that TRPA1 is involved in migraine and diabetic neuropathy (non-patent documents 10, 11), and it is expected as a treatment target in pain-associated diseases such as nociceptive pain, neuropathic pain and the like.

Also, TRPA1 is known to show high expression in the afferent sensory nerve projected on the gastrointestinal tract such as esophagus, stomach, large intestine and the like. It has been reported that TRPA1 knockdown decreases nociceptive reaction due to extension of stomach (non-patent document 12), and large intestine hyperalgesia induced by AITC and 2,4,6-trinitrobenzenesulfonic acid (TNBS) is normalized in TRPA1 gene knockout mouse (non-patent document 13). From the above, TRPA1 is suggested to play an important role in the perception· nociception transmission in the gastrointestinal tract, and is expected to be effective for the treatment of digestive tract diseases such as functional dyspepsia, irritable bowel syndrome, erosive esophagitis, inflammatory bowel disease (Crohn's disease, ulcerative colitis), pancreatitis and the like (non-patent document 14).

Furthermore, TRPA1 plays a key role in the detection of a noxious substance in the trachea. It has been reported that TRPA1 gene knockout suppresses inflammation of the trachea in OVA model (non-patent document 15). Therefore, antagonism of TRPA1 is considered to be also useful for pulmonary diseases such as asthma, chronic coughing, chronic obstructive pulmonary disease (COPD) and the like.

As other diseases involving TRPA1, dermatic diseases such as pruritus, allergic dermatitis including atopic dermatitis, burn and the like (non-patent documents 16 - 19), inflammatory diseases such as burn, osteoarthritis and the like (non-patent document 20), bladder diseases such as overactive bladder·abnormal urination·cystitis and the like (non-patent document 21), neurological diseases such as anticancer agent-induced neuropathy and the like (non-patent documents 22 - 24) and the like are known. Thus, a compound capable of functional regulation of TRPA1 is industrially and therapeutically useful in many aspects. In particular, a compound that antagonizes TRPA1 is highly expected as a new therapeutic drug for pain diseases, digestive tract diseases, lung diseases, dermatic diseases, inflammatory diseases, bladder diseases and neurological diseases in human.

As the TRPA1 antagonist, the following compounds described in patent documents 1 - 7 have been reported. 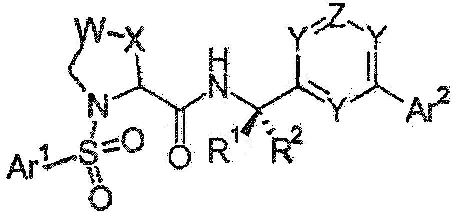 wherein the definition of each symbol is as described in patent document 1 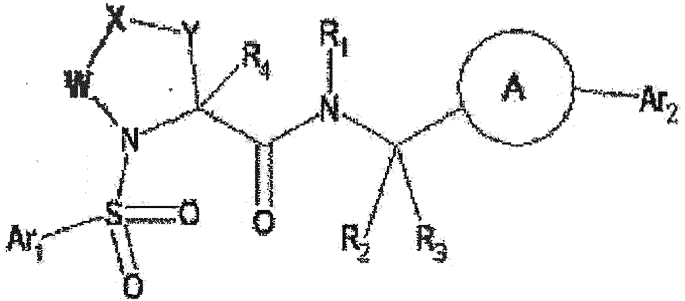 wherein the definition of each symbol is as described in patent document 2 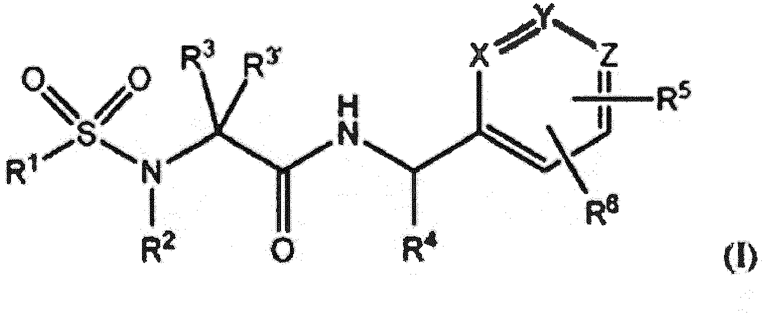 wherein the definition of each symbol is as described in patent document 3 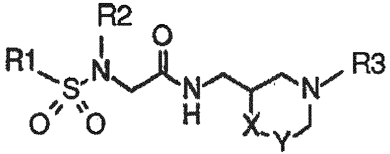 wherein the definition of each symbol is as described in patent document 4 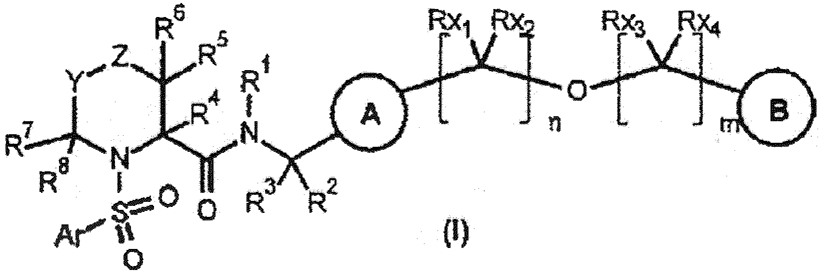 wherein the definition of each symbol is as described in patent document 5 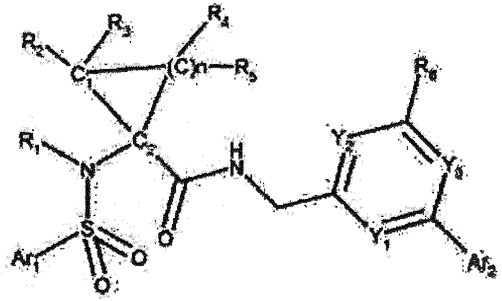 wherein the definition of each symbol is as described in patent document 6 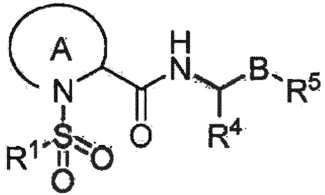 wherein the definition of each symbol is as described in patent document 7

However, these compounds are structurally different from the compound of the present invention represented by the formula (I) described below. To be specific, the compound described in patent document 2 does not have a cyclopropane ring on a carbon atom adjacent to a sulfoneamide bond essential for the compound of the present invention, the compounds described in patent documents 3 and 6 do not have a benzofuran skeleton bonded to a sulfonyl group essential for the compound of the present invention, and the compounds described in patent documents 1, 4, 5 and 7 do not have a cyclopropane ring as well as a benzofuran skeleton. All of them are structurally different from the compound of the present invention.

### [Document List]

### [Patent documents]

patent document 1: WO 2010/141805
patent document 2: WO 2013/108857
patent document 3: WO 2014/049047
patent document 4: WO 2014/076038
patent document 5: WO 2014/098098
patent document 6: WO 2014/135617
patent document 7: WO 2015/052264

### [non-patent document]

non-patent document 1: Bandell M, et al., Neuron. 2004 Mar 25; 41(6): 849-57.
non-patent document 2: Macpherson LJ, et al., Nature. 2007 445(7127):541-5.
non-patent document 3: Trevisani M, et al., Proc Natl Acad Sci U S A. 2007 104(33):13519-24.
non-patent document 4: Zurborg S, et al., Nat Neurosci. 2007 10(3) :277-9.
non-patent document 5: Nagata K, et al., J Neurosci. 2005 25(16):4052-61.
non-patent document 6: Story GM, et al., Cell. 2003 112(6):819-29.
non-patent document 7: Bautista DM, et al., Proc Natl Acad Sci U S A. 2005 102(34):12248-52.
non-patent document 8: Obata K, et al., J Clin Invest. 2005 115(9):2393-401.
non-patent document 9: McNamara CR, et al., Proc Natl Acad Sci U S A. 2007 104(33):13525-30.
non-patent document 10: Benemei S, et al., Br J Pharmacol. 2014 171(10) :2552-67.
non-patent document 11: Wei H, et al., Anesthesiology. 2009 111(1):147-54.
non-patent document 12: Kondo T, et al., Digestion. 2010; 82(3):150-5.
non-patent document 13: Cattaruzza F, et al., Am J Physiol Gastrointest Liver Physiol. 2010 298(1):G81-91.
non-patent document 14: Cattaruzza F, et al., Am J Physiol Gastrointest Liver Physiol. 2013 Jun 1; 304(11):G1002-12.
non-patent document 15: Caceres AI, et al., Proc Natl Acad Sci U S A. 2009 106(22):9099-104.
non-patent document 16: Xiao B, and Patapoutian A., Nat Neurosci. 2011 May; 14(5):540-2.
non-patent document 17: Wilson SR, et al., Nat Neurosci. 2011 May; 14(5):595-602.
non-patent document 18: Oh MH, et al., J Immunol. 2013 Dec 1; 191(11):5371-82.
non-patent document 19: Liu B, et al., FASEB J. 2013 Sep; 27(9):3549-63.
non-patent document 20: McGaraughty S, et al., Mol Pain. 2010 Mar 5; 6:14.
non-patent document 21: Andersson KE, et al., BJU Int. 2010 Oct; 106(8):1114-27.
non-patent document 22: Nassini R, et al., Pain. 2011 Jul; 152(7):1621-31.
non-patent document 23: Materazzi S, et al., Pflugers Arch. 2012 Apr; 463(4):561-9.
non-patent document 24: Trevisan G, et al., Cancer Res. 2013 May 15; 73(10):3120-31.

### [SUMMARY OF THE INVENTION]

### [Problems to be Solved by the Invention]

The present invention aims to provide a novel compound having transient receptor potential ankyrin 1 (TRPA1) antagonist activity.

The present invention also aims to provide a TRPA1 antagonist.

The present invention also aims to provide a medicament containing the above-mentioned novel compound.

The present invention also aims to provide a medicament useful for the prophylaxis or treatment of diseases involving TRPA1.

### [Means of Solving the Problems]

In view of the aforementioned situation, the present inventors have conducted various studies and found that particular cyclopropane compounds have a strong TRPA1 antagonist activity and that those compounds having TRPA1 antagonist activity are useful for the prophylaxis and/or treatment of diseases involving TRPA1 (e.g., pain associated diseases, digestive tract diseases, lung diseases, bladder diseases, inflammatory diseases, dermatic diseases, and neurological diseases), and completed the present invention.

That is, the present invention provides the following.
[1] A compound represented by the formula (I): 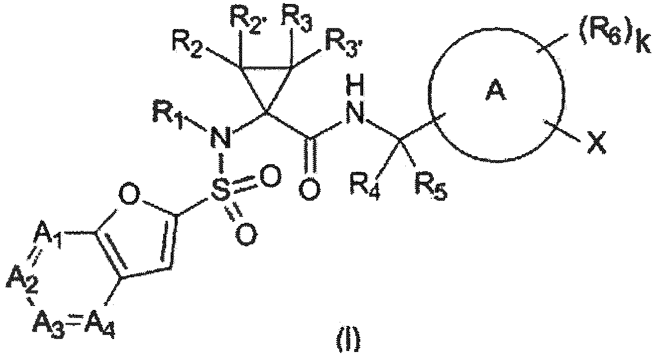 wherein
   ring A is a 5-membered or 6-membered monocyclic aromatic ring or heteroaromatic ring, or bicyclic aromatic ring or heteroaromatic ring;
   A₁ is -C(Ra)= or -N=;
   A₂ is -C(Rb)= or -N=;
   A₃ is -C(Rc)= or -N=;
   A₄ is -C(Rd)= or -N=;
   Ra, Rb, Rc and Rd are the same or different and each is hydrogen, a halogeno group, a cyano group, a hydroxy group, a C₁₋₆ alkyl group, a C₁₋₆ alkoxy group, a halogeno C₁₋₆ alkyl group or a halogeno C₁₋₆ alkoxy group;
   at least two of A₁ - A₄ are not -N=;
   R₁ is hydrogen or a C₁₋₆ alkyl group optionally having substituent(s);
   R₂, R₂', R₃ and R₃' are the same or different and each is hydrogen or a C₁₋₆ alkyl group optionally having substituent(s);
   R₄ is hydrogen or a C₁₋₆ alkyl group;
   R₅ is hydrogen or a C₁₋₆ alkyl group;
   R₄ and R₅ are optionally joined to form cycloalkane;
   X is hydrogen,
   -Cy,
   -C(Rₓ₁Rₓ₂)-Cy,
   -C(Rₓ₁Rₓ₂)-C(Rₓ₃Rₓ₄)-Cy,
   -C(Rₓ₁)=C(Rₓ₂)-Cy,
   -O-Cy,
   -O-C(Rₓ₁Rₓ₂)-Cy,
   -C(Rₓ₁Rₓ₂)-O-Cy,
   -S(O)n-Cy,
   -S(O)n-C(Rₓ₁Rₓ₂)-Cy,
   -C(Rₓ₁Rₓ₂)-S(O)n-Cy,
   -N(Rₓ₅)-Cy,
   -N(Rₓ₅)-C(Rₓ₁Rₓ₂)-Cy,
   -C(Rₓ₁Rₓ₂)-N(Rₓ₅)-Cy,
   -N(Rₓ₅)-N(Rₓ₆)-Cy,
   -O-N(Rₓ₅)-Cy,
   -N(Rₓ₅)-O-Cy,
   -C(O)-N(Rₓ₅)-Cy,
   -N(Rₓ₅)-C(O)-Cy,
   -S(O)m-N(Rₓ₅)-Cy,
   -N(Rₓ₅)-S(O)m-Cy,
   -O-S(O)m-Cy, or
   -S(O)m-O-Cy;
   n is an integer of 0 - 2;
   m is 1 or 2;
   Cy is a saturated or unsaturated cyclic group optionally having substituent(s) (optionally including heteroatom(s));
   Rₓ₁, Rₓ₂, Rₓ₃, Rₓ₄, Rₓ₅ and Rₓ₆ are the same or different and each is hydrogen, a C₁₋₆ alkyl group optionally having substituent(s) or a C₁₋₆ alkoxycarbonyl group optionally having substituent(s);
   R₆ is a C₁₋₆ alkyl group optionally having substituent (s), a C₂₋₆ alkenyl group, a cyclic C₃₋₆ alkyl group (optionally containing heteroatom(s)), a halogeno group, a hydroxy group, a C₁₋₆ alkoxy group optionally having substituent(s), a halogeno C₁₋₆ alkyl group, a halogeno C₁₋₆ alkoxy group, amino group, an amino group mono- or di-substituted by a C₁₋₆ alkyl group optionally having substituent(s), a cyano group, a C₁₋₆ alkylthio group, a carboxyl group, a C₁₋₆ alkoxycarbonyl group optionally having substituent(s), a carbamoyl group, a carbamoyl group mono- or di-substituted by a C₁₋₆ alkyl group optionally having substituent(s) or an amino group substituted by an acyl group optionally having substituent(s);
   when R₆ is present in plurality, they may be the same or different; and
   k is an integer of 0 - 3, or a pharmaceutically acceptable salt thereof.
[2] The compound or pharmaceutically acceptable salt of the above-mentioned [1], wherein ring A is a 6-membered monocyclic aromatic ring or heteroaromatic ring, or bicyclic aromatic ring or heteroaromatic ring.
[3] The compound or pharmaceutically acceptable salt of the above-mentioned [1] or [2], wherein R₁ is a C₁₋₆ alkyl group optionally having substituent(s).
[4] The compound or pharmaceutically acceptable salt of the above-mentioned [1] or [2], wherein R₁ is hydrogen.
[5] The compound or pharmaceutically acceptable salt of any of the above-mentioned [1] to [4], wherein R₂, R₂', R₃, and R₃' are each hydrogen.
[6] The compound or pharmaceutically acceptable salt of any of the above-mentioned [1] to [5], wherein R₄ and R₅ are each hydrogen.
[7] The compound or pharmaceutically acceptable salt of any of the above-mentioned [1] to [6], wherein ring A is a 6-membered monocyclic aromatic ring or heteroaromatic ring.
[8] The compound or pharmaceutically acceptable salt of any of the above-mentioned [1] to [7], wherein ring A is benzene, pyridine or pyrimidine.
[9] The compound or pharmaceutically acceptable salt of any of the above-mentioned [1] to [8], wherein partial structure (b) containing ring A 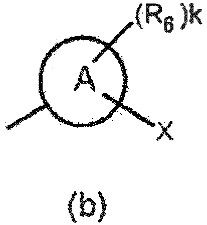 is a group of any of the following illustrations 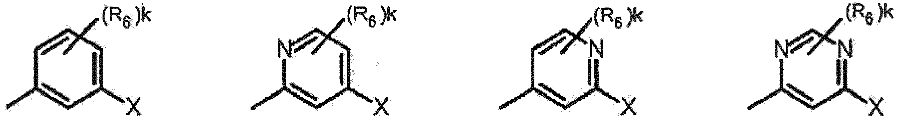 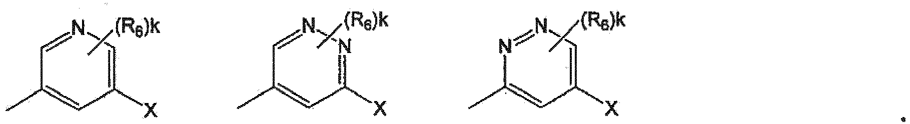
[10] The compound or pharmaceutically acceptable salt of any of the above-mentioned [1] to [9], wherein k is an integer of 0 to 2, and R₆ is a C₁₋₆ alkyl group, a cyclic C₃₋₆ alkyl group (optionally containing heteroatom(s)), a halogeno group, a hydroxy group, a C₁₋₆ alkoxy group optionally having substituent(s), an amino group, a C₁₋₆ alkoxycarbonyl group, or an amino group mono- or di-substituted by a C₁₋₆ alkyl group.
[11] The compound or pharmaceutically acceptable salt of any of the above-mentioned [1] to [9], wherein k is 0.
[12] The compound or pharmaceutically acceptable salt of any of the above-mentioned [1] to [9], wherein partial structure (b) containing ring A 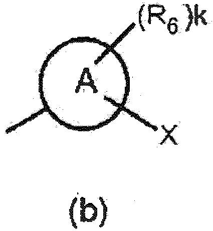 is a group of any of the following illustrations 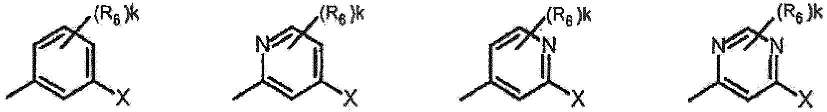 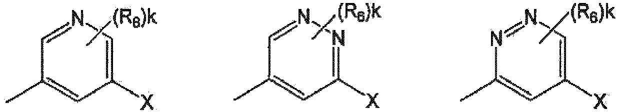 k is 0 or 1, R₆ is a cyclic C₃₋₆ alkyl group (optionally containing heteroatom(s)), a halogeno group, a C₁₋₆ alkoxycarbonyl group, an amino group, an amino group mono- or di-substituted by a C₁₋₆ alkyl group or a hydroxy group.
[13] The compound or pharmaceutically acceptable salt of any of the above-mentioned [1] to [12], wherein partial structure (b) containing ring A 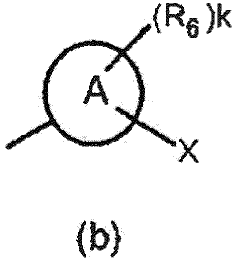 is a group of any of the following illustrations 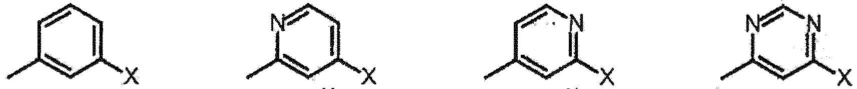 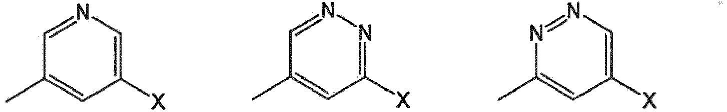 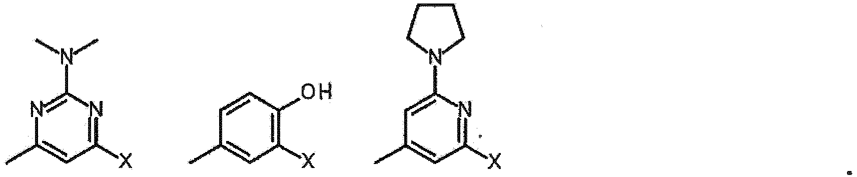
[14] The compound or pharmaceutically acceptable salt of any of the above-mentioned [1] to [13], wherein A₁ is -C(Ra)=, A₂ is - C(Rb)=, A₃ is -C(Rc)=, and A₄ is -C(Rd)=.
[15] The compound or pharmaceutically acceptable salt of any of the above-mentioned [1] to [14], wherein A₁ is -C(Ra)=, A₂ is - C(Rb)=, A₃ is -C(Rc)=, and A₄ is -C(Rd)=;
   Ra, Rb, Rc, and Rd are all hydrogen or any one of them is a halogeno group.
[16] The compound or pharmaceutically acceptable salt of any of the above-mentioned [1] to [15], wherein partial structure (a) 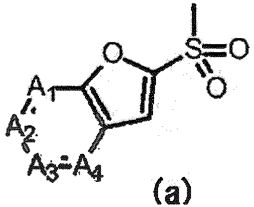 is a group of any of the following illustrations 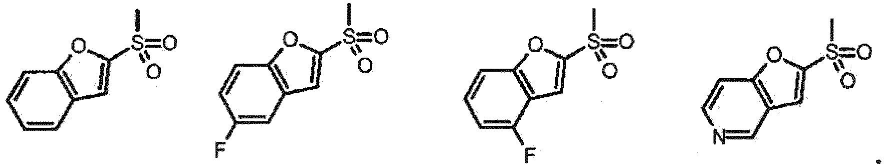
[17] The compound or pharmaceutically acceptable salt of any of the above-mentioned [1] to [16], wherein X is hydrogen, -Cy, - O-Cy or -O-CH₂-Cy.
[18] The compound or pharmaceutically acceptable salt of any of the above-mentioned [1] to [17], wherein X is -Cy.
[19] The compound or pharmaceutically acceptable salt of any of the above-mentioned [1] to [18], wherein Cy is benzene optionally having substituent(s), pyridine optionally having substituent(s), pyrimidine optionally having substituent(s), pyridazine optionally having substituent(s), or pyrazine optionally having substituent(s).
[20] The compound or pharmaceutically acceptable salt of any of the above-mentioned [1] to [19], wherein Cy is a group of any of the following illustrations 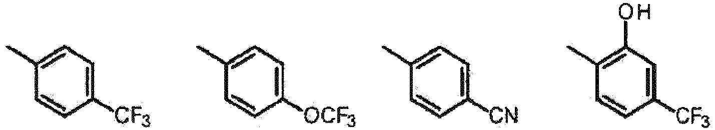 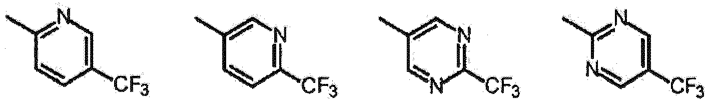 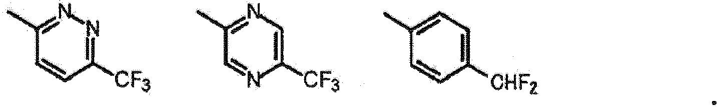
[21] The compound or pharmaceutically acceptable salt of any of the above-mentioned [1] to [20], which is a TRPA1 antagonist.
[22] A medicament comprising a compound or pharmaceutically acceptable salt of any of the above-mentioned [1] to [21] as an active ingredient.
[23] The medicament of the above-mentioned [22] for the prophylaxis and/or treatment of a disease involving TRPA1.
[24] The medicament of the above-mentioned [23], wherein the disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.
[25] The medicament of the above-mentioned [23], wherein the disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.
[26] A method for the prophylaxis and/or treatment of a disease involving TRPA1, the method comprising administering an effective amount of a compound or pharmaceutically acceptable salt of any of the above-mentioned [1] to [20] to a subject in need thereof.
[27] The method of the above-mentioned [26], wherein the disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.
[28] The method of the above-mentioned [26], wherein the disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.
[29] The compound or pharmaceutically acceptable salt of any of the above-mentioned [1] to [20] for use in the prophylaxis and/or treatment of a disease involving TRPA1.
[30] The compound or pharmaceutically acceptable salt of the above-mentioned [29], wherein the disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.
[31] The compound or pharmaceutically acceptable salt of the above-mentioned [29], wherein the disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.

As still other preferable embodiments of compound (I), the compounds described in the below-mentioned Examples or a pharmaceutically acceptable salt thereof can be mentioned.

More preferably, the compounds of Examples 1, 3, 4, 8, 11, 12, 14, 18, 19, 20, 21, 24, 25, 26, 27, 29 described in the following Table (Table 1) or a pharmaceutically acceptable salt thereof.

**[Table 1-1]**

| **Ex. No.** | **structural formula** | **compound name** |
|---|---|---|
| 1 | 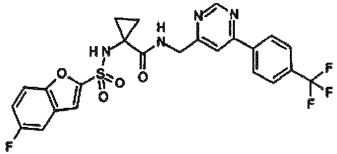 | 1-[(5-fluorobenzofuran-2-yl)sulfonylamino]-N-[[6-[4-(trifluoromethyl)phenyl]pyrimidin-4-yl]methyl]cyclopropanecarboxamide |
| 3 | 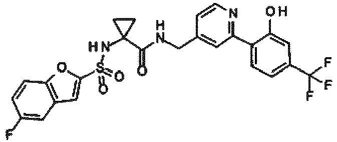 | 1-[(5-fluorobenzofuran-2-yl)sulfonylamino]-N-[[2-[2-hydroxy-4-(trifluoromethyl)phenyl]-4-pyridyl]methyl]cyclopropanecarboxamide |
| 4 | 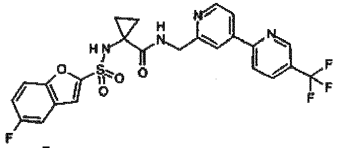 | 1-[(5-fluorobenzofuran-2-yl)sulfonylamino]-N-[[4-[5-(trifluoromethyl)-2-pyridyl]-2-pyridyl]methyl]cyclopropanecarboxamide |
| 8 | 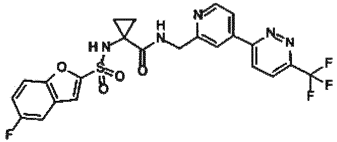 | 1-[(5-fluorobenzofuran-2-yl)sulfonylamino]-N-[[4-[6-(trifluoromethyl)pyridazin-3-yl]-2-pyridyl]methyl]cyclopropanecarboxamide |
| 11 | 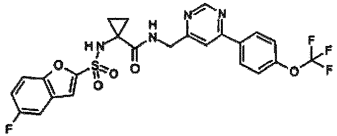 | 1-[(5-fluorobenzofuran-2-yl)sulfonylamino]-N-[[6-[4-(trifluoromethoxy)phenyl]pyrimidin-4-yl]methyl]cyclopropanecarboxamide |
| 12 | 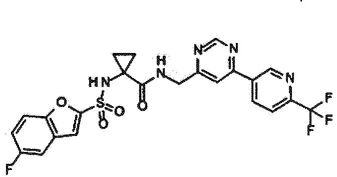 | 1-[(5-fluorobenzofuran-2-yl)sulfonylamino]-N-[[6-[6-(trifluoromethyl)-3-pyridyl]pyrimidin-4-yl]methyl]cyclopropanecarboxamide |
| 14 | 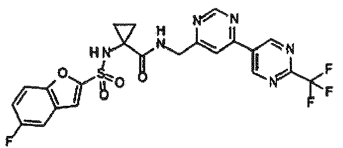 | 1-[(5-fluorobenzofuran-2-yl)sulfonylamino]-N-[[6-[2-(trifluoromethyl)pyrimidin-5-yl]pyrimidin-4-yl]methyl]cyclopropanecarboxamide |
| 18 | 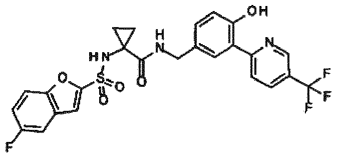 | 1-[(5-fluorobenzofuran-2-yl)sulfonylamino]-N-[[4-hydroxy-3-[5-(trifluoromethyl)-2-pyridyl]-phenyl]methyl]cyclopropanecarboxamide |

**[Table 1-2]**

| **Ex. No.** | **structural formula** | **compound name** |
|---|---|---|
| 19 | 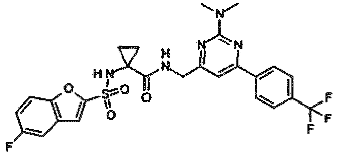 | N-[[2-(dimethylamino)-6-[4-(trifluoromethyl)phenyl]pyrimidin-4-yl]methyl]-1-[(5-fluorobenzofuran-2-yl)sulfonylamino]cyclopropanecarboxamide |
| 20 | 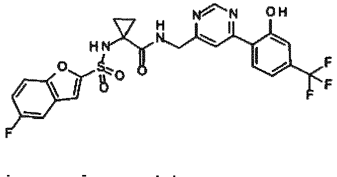 | 1-[(5-fluorobenzofuran-2-yl)sulfonylamino]-N-[[6-[2-hydroxy-4-(trifluoromethyl)-phenyl]pyrimidin-4-yl]methyl]cyclopropanecarboxamide |
| 21 | 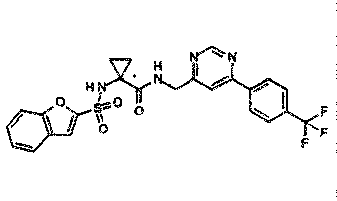 | 1-(benzofuran-2-ylsulfonylamino)-N-[[6-[4-(trifluoromethyl)-phenyl]pyrimidin-4-yl]methyl]cyclopropanecarboxamide |
| 24 | 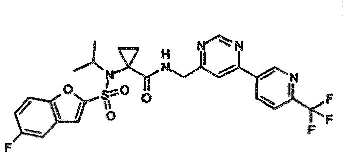 | 1-[(5-fluorobenzofuran-2-yl)sulfonyl-isopropyl-amino]-N-[[6-[6-(trifluoromethyl)-3-pyridyl]pyrimidin-4-yl]methyl]cyclopropanecarboxamide |
| 25 | 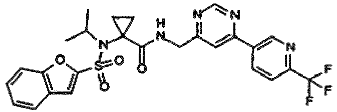 | 1-[benzofuran-2-ylsulfonyl(isopropyl)amino]-N-[[6-[6-(trifluoromethyl)-3-pyridyl]pyrimidin-4-yl]methyl]cyclopropanecarboxamide |
| 26 | 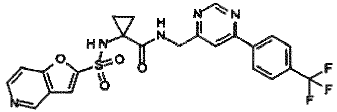 | 1-(furo[3,2-c]pyridin-2-ylsulfonylamino)-N-[[6-[4-(trifluoromethyl)phenyl]pyrimidin-4-yl]methyl]cyclopropanecarboxamide |
| 27 | 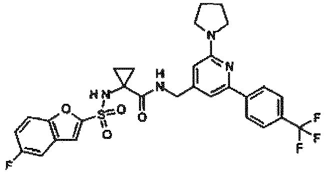 | 1-[(5-fluorobenzofuran-2-yl)sulfonylamino]-N-[[2-pyrrolidin-1-yl-6-[4-(trifluoromethyl)phenyl]-4-pyridyl]methyl]-cyclopropanecarboxamide |
| 29 | 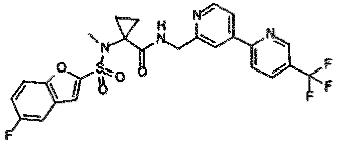 | 1-[(5-fluorobenzofuran-2-yl)sulfonyl-methyl-amino]-N-[[4-[5-(trifluoromethyl)-2-pyridyl]-2-pyridyl]methyl]cyclopropanecarboxamide |

Further preferably, the compounds of Examples 12, 14, 24, 25, 29 having the following structural formulas or a pharmaceutically acceptable salt thereof.

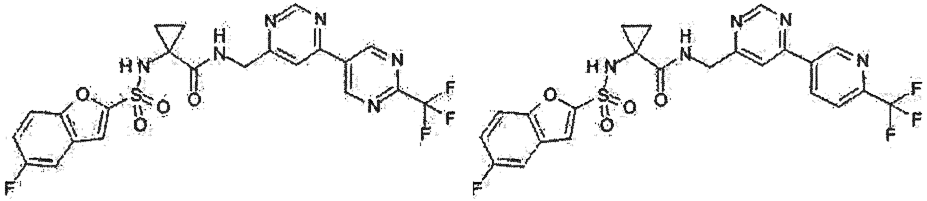

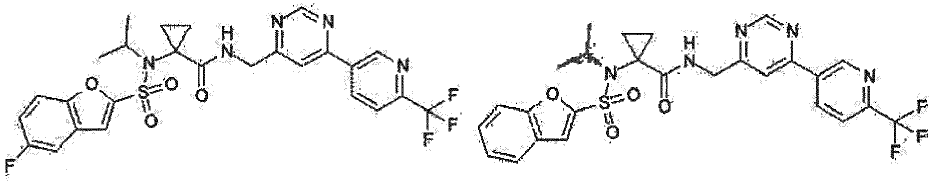

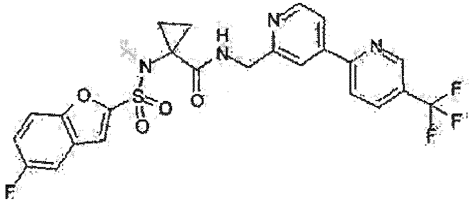

### [Effects of the Invention]

The compound of the present invention has superior TRPA1 antagonist activity, and therefore, is useful for the prophylaxis and/or treatment of diseases involving TRPA1 (e.g., pain associated disease, digestive tract diseases, lung disease, bladder disease, inflammatory diseases, dermatic diseases, and neurological diseases).

### [Description of Embodiments]

The terms used in the present specification are defined below.

The "TRPA1 antagonist activity" refers to an activity capable of inhibiting activation of TRPA1, or down-regulating the biological activity of TRPA1 (e.g., intracellular influx of ion). The TRPA1 antagonist activity can be evaluated by measuring the level of intracellular influx of calcium ion into the cell expressing TRPA1.

The "halogen atom" is a fluorine atom, a chlorine atom, a bromine atom or an iodine atom.

The "halogeno group" is fluoro, chloro, bromo or iodo.

The "C₁₋₆ alkyl group" means a straight chain or branched alkyl group having 1 - 6 carbon atoms and, specifically, groups such as methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like can be mentioned.

The "C₂₋₆ alkenyl group" means a straight chain or branched alkenyl group having 2 - 6 carbon atoms and, specifically, groups such as vinyl, allyl, propenyl, butenyl, pentenyl, hexenyl, heptenyl, butadienyl, hexatrienyl, each isomer thereof and the like can be mentioned.

The "cyclic C₃₋₆ alkyl group" means a cyclic alkyl group having 3 - 6 carbon atoms and, specifically, groups such as cyclopropyl, cyclobutyl, cyclopentyl, cyclohexyl and the like can be mentioned.

The "C₁₋₆ alkoxy group" means a straight chain or branched alkoxy group having 1 - 6 carbon atoms and, specifically, groups such as methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy and the like can be mentioned.

The "C₆₋₁₀ aryl group" means an aryl group having 6 - 10 carbon atoms and, specifically, groups such as phenyl, naphthyl and the like can be mentioned.

The "C₁₋₆ alkyl group", "C₂₋₆ alkenyl group", and "C₁₋₆ alkoxy group" may have a substituent and, as such substituent, the following [substituent group A] can be mentioned.

### [substituent group A]

(1) halogeno group,
(2) hydroxy group,
(3) cyano group,
(4) nitro group,
(5) carboxyl group,
(6) alkenyl group (C₂₋₁₀ alkenyl group; e.g., vinyl, allyl, propenyl, butenyl, pentenyl, hexenyl, heptenyl, butadienyl, hexatrienyl, each isomer thereof),
(7) alkynyl group (C₂₋₁₀ alkynyl group; e.g., ethynyl, propynyl, butynyl, pentynyl, hexynyl, and each isomer thereof),
(8) halogenoalkyl group (e.g., monofluoromethyl, difluoromethyl, trifluoromethyl, monofluoroethyl, difluoroethyl, trifluoroethyl, chloromethyl, chloroethyl, dichloroethyl, each isomer thereof),
(9) cyclic alkyl group(optionally containing heteroatom(s) in the ring) (e.g., cyclopropyl, cyclobutyl, cyclopentyl, cyclohexyl, tetrahydrofuranyl, tetrahydropyranyl, aziridinyl, azetidinyl, pyrrolidinyl, piperidinyl, morpholinyl),
(10) an aryl group (e.g., phenyl, naphthyl),
(11) heteroaryl group (e.g., pyridyl, pyridazinyl, pyrimidinyl, pyrazinyl, furyl, thiophenyl, pyrrolyl, pyrazolyl, imidazolyl, triazolyl (e.g., 1,2,3-triazolyl, 1,2,4-triazolyl), tetrazolyl, oxazolyl, isoxazolyl, thiazolyl, isothiazolyl, oxadiazolyl (e.g., 1,2,3-oxadiazolyl, 1,2,4-oxadiazolyl, 1,3,4-oxadiazolyl), thiadiazolyl (e.g., 1,2,3-thiadiazolyl, 1,2,4-thiadiazolyl, 1,3,4-thiadiazolyl), benzofuryl, benzothiophenyl, indolyl, isoindolyl, benzoxazolyl, benzothiazolyl, benzimidazolyl, indazolyl, benzisoxazolyl, benzisothiazolyl, benzoxadiazolyl, benzothiadiazolyl, purinyl, quinolinyl, isoquinolinyl, cinnolinyl, phthalazinyl, quinazolinyl, quinoxalinyl, pteridinyl, imidazooxazolyl, imidazothiazolyl, imidazoimidazolyl),
(12) alkoxy group (e.g., methoxy, ethoxy, n-propoxy, isopropoxy, n-butoxy, isobutoxy, sec-butoxy, tert-butoxy, n-pentyloxy, isopentyloxy, tert-pentyloxy, neopentyloxy, 2-pentyloxy, 3-pentyloxy, n-hexyloxy, 2-hexyloxy),
(13) alkylthio group (e.g., methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio),
(14) alkoxy group (as defined in the above-mentioned (12)) substituted by an aryl group (as defined in the above-mentioned (10)),
(15) alkylthio group (as defined in the above-mentioned (13)) substituted by an aryl group (as defined in the above-mentioned (10)),
(16) alkoxy group (as defined in the above-mentioned (12)) substituted by a heteroaryl group (as defined in the above-mentioned (11)),
(17) alkylthio group (as defined in the above-mentioned (13)) substituted by a heteroaryl group (as defined in the above-mentioned (11)),
(18) cyclic alkyl(optionally containing heteroatom(s) in the ring)oxy group (e.g., cyclopropyloxy, cyclobutyloxy, cyclopentyloxy, cyclohexyloxy, tetrahydrofuranyloxy, tetrahydropyranyloxy, aziridinyloxy, azetidinyloxy, pyrrolidinyloxy, piperidinyloxy, morpholinyloxy),
(19) aryloxy group (e.g., group wherein aryl group (the above-mentioned (10)) is bonded to oxygen atom),
(20) heteroaryloxy group (e.g., group wherein heteroaryl group (as defined in the above-mentioned (11)) is bonded to oxygen atom),
(21) halogenoalkoxy group (e.g., group wherein halogenoalkyl group (as defined in the above-mentioned (8)) is bonded to oxygen atom),
(22) halogenoalkylthio group (e.g., group wherein halogenoalkyl group (as defined in the above-mentioned (8)) is bonded to sulfur atom),
(23) alkoxy group (as defined in the above-mentioned (12)) substituted by hydroxy group,
(24) alkoxy group (as defined in the above-mentioned (12)) substituted by alkoxy group (as defined in the above-mentioned (12)),
(25) amino group,
(26) amino group mono- or di-substituted by alkyl group,
   wherein "alkyl group" is, for example, C₁₋₆ alkyl group, specifically, methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl and the like, amino group mono- or di-substituted by alkyl group is, for example, amino group mono-substituted by C₁₋₆ alkyl group such as methylamino, ethylamino, n-propylamino, isopropylamino, n-butylamino, isobutylamino, tert-butylamino, n-pentylamino, isopentylamino, hexylamino and the like; amino group di-substituted by C₁₋₆ alkyl group such as dimethylamino, diethylamino, di-n-propylamino, methylethylamino, methylpropylamino, ethylpropylamino and the like,
(27) carbamoyl group,
(28) carbamoyl group mono- or di-substituted by alkyl group (same as the "alkyl group" in the above-mentioned (26)") (e.g., methylcarbamoyl, ethylcarbamoyl, dimethylcarbamoyl, diethylcarbamoyl, ethylmethylcarbamoyl),
(29) sulfamoyl group,
(30) sulfamoyl group mono- or di-substituted by alkyl group (same as the "alkyl group" in the above-mentioned (26)") (e.g., methylsulfamoyl, ethylsulfamoyl, dimethylsulfamoyl, diethylsulfamoyl, ethylmethylsulfamoyl),
(31) alkanoyl group (e.g., carbonyl group wherein a hydrogen atom or alkyl group (same as the "alkyl group" in the above-mentioned (26)") is bonded to carbon atom),
(32) aroyl group (e.g., carbonyl group wherein aryl group (as defined in the above-mentioned (10)) is bonded to carbon atom),
(33) alkylsulfonylamino group (e.g., sulfonylamino group substituted by alkyl group (same as the "alkyl group" in the above-mentioned (26)))
(34) arylsulfonylamino group (e.g., sulfonylamino group substituted by aryl group (as defined in the above-mentioned (10))),
(35) heteroarylsulfonylamino group (e.g., sulfonylamino group substituted by heteroaryl group (as defined in the above-mentioned (11))),
(36) acylamino group (e.g., amino group substituted by acyl group),
   wherein the "acyl group" is an acyl group having a C₁₋₆ alkyl group, a cyclic C₃₋₆ alkyl group, or C₆₋₁₀ aryl group; as the C₁₋₆ alkyl group, a cyclic C₃₋₆ alkyl group and C₆₋₁₀ aryl group, those recited above can be mentioned; as the acyl group, specifically, acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like can be mentioned,
(37) alkoxycarbonylamino group (e.g., carbonylamino group substituted by alkoxy group (as defined in the above-mentioned (12))),
(38) alkylsulfonyl group (e.g., sulfonyl group substituted by alkyl group (same as the "alkyl group" in the above-mentioned (26))),
(39) alkylsulfinyl group (e.g., sulfinyl group substituted by alkyl group (the same as the "alkyl group" in the above-mentioned (26))),
(40) alkoxycarbonyl group (e.g., methoxycarbonyl group, ethoxycarbonyl group), and the like.

When two or more substituents are present, they may be the same or different.

The "cyclic C₃₋₆ alkyl group (optionally containing heteroatom(s) in the ring)" means the above-mentioned cyclic C₃₋₆ alkyl group or a cyclic alkyl group having a carbon number of 3 - 5 and containing at least one heteroatom. Specifically, those exemplified as the above-mentioned "cyclic C₃₋₆ alkyl group" and groups such as tetrahydrofuranyl, tetrahydropyranyl, aziridinyl, azetidinyl, pyrrolidinyl, piperidinyl, piperazinyl, morpholinyl and the like can be mentioned.

The "cycloalkane" is a carbocycle having a carbon number of 3 - 10, preferably 3 - 8, more preferably 3 - 6 and, for example, cyclopropane, cyclobutane, cyclopentane, cyclohexane, cycloheptane, cyclooctane, cyclononane or cyclodecane.

The "C₁₋₆ alkoxycarbonyl group" is a straight chain or branched alkoxycarbonyl group having 1 - 6 carbon atoms and, specifically, groups such as methoxycarbonyl, ethoxycarbonyl, n-propoxycarbonyl, isopropoxycarbonyl, n-butoxycarbonyl, isobutoxycarbonyl, sec-butoxycarbonyl, tert-butoxycarbonyl and the like can be mentioned. The "C₁₋₆ alkoxycarbonyl group" may have a substituent and examples of such substituent include those shown as examples in the above-mentioned [substituent group A].

The "halogeno C₁₋₆ alkyl group" and "halogeno C₁₋₆ alkoxy group" mean a C₁₋₆ alkyl group and a C₁₋₆ alkoxy group, respectively, each of which is substituted by one or more halogeno groups. As the "halogeno C₁₋₆ alkyl group", specifically, groups such as monofluoromethyl, difluoromethyl, trifluoromethyl, monofluoroethyl, difluoroethyl, trifluoroethyl, chloromethyl, chloroethyl, dichloroethyl, each isomer thereof and the like can be mentioned. The "halogeno C₁₋₆ alkoxy group" specifically means a C₁₋₆ alkoxy group substituted by one or more halogeno groups and, specifically, groups such as monofluoromethoxy, difluoromethoxy, trifluoromethoxy, monofluoroethoxy, difluoroethoxy, trifluoroethoxy, chloromethoxy, chloroethoxy, dichloroethoxy, each isomer thereof and the like can be mentioned.

As the "C₁₋₆ alkylthio group", specifically, groups such as methylthio, ethylthio, n-propylthio, isopropylthio, n-butylthio, isobutylthio, sec-butylthio, tert-butylthio, n-pentylthio, isopentylthio, tert-pentylthio, neopentylthio, 2-pentylthio, 3-pentylthio, n-hexylthio, 2-hexylthio and the like can be mentioned.

As the "amino group mono- or di-substituted by a C₁₋₆ alkyl group", specifically, an amino group mono-substituted by C₁₋₆ alkyl, such as methylamino, ethylamino, n-propylamino, isopropylamino, n-butylamino, isobutylamino, tert-butylamino, n-pentylamino, isopentylamino, hexylamino and the like; and an amino group di-substituted by a C₁₋₆ alkyl group, such as dimethylamino, diethylamino, di-n-propylamino, methylethylamino, methylpropylamino, ethylpropylamino and the like can be mentioned. The "amino group mono- or di-substituted by a C₁₋₆ alkyl group" may have a substituent and examples of such substituent include those shown as examples in the above-mentioned [substituent group A].

As the "carbamoyl group mono- or di-substituted by a C₁₋₆ alkyl group", specifically, groups such as methylcarbamoyl, ethylcarbamoyl, dimethylcarbamoyl, diethylcarbamoyl, ethylmethylcarbamoyl and the like can be mentioned. The "carbamoyl group mono- or di-substituted by a C₁₋₆ alkyl group" may have a substituent and examples of such substituent include those shown as examples in the above-mentioned [substituent group A].

As the "amino group substituted by an acyl group", an amino group substituted by an acyl group such as acetyl group, propionyl group, butyroyl group, isobutyroyl group, valeroyl group, isovaleroyl group, pivaloyl group, hexanoyl group, acryloyl group, methacryloyl group, crotonoyl group, isocrotonoyl group, benzoyl group, naphthoyl group and the like can be mentioned. The "amino group substituted by an acyl group" may have a substituent and examples of such substituent include those shown as examples in the above-mentioned [substituent group A].

The "saturated or unsaturated cyclic group (optionally containing heteroatom(s))" means a group derived from a saturated or unsaturated carbocycle (preferably carbon number 5 - 15) or heterocycle (preferably 5-membered to 15-membered).

As the saturated or unsaturated carbocycle, C₅₋₁₅ unsaturated monocycle, bicyclic or tricyclic carbocycle, monocyclic, bicyclic or tricyclic carbocycle in which a part or whole thereof is saturated, spiro-bonded bicyclic carbocycle and bridged bicyclic carbocycle can be mentioned. Examples thereof include cyclopentane, cyclohexane, cycloheptane, cyclopentene, cyclohexene, cycloheptene, cyclopentadiene, cyclohexadiene, cycloheptadiene, benzene, pentalene, perhydropentalene, azulene, perhydroazulene, indene, perhydroindene, indane, naphthalene, dihydronaphthalene, tetrahydronaphthalene, perhydronaphthalene, biphenylene, as-indacene, s-indacene, fluorene, phenanthrene, anthracene, spiro[4.4]nonane, spiro[4.5]decane, spiro[5.5]undecane, bicyclo[2.2.1]heptane, bicyclo[2.2.1]hept-2-ene, bicyclo[3.1.1]heptane, bicyclo[3.1.1]hept-2-ene, bicyclo[2.2.2]octane, bicyclo[2.2.2]oct-2-ene, adamantane, and noradamantane rings.

As the saturated or unsaturated heterocycle, 5 - 15-membered unsaturated monocyclic, bicyclic or tricyclic heterocycle, or monocyclic, bicyclic or tricyclic heterocycle in which a part or whole thereof is saturated, containing, besides at least one carbon atom, 1 - 4 nitrogen atoms, 1 - 2 oxygen atoms and/or 1 - 2 sulfur atoms. Examples thereof include pyrrole, imidazole, triazole, tetrazole, pyrazole, pyridine, pyrazine, triazine, pyrimidine, pyridazine, azepine, diazepine, furan, pyran, oxepin, thiophene, thiopyran, thiepine, oxazole, isoxazole, thiazole, isothiazole, furazan, oxadiazole, oxazine, oxadiazine, oxazepine, oxadiazepine, thiadiazole, thiazine, thiadiazine, thiazepine, thiadiazepine, indole, isoindole, indolizine, benzofuran, isobenzofuran, benzothiophene, isobenzothiophene, dithianaphthalene, indazole, quinoline, isoquinoline, quinolizine, purine, phthalazine, pteridine, naphthyridine, quinoxaline, quinazoline, cinnoline, benzoxazole, benzothiazole, benzimidazole, chromene, benzoxepine, benzoxazepine, benzoxadiazepine, benzothiepine, benzothiazepine, benzothiadiazepine, benzazepine, benzodiazepine, benzofurazan, benzothiadiazole, benzotriazole, carbazole, β-carboline, acridine, phenazine, dibenzofuran, xanthene, dibenzothiophene, phenothiazine, phenoxazine, phenoxathiine, thianthrene, phenanthridine, phenanthrolin, pyrroline, pyrrolidine, imidazoline, imidazolidine, triazoline, triazolidine, tetrazoline, tetrazolidine, pyrazoline, pyrazolidine, dihydropyridine, tetrahydropyridine, piperidine, dihydropyrazine, tetrahydropyrazine, piperazine, dihydropyrimidine, tetrahydropyrimidine, perhydropyrimidine, dihydropyridazine, tetrahydropyridazine, perhydropyridazine, dihydroazepine, tetrahydroazepine, perhydroazepine, dihydrodiazepine, tetrahydrodiazepine, perhydrodiazepine, dihydrofuran, tetrahydrofuran, dihydropyran, tetrahydropyran, dihydrooxepin, tetrahydrooxepin, perhydrooxepin, dihydrothiophene, tetrahydrothiophene, dihydrothiopyran, tetrahydrothiopyran, dihydrothiepine, tetrahydrothiepine, perhydrothiepine, dihydrooxazole, tetrahydrooxazole(oxazolidine), dihydroisoxazole, tetrahydroisoxazole(isoxazolidine), dihydrothiazole, tetrahydrothiazole(thiazolidine), dihydroisothiazole, tetrahydroisothiazole(isothiazolidine), dihydrofurazan, tetrahydrofurazan, dihydrooxadiazole, tetrahydrooxadiazole(oxadiazolidine), dihydrooxazin, tetrahydrooxazin, dihydrooxadiazine, tetrahydrooxadiazine, dihydrooxazepine, tetrahydrooxazepine, perhydrooxazepine, dihydrooxadiazepine, tetrahydrooxadiazepine, perhydrooxadiazepine, dihydrothiadiazole, tetrahydrothiadiazole(thiadiazolidine), dihydrothiazine, tetrahydrothiazine, dihydrothiadiazine, tetrahydrothiadiazine, dihydrothiazepine, tetrahydrothiazepine, perhydrothiazepine, dihydrothiadiazepine, tetrahydrothiadiazepine, perhydrothiadiazepine, morpholine, thiomorpholine, oxathiane, indoline, isoindoline, dihydrobenzofuran, perhydrobenzofuran, dihydroisobenzofuran, perhydroisobenzofuran, dihydrobenzothiophene, perhydrobenzothiophene, dihydroisobenzothiophene, perhydroisobenzothiophene, dihydroindazole, perhydroindazole, dihydroquinoline, tetrahydroquinoline, perhydroquinoline, dihydroisoquinoline, tetrahydroisoquinoline, perhydroisoquinoline, dihydrophthalazine, tetrahydrophthalazine, perhydrophthalazine, dihydronaphthyridine, tetrahydronaphthyridine, perhydronaphthyridine, dihydroquinoxaline, tetrahydroquinoxaline, perhydroquinoxaline, dihydroquinazoline, tetrahydroquinazoline, perhydroquinazoline, dihydrocinnoline, tetrahydrocinnoline, perhydrocinnoline, benzoxathiane, dihydrobenzoxazine, dihydrobenzothiazine, pyrazinomorpholine, dihydrobenzoxazole, perhydrobenzoxazole, dihydrobenzothiazole, perhydrobenzothiazole, dihydrobenzimidazole, perhydrobenzimidazole, dihydrobenzazepine, tetrahydrobenzazepine, dihydrobenzodiazepine, tetrahydrobenzodiazepine, benzodioxepane, dihydrobenzoxazepine, tetrahydrobenzoxazepine, dihydrocarbazole, tetrahydrocarbazole, perhydrocarbazole, dihydroacridine, tetrahydroacridine, perhydroacridine, dihydrodibenzofuran, dihydrodibenzothiophene, tetrahydrodibenzofuran, tetrahydrodibenzothiophene, perhydrodibenzofuran, perhydrodibenzothiophene, dioxolane, dioxane, dithiolane, dithiane, dioxaindane, benzodioxane, chromane, benzodithiolane, benzodithiane and the like.

As the substituent that the "saturated or unsaturated cyclic group (optionally containing heteroatom(s))" optionally has, the groups shown as examples in the above-mentioned [substituent group A] and an alkyl group (e.g., methyl, ethyl, n-propyl, isopropyl, n-butyl, isobutyl, sec-butyl, tert-butyl, n-pentyl, isopentyl, tert-pentyl, neopentyl, 2-pentyl, 3-pentyl, n-hexyl, 2-hexyl) can be mentioned (hereinafter [substituent group B]).

When two or more substituents are present, they may be the same or different.

The present invention provides a compound represented by the formula (I): 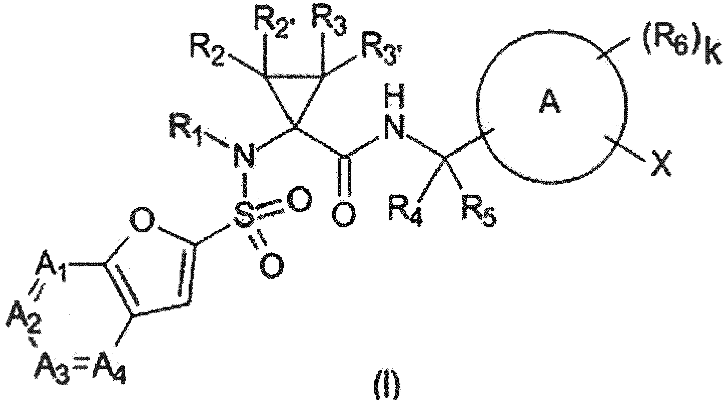 wherein each symbol is as defined above (hereinafter to be also referred to as compound (I)), or a pharmaceutically acceptable salt thereof.

In the formula (I),
R₁ is hydrogen or a C₁₋₆ alkyl group optionally having substituent(s) (e.g., C₂₋₁₀ alkenyl group, C₂₋₁₀ alkynyl group, cyclic alkyl group (optionally containing heteroatom(s) in the ring), aryl group, heteroaryl group, alkoxy group) (preferably hydrogen or a C₁₋₆ alkyl group); R₂, R₂', R₃ and R₃' are the same or different and each is hydrogen or a C₁₋₆ alkyl group (e.g., methyl, ethyl) optionally having substituent(s) (e.g., C₁₋₆ alkoxy group, halogen atom) . Preferably, R₂, R₂', R₃ and R₃' are the same and hydrogens.

In the formula (I),
ring A is a 5-membered or 6-membered monocyclic aromatic ring or heteroaromatic ring or a bicyclic aromatic ring or heteroaromatic ring.

As the 5-membered monocyclic heteroaromatic ring, a 5-membered aromatic monocyclic heterocycle can be mentioned from among the rings exemplified as the above-mentioned "saturated or unsaturated cyclic group (optionally containing heteroatom(s))". Specifically, furan, thiophene, pyrrole, oxazole, isoxazole, thiazole, isothiazole, imidazole, pyrazole, oxadiazole, thiadiazole, triazole, tetrazole and the like can be mentioned.

As the 6-membered monocyclic aromatic ring, 6-membered aromatic monocyclic carbocycle, specifically benzene, can be mentioned from among the rings exemplified as the above-mentioned "saturated or unsaturated cyclic group (optionally containing heteroatom(s))".

As the 6-membered monocyclic heteroaromatic ring, 6-membered aromatic monocyclic heterocycle can be mentioned from among the rings exemplified as the above-mentioned "saturated or unsaturated cyclic group (optionally containing heteroatom(s))". Specifically, pyridine, pyridazine, pyrimidine, pyrazine, triazine and the like can be mentioned.

As the bicyclic aromatic ring, aromatic bicyclic carbocycle, specifically naphthalene and the like, can be mentioned from among the rings exemplified as the above-mentioned "saturated or unsaturated cyclic group (optionally containing heteroatom(s))".

As the bicyclic heteroaromatic ring, aromatic bicyclic heterocycle, specifically, benzofuran, isobenzofuran, benzothiophene, indole, isoindole, indazoline, benzimidazole, benzoxazole, benzisoxazole, benzothiazole, benzisothiazole, benzotriazole, quinoline, isoquinoline, cinnoline, quinazoline and the like, can be mentioned from among the rings exemplified as the above-mentioned "saturated or unsaturated cyclic group (optionally containing heteroatom(s))".

Ring A is preferably a 6-membered monocyclic aromatic ring or heteroaromatic ring or bicyclic aromatic ring or heteroaromatic ring. More preferably, it is a 6-membered monocyclic aromatic ring or heteroaromatic ring. Specifically, benzene, pyridine, pyrimidine, pyridazine and benzofuran are preferable, and benzene, pyridine and pyrimidine are particularly preferable.

In the formula (I),
A₁ is -C(Ra)= or -N=, preferably -C(Ra)=;
A₂ is -C(Rb)= or -N=, preferably -C(Rb)=;
A₃ is -C(Rc)= or -N=, preferably -C(Rc)=;
A₄ is -C(Rd)= or -N=, preferably -C(Rd)=.

At least two of A₁ - A₄ are not -N=.

Ra, Rb, Rc and Rd are the same or different and each is hydrogen, a halogeno group (e.g., fluoro), a cyano group, a hydroxy group, a C₁₋₆ alkyl group, a C₁₋₆ alkoxy group, a halogeno C₁₋₆ alkyl group or a halogeno C₁₋₆ alkoxy group, preferably hydrogen or a halogeno group, more preferably all of them are hydrogens or any one of them is a halogeno group.

In the formula (I), partial structure (a): 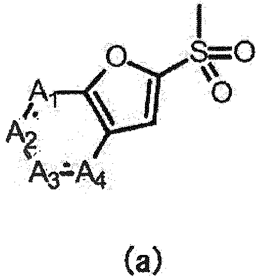 is preferably a group of any of the following illustrations 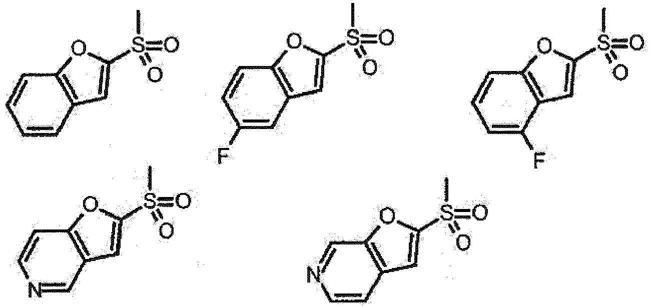 more preferably, a group of any of the following illustrations 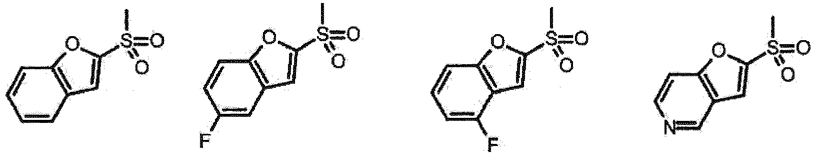 further preferably, a group of any of the following illustrations

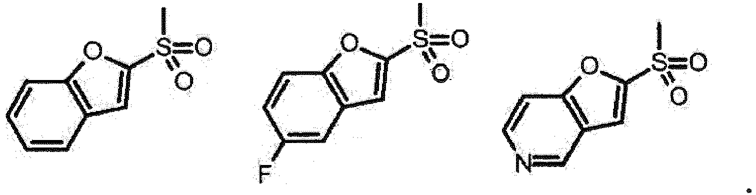

In the formula (I), R₄ and R₅ are the same or different and each is hydrogen or a C₁₋₆ alkyl group, or R₄ and R₅ may be joined to form cycloalkane (e.g., cyclopropane). Preferably, R₄ and R₅ are the same or different and each is hydrogen or a C₁₋₆ alkyl group (wherein R₄ and R₅ are not joined to form cycloalkane), more preferably both R₄ and R₅ are hydrogens.

In the formula (I), X is
(a) hydrogen,
(b) -Cy,
(c) -C(Rₓ₁Rₓ₂)-Cy,
(d) -C(Rₓ₁Rₓ₂)-C(Rₓ₃Rₓ₄)-Cy,
(e) -C(Rₓ₁)=C(Rₓ₂)-Cy,
(f) -O-Cy,
(g) -O-C(Rₓ₁Rₓ₂)-Cy,
(h) -C(Rₓ₁Rₓ₂)-O-Cy,
(i) -S(O)n-Cy,
(j) -S(O)n-C(Rₓ₁Rₓ₂)-Cy,
(k) -C(Rₓ₁Rₓ₂)-S(O)n-Cy,
(l) -N(Rₓ₅)-Cy,
(m) -N(Rₓ₅)-C(Rₓ₁Rₓ₂)-Cy,
(n) -C(Rₓ₁Rₓ₂)-N(Rₓ₅)-Cy,
(o) -N(Rₓ₅)-N(Rₓ₆)-Cy,
(p) -O-N(Rₓ₅)-Cy,
(q) -N(Rₓ₃)-O-Cy,
(r) -C(O)-N(Rₓ₅)-Cy,
(s) -N(Rₓ₅)-C(O)-Cy,
(t) -S(O)m-N(Rₓ₅)-Cy,
(u) -N(Rₓ₅)-S(O)m-Cy,
(v) -O-S(O)m-Cy, or
(w) -S(O)m-O-Cy,
preferably,
(a) hydrogen,
(b) -Cy,
(c) -C(Rₓ₁Rₓ₂)-Cy,
(d) -C(Rₓ₁Rₓ₂)-C(Rₓ₃Rₓ₄)-Cy,
(e) -C(Rₓ₁)=C(Rₓ₂)-Cy,
(f) -O-Cy,
(g) -O-C(Rₓ₁Rₓ₂)-Cy,
(h) -C(Rₓ₁Rₓ₂)-O-Cy,
(i) -S(O)n-Cy,
(j) -S(O)n-C(Rₓ₁Rₓ₂)-Cy,
(k) -C(Rₓ₁Rₓ₂)-S(O)n-Cy,
(l) -N(Rₓ₅)-Cy,
(m) -N(Rₓ₅)-C(Rₓ₁Rₓ₂)-Cy,
(n) -C(Rₓ₁Rₓ₂)-N(Rₓ₅)-Cy,
(o) -N(Rₓ₅)-N(Rₓ₆)-Cy,
(p) -O-N(Rₓ₅)-Cy,
(q) -N(Rₓ₅)-O-Cy,
(r) -C(O)-N(Rₓ₃)-Cy,
(s) -N(Rₓ₅)-C(O)-Cy,
(t) -S(O)m-N(Rₓ₅)-Cy, or
(u) -N(Rₓ₅)-S(O)m-Cy,
more preferably,
(a) hydrogen,
(b) -Cy,
(f) -O-Cy,
(g) -O-C(Rₓ₁Rₓ₂)-Cy,
(h) -C(Rₓ₁Rₓ₂)-O-Cy,
(j) -S(O)n-C(Rₓ₁Rₓ₂)-Cy, or
(m) -N(Rₓ₅)-C(Rₓ₁Rₓ₂)-Cy
(each symbol is as defined in the formula (I)).

More preferably, X is hydrogen, -Cy, -O-Cy or -O-CH₂-Cy, particularly preferably -Cy.

Cy is a saturated or unsaturated cyclic group (optionally containing heteroatom(s)) optionally having substituent(s), preferably monocyclic or bicyclic saturated or unsaturated cyclic group (optionally containing heteroatom(s)), more preferably a monocyclic saturated or unsaturated cyclic group (optionally containing heteroatom(s)). Specifically, cyclopentane, cyclohexane, cyclohexene, benzene, naphthalene, pyrrole, imidazole, triazole, tetrazole, pyrazole, pyridine, pyrazine, triazine, pyrimidine, pyridazine, furan, thiophene, oxazole, isoxazole, thiazole, isothiazole, oxadiazole, thiadiazole, indole, benzofuran, benzothiophene, quinoline, isoquinoline, quinazoline, benzoxazole, benzothiazole, benzimidazole, tetrahydrofuran, dihydropyran or tetrahydropyran is preferable, cyclopentane, cyclohexane, benzene, pyrazole, pyridine, pyrazine, pyrimidine, pyridazine, furan, thiophene, tetrahydrofuran or tetrahydropyran is further preferable, benzene, pyridine, pyrazine, pyrimidine or pyridazine is particularly preferable.

Regarding Cy, as the substituent that the "saturated or unsaturated cyclic group (optionally containing heteroatom(s))" optionally has, those exemplified in the above-mentioned [substituent group B] can be mentioned. Preferably, it is unsubstituted, and alkyl group, alkenyl group, halogenoalkyl group, cyclic alkyl group (optionally containing heteroatom(s) in the ring), halogeno group, hydroxy group, alkoxy group, halogenoalkoxy group, alkyl group substituted by halogenoalkoxy group, amino group, amino group mono- or di-substituted by alkyl group, cyano group, alkylthio group, carboxyl group, alkoxycarbonyl group, carbamoyl group, carbamoyl group mono- or di-substituted by alkyl group, acylamino group and the like can be mentioned. Further preferably, it is unsubstituted, halogeno group, halogenoalkyl group, hydroxy group, halogenoalkoxy group, or cyano group.

Rₓ₁, Rₓ₂, Rₓ₃, Rₓ₄, Rₓ₅ and Rₓ₆ are the same or different and each is hydrogen, a C₁₋₆ alkyl group optionally having substituent(s) or a C₁₋₆ alkoxycarbonyl group optionally having substituent(s). Preferably, it is hydrogen.

Cy is preferably is a group of any of the following illustrations 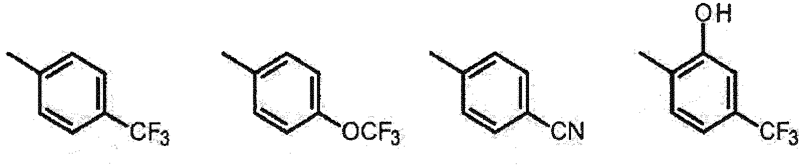 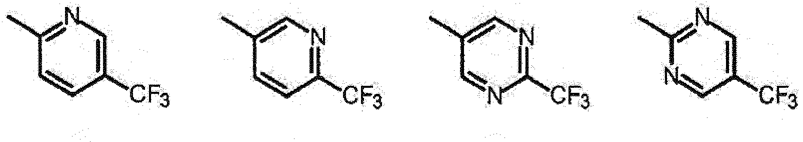 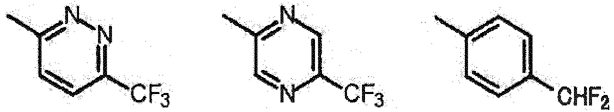 more preferably, a group of any of the following illustrations

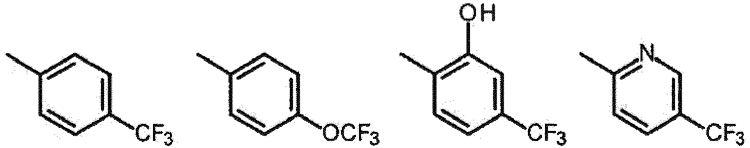

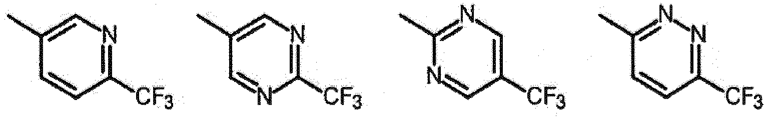

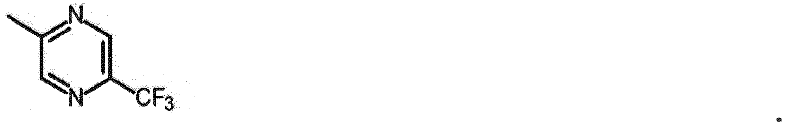

In the formula (I),
R₆ is a C₁₋₆ alkyl group optionally having substituent(s), a C₂₋₆ alkenyl group, a cyclic C₃₋₆ alkyl group (optionally containing heteroatom(s)), a halogeno group, a hydroxy group, a C₁₋₆ alkoxy group optionally having substituent(s), a halogeno C₁₋₆ alkyl group, a halogeno C₁₋₆ alkoxy group, an amino group, an amino group mono- or di-substituted by a C₁₋₆ alkyl group optionally having substituent(s), a cyano group, a C₁₋₆ alkylthio group, a carboxyl group, a C₁₋₆ alkoxycarbonyl group optionally having substituent(s), a carbamoyl group, a carbamoyl group mono- or di-substituted by a C₁₋₆ alkyl group optionally having substituent(s) or an amino group substituted by an acyl group optionally having substituent(s). When R₆ is present in plurality, they may be the same or different. As the substituent that the "C₁₋₆ alkyl group", "C₂₋₆ alkenyl group", "C₁₋₆ alkoxy group" optionally have, the groups shown as examples in the above-mentioned [substituent group A] can be mentioned, with preference given to alkyl group, alkenyl group, aryl group, cyclic alkyl group (optionally containing heteroatom(s) in the ring), halogeno group, hydroxy group, alkoxy group, amino group, amino group mono- or di-substituted by alkyl group, cyano group, alkylthio group, carboxyl group, alkoxycarbonyl group, carbamoyl group, carbamoyl group mono- or di-substituted by alkyl group, acylamino group and the like.

R₆ is preferably a C₁₋₆ alkyl group, a cyclic C₃₋₆ alkyl group (optionally containing heteroatom(s)), a halogeno group, a hydroxy group, a C₁₋₆ alkoxy group optionally having substituent(s), a halogeno C₁₋₆ alkoxy group, an amino group, or an amino group mono- or di-substituted by a C₁₋₆ alkoxycarbonyl group or C₁₋₆ alkyl group, more preferably a cyclic C₃₋₆ alkyl group (optionally containing heteroatom(s)), a halogeno group, a hydroxy group, a C₁₋₆ alkoxy group optionally having substituent(s), a halogeno C₁₋₆ alkoxy group, or an amino group mono- or di-substituted by a C₁₋₆ alkyl group.

k is an integer of 0 - 3, preferably 0 to 2, more preferably 0 or 1.

In the formula (I),
partial structure (b) containing ring A: 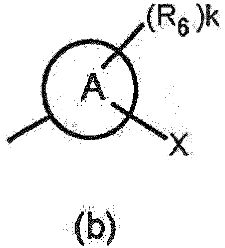 is preferably a group of any of the following illustrations 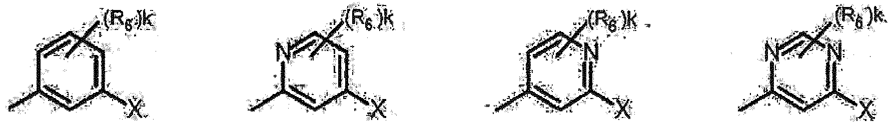 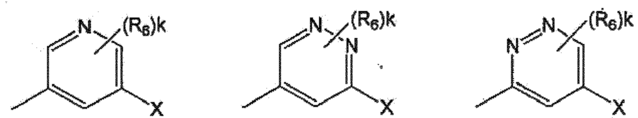 wherein each symbol is as defined in the formula (I).

More preferably, partial structure (b) is a group of any of the following illustrations

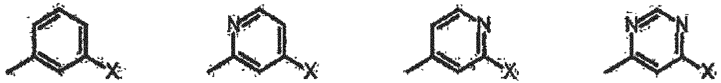

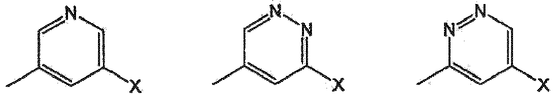

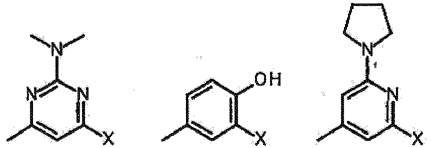

A compound represented by the formula (I) or a pharmaceutically acceptable salt thereof is also referred to as the compound of the present invention.

In the present invention, preferable compounds of the present invention include the following compounds.

### (embodiment 1)

A compound wherein ring A is a 6-membered monocyclic aromatic ring or heteroaromatic ring (benzene, pyrimidine, pyridine, pyridazine, preferably benzene, pyrimidine, pyridine, more preferably pyridine, pyrimidine); k=0 or 1; partial structure (a) is 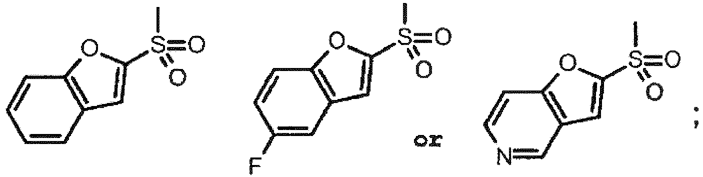 R₂, R₂', R₃ and R₃' are each hydrogen, or a pharmaceutically acceptable salt thereof.

As the compound of the present invention, preferred are the compounds described in the below-mentioned Examples or a pharmaceutically acceptable salt thereof, more preferred are the compounds of Examples 1, 3, 4, 8, 11, 12, 14, 18, 19, 20, 21, 24, 25, 26, 27, 29 or a pharmaceutically acceptable salt thereof.

Further preferred are the compounds of Examples 12, 14, 24, 25, 29 represented by the following structural formulas or a pharmaceutically acceptable salt thereof.

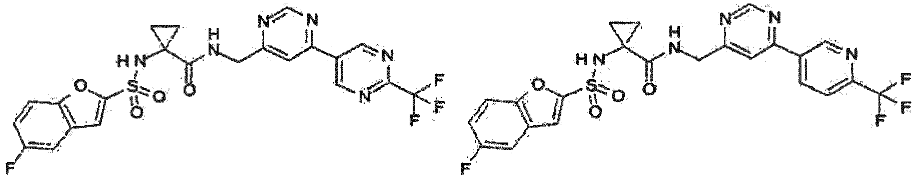

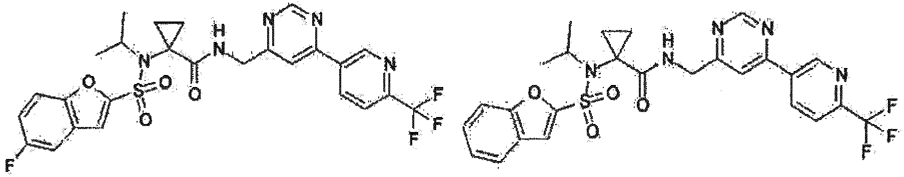

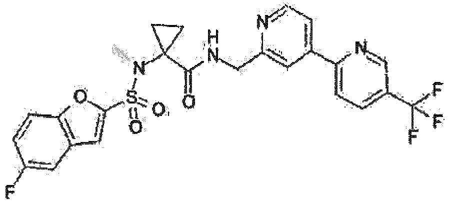

When the compound of the present invention can form a salt, the salt only needs to be pharmaceutically acceptable. For example, when an acidic group such as a carboxyl group and the like is present in the formula, ammonium salt, salts with alkali metal such as sodium, potassium and the like, salts with alkaline earth metal such as calcium, magnesium and the like, aluminum salt, zinc salt, salts with organic amine such as triethylamine, ethanolamine, morpholine, piperidine, dicyclohexylamine and the like, and salts with basic amino acid such as arginine, lysine and the like can be mentioned with regard to the acidic group. When a basic group is present in the formula, salts with inorganic acid such as hydrochloric acid, sulfuric acid, phosphoric acid, nitric acid, hydrobromic acid and the like, salts with organic carboxylic acid such as acetic acid, trifluoroacetic acid, citric acid, benzoic acid, maleic acid, fumaric acid, tartaric acid, succinic acid, tannic acid, butyric acid, phthalein acid, pamoic acid, enanthic acid, decanoic acid, 8-chlorotheophylline, salicylic acid, lactic acid, oxalic acid, mandelic acid, malic acid and the like, and salts with organic sulfonic acid such as methanesulfonic acid, benzenesulfonic acid, p-toluenesulfonic acid and the like can be mentioned with regard to the basic group. As a method for forming a salt, the compound of the present invention and necessary acid or base are mixed at a suitable quantitative ratio in a solvent or a dispersing agent, or cation exchange or anion exchange of other salt form is employed.

The compound of the present invention also encompasses optical isomer, stereoisomer, tautomer, rotamer, and mixtures thereof at optional ratios. These can be obtained each as a single product according to a synthesis method and separation method known per se. For example, an optical isomer can be obtained by using an optically active synthesis intermediate or by optically resolving a racemate of a synthesis intermediate or final product by a conventional method.

Furthermore, it also encompasses a stable isotope and a radioactive isotope.

The compound of the present invention also includes solvates of the compound such as hydrate, alcohol adduct and the like.

The compound of the present invention can also be converted to a prodrug. The prodrug in the present invention is a compound that is converted in the body to produce the compound of the present invention. For example, when the active component contains a carboxyl group or a phosphoric acid group, an ester, amide and the like thereof can be mentioned. When the active component contains an amino group, an amide, carbamate and the like thereof can be mentioned. When the active component contains a hydroxy group, an ester, carbonate, carbamate and the like thereof can be mentioned. When the compound of the present invention is converted to a prodrug, it may be bonded to an amino acid or saccharides.

The present invention also encompasses a metabolite of the compound of the present invention. The metabolite of the compound of present invention means a compound resulting from the conversion of the compound of the present invention by a metabolic enzyme and the like in the body. For example, a compound wherein a hydroxy group is introduced on the benzene ring of the compound of the present invention due to the metabolism, a compound wherein glucuronic acid, glucose or amino acid is bonded to the carboxylic acid moiety of the compound of the present invention or a hydroxy group added by the metabolism, and the like can be mentioned.

The compound of the present invention has a TRPA1 antagonist activity for mammals such as human, bovine, horse, dog, mouse, rat and the like, and can be used as a medicament, which is administered as it is or as a pharmaceutical composition containing the same mixed with a pharmaceutically acceptable carrier according to a method known per se. While oral administration is generally preferable, parenteral administration (e.g., routes such as intravenous, subcutaneous, intramuscular, suppository, enema, ointment, patch, sublingual, eye drop, inhalation administrations and the like) can also be employed. While the dose used for the above-mentioned objects is determined according to the desired treatment effect, administration method, duration of treatment, age, body weight and the like, a daily dose of 1 µg - 10 g for oral administration and 0.01 µg - 1 g for parenteral administration is used, which is generally administered to an adult by an oral or parenteral route in one to several portions per day or once per several days. In addition, the content of the compound of the present invention in the above-mentioned pharmaceutical composition is about 0.01 wt% - 100 wt% of the whole composition.

Examples of the pharmaceutically acceptable carrier for the pharmaceutical composition of the present invention include various organic or inorganic carrier substances conventionally used as preparation materials. For example, excipient, lubricant, binder, disintegrant, water-soluble polymer and basic inorganic salt in solid preparation; solvent, solubilizing agents, suspending agent, isotonicity agent, buffering agent and soothing agent in liquid preparation, and the like can be mentioned. Where necessary, general additives such as preservative, antioxidant, colorant, sweetening agent, souring agent, foaming agent, flavor and the like can also be used.

The dosage form of such pharmaceutical composition may be tablet, powder, pill, granule, capsule, suppository, solution, sugar-coated agent, depot, syrup, suspension, emulsion, troche, sublingual agent, adhesive preparation, oral disintegrant (tablet), inhalant, enema, ointment, patch, tape and eye drop, and these can be produced using conventional formulation auxiliaries and according to a conventional method.

The pharmaceutical composition of the present invention can be produced according to a method conventionally used in the technical field of pharmaceutical formulation, for example, the method described in the Japanese Pharmacopoeia and the like. Specific production methods of the preparation are explained in detail in the following.

For example, when the compound of the present invention is prepared as an oral preparation, excipient and, where necessary, binder, disintegrant, lubricant, colorant, flavoring agent and the like are further added and the mixture is processed to give, for example, tablet, powder, pill, granule, capsule, solution, sugar-coated agent, depot, syrup and the like according to a conventional method. Examples of the excipient include lactose, cornstarch, sucrose, glucose, sorbitol, crystalline cellulose and the like. Examples of the binder include poly(vinyl alcohol), polyvinyl ether, ethylcellulose, methylcellulose, gum arabic, tragacanth, gelatin, shellac, hydroxypropylcellulose, hydroxypropylstarch, polyvinylpyrrolidone and the like. Examples of the disintegrant include starch, agar, gelatin powder, crystalline cellulose, calcium carbonate, sodium hydrogen carbonate, calcium citrate, dextran, pectin and the like. Examples of the lubricant include magnesium stearate, talc, polyethylene glycol, silica, hydrogenated vegetable oil and the like. As the colorant, one allowed to add to a pharmaceutical product is used, and as the flavoring agent, cocoa powder, menthol, aromatic acid, peppermint oil, borneol, powdered cinnamon bark and the like are used. Where necessary, these tablets and granules are applied with a coating as appropriate such as sugar coating, gelatin coating, and the like.

When an injection is to be prepared, pH adjuster, buffering agent, stabilizer, preservative and the like are added where necessary and the mixture is processed to give subcutaneous, intramuscular or intravenous injection according to a conventional method.

As mentioned above, since the compound of the present invention shows a superior TRPA1 antagonist activity for mammals (e.g., mouse, rat, hamster, rabbit, cat, dog, swine, bovine, sheep, horse, monkey, human etc., preferably human), it is useful as a TRPA1 antagonist. Moreover, the compound of the present invention is possibly utilizable for the prophylaxis and/or treatment of diseases involving TRPA1, and the compound of the present invention can be provided as a medicament for the prophylaxis and/or treatment of such diseases.

As the disease involving TRPA1, pain associated disease, digestive tract diseases, lung disease, bladder disease, inflammatory disease, dermatic diseases, and neurological disease and the like can be mentioned.

As the pain-associated disease, specifically, chronic pain, neuropathic pain, acute pain, inflammatory pain, postherpetic neuralgia, neuropathy, neuralgia, diabetic neuropathy, HIV related neuropathy, nerve injury, rheumatoid arthritis pain, osteoarthritis pain, back pain, lumbago, cancer pain, toothache, headache, migraine, carpal-tunnel syndrome, fibromyalgia syndrome, neuritis, sciatic neuralgia, pelvic hypersensitivity, pelvic pain, menstrual pain, organ pain, pain after operation and the like can be mentioned.

As the digestive tract disease, functional gastrointestinal disorder {dysphagia, functional dyspepsia (FD), irritable bowel syndrome (IBS), functional abdominal pain syndrome}, erosive esophagitis (GERD), ulcer, inflammatory bowel disease (IBD), vomiting (cancer chemotherapy-induced vomiting), pancreatitis and the like can be mentioned.

As the lung disease, asthma, chronic obstructive pulmonary diseases (COPD), bronchoconstriction and the like can be mentioned.

As the bladder disease, overactive bladder, abnormal urination, cystitis and the like can be mentioned.

As the inflammatory disease, burn, osteoarthritis and the like can be mentioned.

As the dermatic disease, allergic dermatitis including atopic dermatitis, pruritus and the like can be mentioned.

As the neurological disease, anticancer agent-induced neuropathy and the like can be mentioned.

As the disease involving TRPA1, preferably, chronic pain, neuropathic pain, acute pain, asthma, chronic obstructive pulmonary diseases, functional gastrointestinal disorder, erosive esophagitis, inflammatory bowel disease, pruritus, anticancer agent-induced neuropathy and the like can be mentioned.

The production methods of the representative compounds among the compounds of the present invention are shown below, but the production method of the compound of the present invention is not limited to them. Unless particularly indicated, each symbol in the formulas is as defined above.

One embodiment of the synthesis method of compound (I) is shown below.

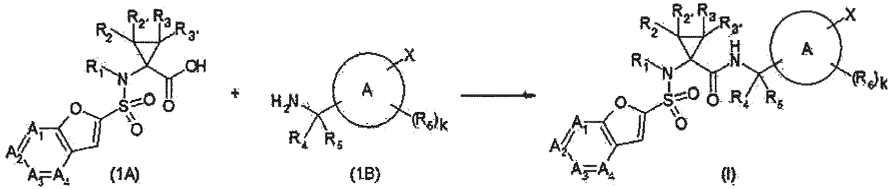

The object compound (I) can be synthesized by reacting carboxylic acid derivative (1A) and amine derivative (1B) in a solvent that does not adversely influence the reaction such as dichloromethane and the like in the presence or absence of an additive such as 1-hydroxybenzotriazole and the like with a condensing agent represented by 1-ethyl-3-(3'-dimethylaminopropyl)carbodiimide (WSC) in the presence or absence of a base such as triethylamine and the like.

The above-mentioned carboxylic acid derivative (1A) can be synthesized as follows.

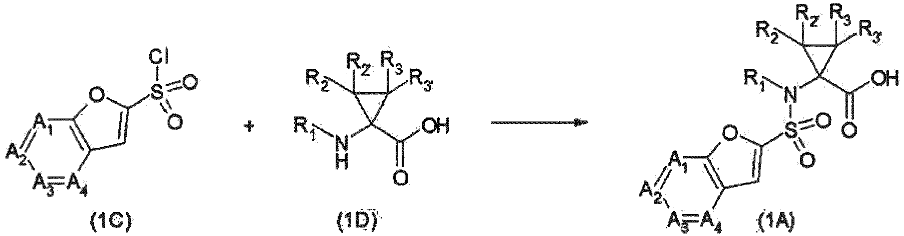

Carboxylic acid derivative (1A) can be synthesized by reacting sulfonyl chloride derivative (1C) and amine derivative (1D) in a solvent that does not adversely influence the reaction such as a mixed solvent of tetrahydrofuran and water and the like in the presence of a base such as sodium hydroxide and the like (sulfoneamidation). Carboxylic acid derivative (1A) can also be synthesized by protecting carboxylic acid of amine derivative (1D) with an appropriate protecting group (e.g., methyl, ethyl, benzyl, tert-butyl and the like) where necessary, and removing the protecting group by an appropriate method such as acid treatment and the like after the above-mentioned sulfoneamidation.

Sulfonyl chloride derivative (1C) can be synthesized as follows.

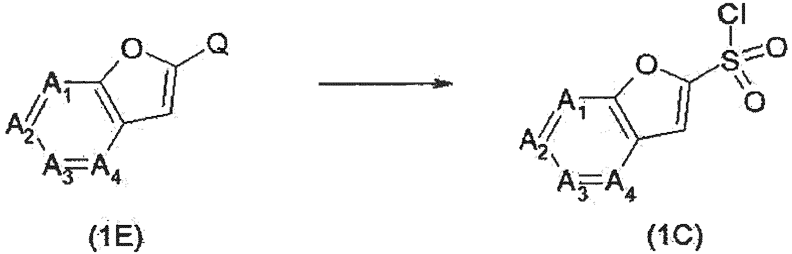

Sulfonyl chloride derivative (1C) can be synthesized by reacting furan derivative (1E) (wherein Q is a bromine atom, an iodine atom, a chlorine atom, a hydrogen atom or the like) with, for example, an alkyllithium reagent such as n-butyllithium, sec-butyllithium or tert-butyllithium and the like in a solvent that does not adversely influence the reaction such as diethyl ether, tetrahydrofuran and the like and reacting same with, for example, sulfur dioxide and reacting same with, for example, a chlorinating agent such as N-chlorosuccinimide and the like.

For example, a synthesis method of a compound represented by (1B-1), wherein, in the formula (1B), when -X is a group represented by -Cy, i.e., 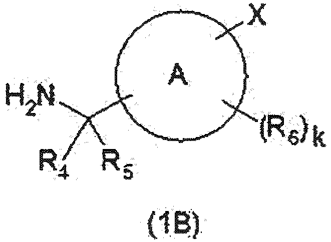 is 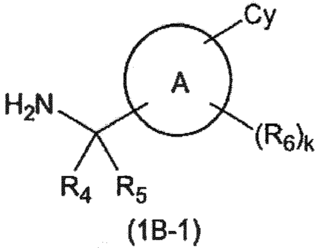 is shown below

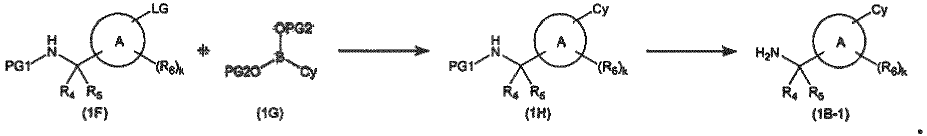

Amine derivative (1H) can be synthesized by reacting amine derivative (1F) (wherein PG1 is a suitable protecting group such as tert-butoxycarbonyl group (Boc group), benzyloxycarbonyl group (Cbz group) and the like, and LG is a suitable leaving group such as chlorine atom, bromine atom, iodine atom, trifluoromethanesulfonyloxy group and the like) and boronic acid derivative (1G) (wherein -B(OPG2)₂ is -B(OH)₂ or a suitable boronic acid derivative such as catecholborane, pinacolborane, N-methyliminodiacetic acid boronate and the like) in a solvent that does not adversely influence the reaction such as 1,4-dioxane or toluene, butanol and the like in the presence or absence of a cosolvent such as water and the like, with a base such as sodium hydroxide, sodium carbonate, potassium carbonate, sodium hydrogen carbonate, potassium hydrogen carbonate, tripotassium phosphate and the like, in the presence or absence of an additive such as copper acetate and the like, in the presence or absence of a ligand such as 2,4,6-triisopropyl-2'-(dicyclohexylphosphino)biphenyl and the like, and in the presence of a catalyst such as [1,1'-bis(diphenylphosphino)ferrocene]dichloropalladium, tris(dibenzylideneacetone)dipalladium, tetrakis(triphenylphosphine)palladium and the like. The object compound (1B-1) can be produced by removing the protecting group PG1 of the obtained amine derivative (1H) thereafter. The deprotection reaction is known and when, for example, PG1 is a tert-butoxycarbonyl group, a method using protic acid such as hydrochloric acid and trifluoroacetic acid, and a method using a Lewis acid such as boron trifluoride and tin tetrachloride can be mentioned. In addition, when, for example, PG1 is a benzyloxycarbonyl group, a method using a hydrogenation reaction in the presence of a catalytic amount of palladium/carbon and the like under normal pressure or pressurized hydrogen atmosphere, a method using a hydrobromic acid/acetic acid, and the like can be mentioned.

When a compound wherein R₄ and R₅ are joined to form cycloalkane is synthesized, a compound in any stepof the above-mentioned scheme may be subjected to a ring (cycloalkane) forming reaction.

Amine derivative (1B-1) can also be synthesized as follows.

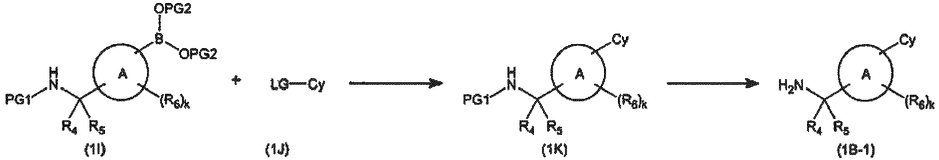

Amine derivative (1K) can be synthesized by reacting amine derivative(1I) (wherein -B(OPG2)₂ is -B(OH)₂ or a suitable boronic acid derivative such as catecholborane, pinacolborane, N-methyliminodiacetic acid boronate and the like) with compound (1J) having an appropriate leaving group (wherein LG is a suitable leaving group such as chlorine atom, bromine atom, iodine atom, trifluoromethanesulfonyloxy group and the like) in a solvent that does not adversely influence the reaction such as 1,4-dioxane or toluene, butanol and the like in the presence or absence of a cosolvent such as water and the like, with a base such as sodium hydroxide, sodium carbonate, potassium carbonate, sodium hydrogen carbonate, potassium hydrogen carbonate, tripotassium phosphate and the like, in the presence or absence of an additive such as copper acetate and the like, in the presence or absence of a ligand such as 2,4,6-triisopropyl-2'-(dicyclohexylphosphino)biphenyl and the like and in the presence of catalyst such as [1,1'-bis(diphenylphosphino)ferrocene]dichloropalladium, tris(dibenzylideneacetone)dipalladium, tetrakis(triphenylphosphine)palladium and the like, and amine derivative (1B-1) can be synthesized by removing the protecting group PG1 by the aforementioned method and the like.

When a compound wherein R₄ and R₅ are joined to form cycloalkane is synthesized, a compound in any step of the above-mentioned scheme may be subjected to a ring (cycloalkane) forming reaction.

For example, a synthesis method of a compound represented by (1B-2), wherein, in the formula (1B), when -X is a group represented by -Cy; and both R₄ and R₅ are hydrogen atoms, i.e., 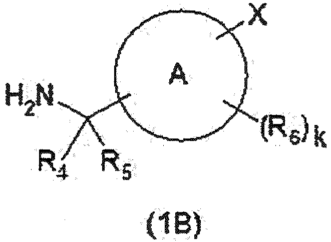 is 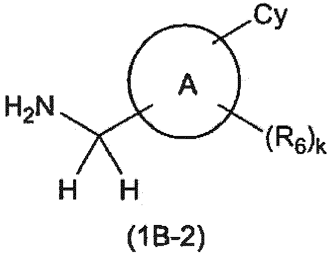 is shown below

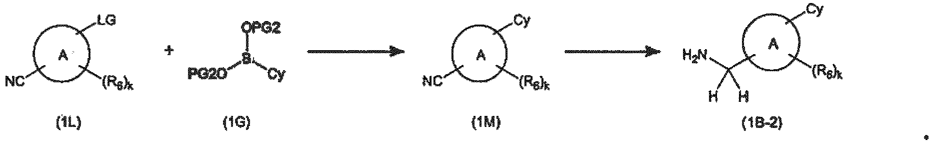

Nitrile derivative (1M) can be synthesized by reacting nitrile derivative (1L) (wherein LG is a suitable leaving group such as chlorine atom, bromine atom, iodine atom, trifluoromethanesulfonyloxy group and the like) and boronic acid derivative (1G) (wherein -B(OPG2)₂ is -B(OH)₂ or a suitable boronic acid derivative such as catecholborane, pinacolborane, N-methyliminodiacetic acid boronate and the like) in a solvent that does not adversely influence the reaction such as 1,4-dioxane or toluene, butanol and the like in the presence or absence of a cosolvent such as water and the like, with a base such as sodium hydroxide, sodium carbonate, potassium carbonate, sodium hydrogen carbonate, potassium hydrogen carbonate, tripotassium phosphate and the like, in the presence or absence of an additive such as copper acetate and the like, in the presence or absence of a ligand such as 2,4,6-triisopropyl-2'-(dicyclohexylphosphino)biphenyl and the like, and in the presence of a catalyst such as [1,1'-bis (diphenylphosphino)ferrocene]dichloropalladium, tris(dibenzylideneacetone)dipalladium, tetrakis(triphenylphosphine)palladium and the like. Amine derivative (1B-2) can be synthesized by reducing the obtained nitrile derivative (1M) in a solvent that does not adversely influence the reaction such as water, methanol, ethanol, tetrahydrofuran and the like in the presence of a catalyst such as palladium/carbon, palladium hydroxide, platinum/carbon and the like in the presence or absence of an acid such as acetic acid, hydrochloric acid and the like, under a hydrogen atmosphere at normal pressure or under pressurization. Amine derivative (1B-2) can also be synthesized by reacting in a solvent that does not adversely influence the reaction such as tetrahydrofuran and the like with lithium aluminum hydride, borane tetrahydrofuran complex and the like. In addition, amine derivative (1B-2) can also be synthesized by a reaction with sodium tetrahydroborate and the like in a solvent that does not adversely influence the reaction such as tetrahydrofuran and the like in the presence or absence of a cosolvent such as water and the like in the presence of a catalyst such as cobalt chloride and the like.

In addition, amine derivative (1B-2) can also be synthesized as follows.

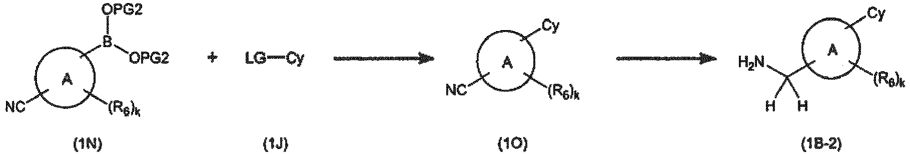

Nitrile derivative (10) can be synthesized by reacting nitrile derivative (1N) (wherein -B(OPG2)₂ is -B(OH)₂ or a suitable boronic acid derivative such as catecholborane, pinacolborane, N-methyliminodiacetic acid boronate and the like) with compound (1J) having an appropriate leaving group (wherein LG is a suitable leaving group such as chlorine atom, bromine atom, iodine atom, trifluoromethanesulfonyloxy group and the like) in a solvent that does not adversely influence the reaction such as 1,4-dioxane or toluene, butanol and the like in the presence or absence of a cosolvent such as water and the like, with a base such as sodium hydroxide, sodium carbonate, potassium carbonate, sodium hydrogen carbonate, potassium hydrogen carbonate, tripotassium phosphate and the like, in the presence or absence of an additive such as copper acetate and the like, in the presence or absence of a ligand such as 2,4,6-triisopropyl-2'-(dicyclohexylphosphino)biphenyl and the like and in the presence of catalyst such as [1,1'-bis(diphenylphosphino)ferrocene]dichloropalladium, tris(dibenzylideneacetone)dipalladium, tetrakis(triphenylphosphine)palladium and the like, and amine derivative (1B-2) can be synthesized by reducing the nitrile group by the aforementioned method and the like.

### [Examples]

The present invention is explained in detail in the following by referring to Reference Examples, Examples and Experimental Examples, which are not to be construed as limitative. Unless particularly indicated, the apparatuses, reagents and the like to be used in the Examples can be easily prepared according to a method generally practiced in the pertinent field or are commercially available. In addition, % in the title compound means the yield.

**Table 2**

| Ref. Ex. No. | structural formula | MS(ESI) *m*/*z* (M+H)⁺ | NMR |
|---|---|---|---|
| A-1 | 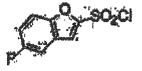 O SO2Cl F | 235 | ¹H NMR (400 MHz, CD₃OD) δ 7.67-7.63 (m, 2H), 7.47-7.34 (m, 2H). |

### Reference Example A-1: Synthesis of 5-fluorobenzofuran-2-ylsulfonyl chloride (A-1)

### (step 1) Synthesis of 2-(2,2-dibromovinyl)-4-fluorophenol

A solution of carbon tetrabromide (1.70 kg, 5.14 mol) in dichloromethane (80 mL) was cooled to 0°C, triphenylphosphine (2.07 kg, 7.91 mol) was added and the mixture was stirred for 30 min. To the reaction mixture was added triethylamine (1.30 kg, 12.8 mol), and 5-fluoro-2-hydroxybenzaldehyde (300 g, 2.14 mol) was slowly added while maintaining the reaction temperature to 5°C or below. The reaction mixture was stirred at 30°C for 2 hr and concentrated under reduced pressure, and the obtained residue was purified by silica gel column chromatography (hexane/ethyl acetate) to give the title compound (300 g, 1.01 mol, 47%).
¹H NMR (400 MHz, CDCl₃) δ 7.54 (s, 1H), 7.35-7.32 (m, 1H), 6.95-6.90 (m, 1H), 6.79-6.75 (m, 1H), 5.41 (s, 1H).

### (step 2) Synthesis of 2-bromo-5-fluorobenzofuran

To the compound (300 g, 1.01 mol) obtained in step 1, copper(I) iodide (15.5 g, 81 mmol) and potassium phosphate (430 g, 2.03 mol) was added tetrahydrofuran (2 L) and the mixture was stirred at 80°C for 2 hr. The insoluble material was filtered off, and the filtrate was concentrated under reduced pressure. The obtained residue was purified by silica gel column chromatography (hexane) to give the title compound (120 g, 0.56 mol, 55%).
¹H NMR (300 MHz, CDCl₃) δ 7.41-7.37 (m, 1H), 7.20-7.17 (m, 1H), 7.03-6.96 (m, 1H), 6.71 (s, 1H).

### (step 3) Synthesis of 5-fluorobenzofuran-2-ylsulfonyl chloride (A-1)

To the compound (80 g, 0.37 mol) obtained in step 2 was added diethylether (2 L), and the mixture was cooled to 0°C. 1.3 mol/L tert-Butyllithium (n-pentane solution, 375 mL, 0.49 mol) was slowly added dropwise while maintaining the reaction temperature to 5°C or below. After stirring at 0°C for 30 min, sulfur dioxide was blown into the reaction mixture for 25 min while maintaining the reaction temperature to 5°C or below. N-chlorosuccinimide (65 g, 0.49 mol) was added at 0°C and the mixture was stirred for 20 min. The reaction mixture was poured into ice water, and extracted with dichloromethane. The organic layer was dried over sodium sulfate. The desiccant was filtered off, the solvent was evaporated and the obtained residue was purified by silica gel column chromatography (hexane) to give the title compound (28 g, 0.12 mol, 32%).
MS (ESI) m/z 235 (M+H)⁺
¹H NMR (400 MHz, CD₃OD) δ 7.67-7.63 (m, 2H), 7.47-7.34 (m, 2H).

**Table 3**

| Ref. Ex. No. | structural formula | MS (ESI) m/z (M+H)⁺ | NMR |
|---|---|---|---|
| B-1 | 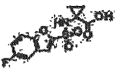 | 300 | - |
| B-2 | 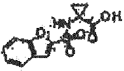 | 282 | - |
| B-3 | 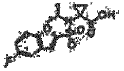 | 342 | - |
| B-4 | 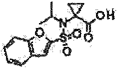 | 324 | - |
| B-5 | 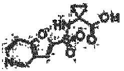 | 283 | ¹H NMR (400 MHz, DMSO-d₆): δ 12.56 (s, 1H), 9.38 (s, 1H), 9.06 (s, 1H), 8.62 ( (d, J=5.6 Hz, 1H), 7.81 (d, J=5.6 Hz, 1H), 7.64 (s, 1H), 1.35-1.31 (m, 2H), 1.29-1.24 (m, 2H). |

### Reference Example B-1: Synthesis of 1-[(5-fluorobenzofuran-2-yl)sulfonylamino]cyclopropanecarboxylic acid (B-1)

To a solution of 1-aminocyclopropanecarboxylic acid (0.37 g, 3.6 mmol) in tetrahydrofuran (3 mL) were added A-1 (1.0 g, 4.3 mmol) and 2 mol/L aqueous sodium hydroxide solution (3.0 mL, 6.0 mmol) and the mixture was stirred at room temperature for 4 hr. To the reaction mixture was added dichloromethane and the mixture was extracted with water. The aqueous layer was acidified with 2 mol/L hydrochloric acid and extracted with dichloromethane. The organic layer was concentrated under reduced pressure and the obtained residue was purified by high performance liquid chromatography (water-acetonitrile, each containing 0.1% trifluoroacetic acid) to give the title compound (0.30 g, 1.0 mmol, 28%).
MS (ESI) m/z 300 (M+H)⁺

### Reference Example B-2: Synthesis of 1-(benzofuran-2-ylsulfonylamino)cyclopropanecarboxylic acid (B-2)

Using benzofuran-2-ylsulfonyl chloride instead of A-1, an operation similar to Reference Example B-1 was performed to give the title compound (yield 25%).
MS (ESI) m/z 282 (M+H)⁺

### Reference Example B-3: Synthesis of 1-[(5-fluorobenzofuran-2-yl)sulfonyl-isopropyl-amino]cyclopropanecarboxylic acid (B-3)

### (step 1) Synthesis of methyl 1-[(5-fluorobenzofuran-2-yl)sulfonyl-isopropyl-amino]cyclopropanecarboxylate

To a solution of methyl 1-aminocyclopropanecarboxylate (0.30 g, 2.0 mmol) in dichloromethane (5 mL) were added A-1 (0.47 g, 2.0 mmol) and triethylamine (0.70 mL, 5.0 mmol), and the mixture was stirred at room temperature overnight. To the reaction mixture was added water, and the mixture was extracted with dichloromethane. The organic layer was dried over sodium sulfate. The desiccant was filtered off and concentrated under reduced pressure. To the obtained residue was added acetonitrile (10 mL) for dissolution. To the solution were added cesium carbonate (2.6 g, 8.0 mmol) and isopropyl iodide (1.2 mL, 12 mmol) and the mixture was stirred at 65°C for 3 hr. To the reaction mixture was added water, and the mixture was extracted with ethyl acetate. The organic layer was dried over sodium sulfate. The desiccant was filtered off, concentrated under reduced pressure and the obtained residue was purified by silica gel column chromatography (hexane/ethyl acetate) to give the title compound (0.31 g, 0.87 mmol, 43%).
MS (ESI) m/z 356 (M+H)⁺

### (step 2) Synthesis of 1-[(5-fluorobenzofuran-2-yl)sulfonyl-isopropyl-amino]cyclopropanecarboxylic acid (B-3)

To a solution of the compound obtained in step 1 (0.10 g, 0.28 mmol) in tetrahydrofuran (1 mL) was added 2 mol/L aqueous sodium hydroxide solution (1 mL). Methanol was added until the reaction mixture became a monolayer, and the mixture was stirred at room temperature overnight. To the reaction mixture was added dichloromethane, and the mixture was extracted with water. The aqueous layer was adjusted to pH3 by adding 1 mol/L hydrochloric acid and extracted with dichloromethane. The organic layer was dried over sodium hydroxide and the desiccant was filtered off and concentrated under reduced pressure to give the title compound (0.63 g, 0.18 mmol, 65%). MS (ESI) m/z 342 (M+H)⁺

### Reference Example B-4: Synthesis of 1-[benzofuran-2-ylsulfonyl(isopropyl)amino]cyclopropanecarboxylic acid (B-4)

Using benzofuran-2-ylsulfonyl chloride instead of A-1, an operation similar to Reference Example B-3 was performed to give the title compound (yield 27%).
MS (ESI) m/z 324 (M+H)⁺

### Reference Example B-5: Synthesis of 1-(furo[3,2-c]pyridin-2-ylsulfonylamino)cyclopropanecarboxylic acid (B-5)

### (step 1) Synthesis of 4-chlorofuro[3,2-c]pyridine-2-sulfonyl chloride

A solution of 4-chlorofuro[3,2-c]pyridine (3.0 g, 20 mmol) in tetrahydrofuran (80 mL) was cooled to -40°C, 2.5 mol/L n-butyllithium (hexane solution, 9.4 mL, 24 mmol) was added and the mixture was stirred for 1 hr. Sulfur dioxide was blown into the reaction mixture for 30 min while maintaining the temperature at -40°C to -30°C, and the mixture was stirred at room temperature for 1.5 hr. To the reaction mixture was added hexane (100 mL), and the insoluble material was collected by filtration, and dried. To the obtained solid was added dichloromethane (75 mL) and N-chlorosuccinimide (3.1 g, 23 mmol) was added at 0°C, and the mixture was stirred at room temperature for 1 hr. The reaction mixture was washed 5 times with water. The organic layer was dried over sodium sulfate, the desiccant was filtered off, and the solvent was evaporated to give the title compound (3.5 g, 14 mmol, 71%).
MS (ESI) m/z 252 (M+H)⁺
¹H NMR (400 MHz, CDCl₃) δ 8.55 (d, J = 6.0 Hz, 1H), 7.74 (d, J = 1.0 Hz, 1H), 7.59 (dd, J = 6.0, 1.0 Hz, 1H).

### (step 2) Synthesis of methyl 1-[(4-chlorofuro[3,2-c]pyridin-2-yl)sulfonylamino]cyclopropanecarboxylate

To the compound (3.4 g, 14 mmol) obtained in step 1 and methyl 1-aminocyclopropanecarboxylate (2.0 g, 13 mmol) were added dichloromethane (150 mL) and pyridine (24 mL), and the mixture was stirred at room temperature for 1.5 hr. To the reaction mixture was added water, and the organic layer was separated. The organic layer was washed with saturated brine, dried over sodium sulfate. The desiccant was filtered off, and the filtrate was dried under reduced pressure and the obtained residue was purified by silica gel column chromatography (petroleum ether/ethyl acetate) to give the title compound (0.50 g, 1.5 mmol, 11%).

### (step 3) Synthesis of methyl 1-(furo[3,2-c]pyridin-2-ylsulfonylamino)cyclopropanecarboxylate

To the compound (0.50 g, 1.5 mmol) obtained in step 2 and 10% palladium/carbon (0.40 g) were added triethylamine (0.50 mL) and methanol (25 mL), and the mixture was stirred under a hydrogen atmosphere at 35°C overnight. The catalyst was filtered off, and the filtrate was concentrated under reduced pressure and the obtained residue was purified by preparative TLC (dichloromethane/methanol) to give the title compound (0.16 g, 0.52 mmol, 35%).
¹H NMR (300 MHz, CDCl₃) : δ 9.05 (s, 1H), 8.66 (d, J = 6.0 Hz, 1H), 7.53 (d, J = 6.0 Hz, 1H), 7.45 (s, 1H), 5.92 (s, 1H), 3.32 (s, 3H), 1.64-1.61 (m, 2H), 1.58-1.56 (m, 2H).

### (step 4) Synthesis of 1-(furo[3,2-c]pyridin-2-ylsulfonylamino)cyclopropanecarboxylic acid (B-5)

To a solution of the compound (0.16 g, 0.52 mmol) obtained in step 3 in tetrahydrofuran (3 mL) was added aqueous 2 mol/L lithium hydroxide solution (3 mL) and the mixture was stirred at room temperature overnight. Tetrahydrofuran was evaporated under reduced pressure at 35°C, concentrated hydrochloric acid was added to the obtained aqueous solution at 0°C to adjust the mixture to pH4. The insoluble material was collected by filtration, and dried to give the title compound (0.12 g, 0.41 mmol, 80%).
MS (ESI) m/z 283 (M+H)⁺
¹H NMR (400 MHz, DMSO-d₆) : δ 12.56 (s, 1H), 9.38 (s, 1H), 9.06 (s, 1H), 8.62 (d, J = 5.6 Hz, 1H), 7.81 (d, J = 5.6 Hz, 1H), 7.64 (s, 1H), 1.35-1.31 (m, 2H), 1.29-1.24 (m, 2H).

**[Table 4-1]**

| Ref. Ex. No. | structural formula | MS(ESI) *m*/*z* (M+H)⁺ | NMR |
|---|---|---|---|
| C-1 | 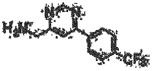 | 254 | ¹H NMR (MHz, CD₃OD): δ 9.26 (s, 1H), 8.40 (d, J=8.1 Hz, 2H), 4.26 (s, 2H). |
| C-2 | 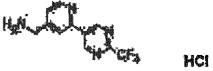 | 255 | ¹H NMR (400 MHz, CD₃OD) δ 9.51 (s, 2H), 8.83 (d, J = 5.0 Hz, 1H), 6.24 (s, 1H), 7.68 (d, J = 5.0 Hz, 1H), 4.31 (s, 2H). |
| C-3 | 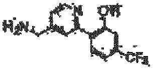 | 269 | ¹H NMR (400 MHz, DMSO-D₆) δ 8.59 (d, J = 5.0 Hz, 1H), 8.30 (s, 1H), 8.27 (d, J = 6.2 Hz, 1H). 7.51 (d, J = 5.0 Hz, 1H, 1H), 7.25 (d, J = 8.2 Hz, 1H), 7.23 (s, 1H), 3.88 (s 2H). |
| C-4 | 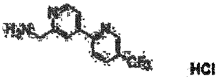 | 254 | ¹H NMR (400 MHz, CD₃OD) δ 9.14 (s, 1H), 8.95 (d, J = 5.4 Mz, 1H), 8.61 (s, 1H), 6.45 (d, J = 5.4 Hz, 1H), 8.44 - 8.40 (m, 2H), 4.56 (s, 2H). |
| C-5 | 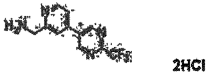 | 255 | ¹H NMR (400 MHz, DMSO-D₆) δ 12.84 (br s, 1H), 9.61 (s, 2H), 6.93 - 6.76 (m, 4H), 8.36 (s, 1H), 8.06 (d, J = 5.2 Hz, 1H), 4.40 -4.28 (m, 2H). |

**[Table 4-3]**

| Ref. Ex. No. | structural formula | MS(ESI) *m*/*z* (M+H)⁺ | NMR |
|---|---|---|---|
| C-6 | 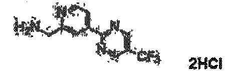 H₂N HN N N CF₃ 2HCl | 255 | ¹H NMR (400 MHz, DMSO-d₆) δ 11.15 (br s, 1H), 9.51 (s, 2H), 8.89 (d, J= 5.0 Hz, 1H), 8.69 (s, 3H), 8.48 (br s, 1H), 8.34 (dd, J = 5.0, 1.6 Hz, 1H), 4.38 - 4.28 (m, 2H). |
| C-7 | 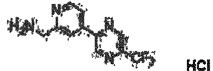 HCl | 255 | ¹H NMR (400 MHz, CD₃OD) δ 9.49 (s, 1H), 9.23 (s, 1H), 8.90 (d, J = 5.4 Hz, 1H), 8.36 (br s, 1H), 8.27 (dd, J = 5.4, 1.6 Hz, 1H), 4.47 (s, 2H). |
| C-8 | 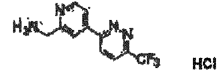 N H2N N N CF3    HCl | 255 | ¹H NMR (400 MHz, CD₃OD) δ 8.92 (d, J = 5.3 Hz, 1H), 8.59 (d, J = 9.0 Hz, 1H), 6.37 (br s, 1H), 8.33 (d, J = 9.0 Hz, 1H), 8.24 (dd, J =5.3, 1.4 Hz, 1H), 4.49 (s, 2H). |
| C-9 | 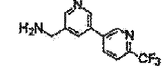 N H2N N CF3 | 254 | ¹H NMR (400 MHz, CD₃OD) δ 9.10 (s, 1H), 8.87 (d, J = 1.6 Hz, 1H), 8.68 (s, 1H), 8.41 (dd, J = 8.0, 1.6 Hz, 1H), 8.27 (s, 1H), 7.99 (d, J = 8.0 Hz, 1H), 4.02 (s, 2H). |
| C-10 | 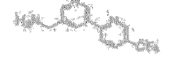 | 254 | ¹H NMR (400 MHz, CD₃OD) δ 9.44 (d, J = 1.8 Hz, 8.95 (s, 3H), 8.68 (d, J = 1.8 Hz, 1H), 8.40 (d, J = 8.4 Hz, 2H), 8.27 - 8.19 (m, 1H, 8.00 (d, J = 8.4 Hz, 2H), 4.27 - 4.23 (m, 2H). |

**[Table 4-3]**

| Ref. Ex. No. | structural formula | MS(ESI) *m*/*z* (M+H)⁺ | NMR |
|---|---|---|---|
| C-11 | 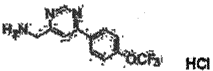 HCl | 270 | ¹H NMR (400 MHz, DMSO-D₆) δ 9.31 (s, 1H), 8.73 (s, 3H), 8.37 - 8.34(m, 3H), 7.61 (d, J = 8.4 Hz, 2H), 4.32 - 4.28 (m, 2H). |
| C-12 | 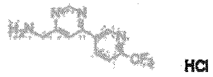 HCl | 255 | ¹H NMR (400 MHz, CD₃OD) δ 9.53 (s, 1H), 9.37 (s, 1H), 8.67 (d, J =8.0 Hz, 1H), 8.30 (s, 1H), 8.04 (d, J = 8.0 Hz, 1H), 4.51 (s, 2H). |
| C-13 | 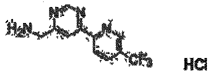 HCl | 255 | ¹HNMR (400 MHz, CD₃OD) δ 9.37 (s, 1H), 9.08 (s, 1H), 8.76 (d, J = 8.2 Hz, 1H), 8.59 (s, 1H, 8.36 (dd, J = 8.2, 1.5 Hz, 1H), 4.49 (s, 2H). |
| C-14 | 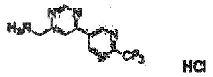 N N H2N N N CF3 HCl | 256 | ¹H NMR (400 MHz, DMSO-D₆) δ 9.78 (s, 2H), 9.46 (d, J = 0.8 Hz, 1H) 8.73 brs, 3H), 8.60 (d, J = 0.8 Hz, 1H), 4.37 (q, J = 5.6 Hz, 2H) |
| C-15 | 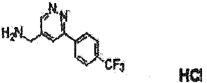 N N H2N CF3 HCl | 254 | ¹H NMR (400 MHz, DMSO-d₆) δ 9.44 (d, J = 1.8 Hz, 1H), 8.95 (s, 3H), 8.68 (d, J = 1.8 Hz, 1H), 8.40 (d, J = 8.4 Hz, 2H), 8.24 - 8.19 (m, 1H), 8.00 (d, J = 8.4 Hz, 2H), 4.27 - 4.23 (m, 2H). |

**[Table 4-4]**

| Ref. Ex. No. | structural formula | MS(ESI) *m*/*z* (M+H)⁺ | NMR |
|---|---|---|---|
| C-16 | 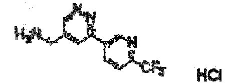 HCl | 255 | ¹H NMR (400 MHz, CD₃OD) δ 9.56 (d, J = 2.0 Hz, 1H), 9.50 (d, J = 2.0 Hz, 1H, 8.84 (dd, J = 8.2, 2.0 Hz, 1H), 8.76 (d, J = 2.0 Hz, 1H), 8.10 (d, J = 8.2 Hz, 1H), 4.50 (s, 2H). |
| C-17 | 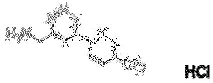 HCl | 255 | ¹H NMR (400 MHz, CD₃OD) δ 9.98 (d, *J* = 2.1 Hz, 1H), 9.22 (s, 1H), 8.75 (s, 3H), 8.59 - 8.52 (m, 3H), 4.54 - 4.50 (m, 2H). |
| C-18 | 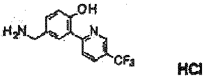 OH H2N N CF3 HCl | 269 | ¹H NMR (400 MHz, DMSO-D₆ δ 9.08 (s, 1H), 8.46 - 8.41 (m, 5H), 8.30 (d, J = 2.0 Hz, 1H), 7.50 (dd, J =8.4, 2.0 Hz, 1H), 7.05 (d, J = 8.4 Hz, 1H), 4.03 - 3.99 (m, 2H). |
| C-19 | 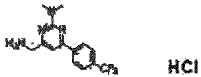 HCl | 297 | - |
| C-20 | 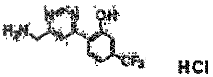 N N OH H2N CF3 HCl | 270 | ¹H NMR (400MHz, DMSO-D₆ δ 9.35 (s, 1H), 8.75 (br s, 3H), 8.47 (s, 1H), 8.26 (d, J = 8.4 Hz, 1H), 7.48 (s, 1H), 7.34 (d, J = 8.4 Hz, 1H), 4.35-4.31 (m, 2H). |

### Reference Example C-1: Synthesis of [6-[4-(trifluoromethyl)phenyl]pyrimidin-4-yl]methylamine (C-1)

### (step 1) Synthesis of 4-methyl-6-[4-(trifluoromethyl)phenyl]pyrimidine

To 4-chloro-6-methylpyrimidine (4.78 g, 37.4 mmol), 4-trifluoromethylphenylboronic acid (8.47 g, 44.6 mmol) and tetrakis(triphenylphosphine)palladium(0) (1.40 g, 1.21 mmol) was added acetonitrile (50 mL). To the reaction mixture was added a solution of sodium carbonate (12.9 g, 121 mmol) in water (9 mL), and the mixture was heated under reflux under an argon atmosphere for 3 hr and added to water. Further, the mixture was extracted with ethyl acetate, and the organic layer was washed with saturated brine and dried over sodium sulfate. The desiccant was filtered off, the solvent was evaporated and the obtained residue was purified by silica gel column chromatography (petroleum ether/ethyl acetate) to give the title compound (6.13 g, 25.8 mmol, 69%).
MS (ESI) m/z 239 (M+H)⁺
¹H NMR (300 MHz, CDCl₃) : δ 9.17 (s, 1H), 8.17 (d, J = 8.1 Hz, 2H), 7.74 (d, J = 8.1 Hz, 2H), 7.60 (s, 1H), 2.61 (s, 3H).

### (step 2) Synthesis of 4-bromomethyl-6-[4-(trifluoromethyl)phenyl]pyrimidine

To the compound obtained in step 1 (5.92 g, 24.7 mmol), N-bromosuccinimide (39.2 g, 223 mmol) and benzoyl peroxide (4.86 g, 20.1 mmol) was added carbon tetrachloride (100 mL), and the mixture was stirred at 100°C overnight. To the reaction mixture was added saturated aqueous sodium hydrogen carbonate, and the mixture was extracted with ethyl acetate. The organic layer was washed with saturated brine and dried over sodium sulfate. The desiccant was filtered off, the solvent was evaporated and the obtained residue was purified by silica gel column chromatography (petroleum ether/ethyl acetate) to give the title compound (0.73 g, 2.3 mmol, 9%).
MS (ESI) m/z 317 (M+H)⁺
¹H NMR (300 MHz, CDCl₃): δ 9.23 (s, 1H), 8.22 (d, J = 8.1 Hz, 2H), 7.91 (s, 1H), 7.80 (d, J = 8.1 Hz, 2H), 4.47 (s, 2H).

### (step 3) Synthesis of [6-[4-(trifluoromethyl)phenyl]pyrimidin-4-yl]methylamine (C-1)

To a solution of the compound obtained in step 2 (0.73 g, 2.3 mmol) in ethanol (10 mL) was added dropwise concentrated aqueous ammonia (15 mL) over 10 min and the mixture was stirred at room temperature for 1 hr. The reaction mixture was concentrated under reduced pressure and the obtained residue was purified by silica gel column chromatography (petroleum ether/ethyl acetate) to give the title compound (0.17 g, 0.69 mmol, 30%).
MS (ESI) m/z 254 (M+H)⁺
¹H NMR (300 MHz, CD₃OD): δ 9.26 (s, 1H), 8.40 (d, J = 8.1 Hz, 2H), 8.13 (s, 1H), 7.88 (d, J = 8.1 Hz, 2H), 4.26 (s, 2H) .

### Reference Example C-2: Synthesis of [2-[2-(trifluoromethyl)pyrimidin-5-yl]-4-pyridyl]methylamine hydrochloride (C-2)

### (step 1) Synthesis of 5-(4,4,5,5-tetramethyl-1,3,2-dioxaborolan-2-yl)-2-(trifluoromethyl)pyrimidine

To 5-bromo-2-trifluoromethylpyrimidine (6.8 g, 30 mmol), bis(pinacolato)diboron (11 g, 40 mmol), potassium acetate (8.8 g, 90 mmol) and 1,1'-bis(diphenylphosphino)ferrocenedichloropalladium(II) (0.10 g, 0.14 mmol) was added 1,4-dioxane (100 mL), and the mixture was stirred at 110°C for 4 hr. Insoluble material was filtered off, the solvent was evaporated and the obtained residue was purified by silica gel column chromatography (petroleum ether/ethyl acetate) to give the title compound (5.0 g, 18 mmol, 61%).
MS (ESI) m/z 275 (M+H)⁺

### (step 2) Synthesis of tert-butyl N-[(2-chloro-4-pyridyl)methyl]carbamate

To (2-chloro-4-pyridyl)methylamine (14 g, 0.10 mol) was added dichloromethane (120 mL) for dissolution, triethylamine (28 mL, 0.20 mol) and di-tert-butyl dicarbonate (26 g, 0.12 mol) were added, and the mixture was stirred at room temperature for 2 hr. The solvent was evaporated from the reaction mixture and the obtained residue was purified by silica gel column chromatography (petroleum ether/ethyl acetate) to give the title compound (22 g, 0.091 mmol, 91%).
MS (ESI) m/z 243 (M+H)⁺

### (step 3) Synthesis of tert-butyl N-[[2-[2-(trifluoromethyl)pyrimidin-5-yl]-4-pyridyl]methyl]carbamate

To the compound obtained in step 1 (1.7 g, 6.0 mmol), the compound obtained in step 2 (2.5 g, 10 mmol), sodium carbonate (2.4 g, 17 mmol) and 1,1'-bis(diphenylphosphino)ferrocenedichloropalladium(II) (30 mg, 0.041 mmol) were added 1,4-dioxane (25 mL) and water (5 mL), and the mixture was stirred at 110°C for 2 hr. Insoluble material was filtered off, ethyl acetate was added to the filtrate and the mixture was successively washed with water and saturated brine and dried over sodium sulfate. The desiccant was filtered off. The solvent was evaporated and the obtained residue was purified by silica gel column chromatography (petroleum ether/ethyl acetate) to give the title compound (1.1 g, 3.0 mmol, 50%).
MS (ESI) m/z 355 (M+H)⁺

### (step 4) Synthesis of [2-[2-(trifluoromethyl)pyrimidin-5-yl]-4-pyridyl]methylamine hydrochloride (C-2)

To the compound obtained in step 3 (1.1 g, 3.0 mmol) was added 4 mol/L hydrogen chloride (dichloromethane solution, 25 mL, 0.10 mol), and the mixture was stirred at room temperature for 1 hr and concentrated under reduced pressure to give the title compound (0.68 g, 2.3 mmol, 79%).
MS (ESI) m/z 255 (M+H)⁺
¹H NMR (400 MHz, CD₃OD) δ 9.51 (s, 2H), 8.83 (d, J = 5.0 Hz, 1H), 8.24 (s, 1H), 7.68 (d, J = 5.0 Hz, 1H), 4.31 (s, 2H).

### Reference Example C-3: Synthesis of 2-[4-(aminomethyl)-2-pyridyl]-5-(trifluoromethyl)phenol (C-3)

### (step 1) Synthesis of 2-[2-methoxy-4-(trifluoromethyl)phenyl]pyridine-4-carbonitrile

To 2-chloropyridine-4-carbonitrile (26 g, 0.19 mol) and [2-methoxy-4-(trifluoromethyl)phenyl]boronic acid (44 g, 0.23 mol), sodium carbonate (40 g, 0.38 mol) and 1,1'-bis(diphenylphosphino)ferrocenedichloropalladium(II) (7.0 g, 9.5 mmol) were added N,N-dimethylformamide (400 mL) and water (100 mL), and the mixture was stirred at 100°C for 4 hr. Insoluble material was filtered off, ethyl acetate was added to the filtrate and the mixture was washed successively with water and saturated brine and dried over sodium sulfate. The desiccant was filtered off. The solvent was evaporated and the obtained residue was purified by silica gel column chromatography (petroleum ether/ethyl acetate) to give the title compound (40 g, 0.14 mol, 76%).
MS (ESI) m/z 279 (M+H)⁺
¹H NMR (400 MHz, DMSO-d₆) δ 8.96 (d, J = 5.0 Hz, 1H), 8.30 (d, J = 1.2 Hz, 1H), 7.95 (d, J = 8.2 Hz, 1H), 7.86 (dd, J = 5.0, 1.2 Hz, 1H), 7.48 (s, 1H), 7.45 (d, J = 8.2 Hz, 1H), 3.97 (s, 3H) .

### (step 2) Synthesis of 2-[2-hydroxy-4-(trifluoromethyl)phenyl]pyridine-4-carbonitrile

To a solution of the compound obtained in step 1 (40 g, 0.14 mol) in dichloromethane (4 L) was added 1 mol/L boron tribromide (dichloromethane solution, 0.25 L, 0.25 mol) at - 70°C, and the mixture was stirred at -20°C for 3 hr. The reaction mixture was poured into ice water, and extracted with dichloromethane. The organic layer was washed successively with saturated aqueous sodium hydrogen carbonate and saturated brine, and dried over sodium sulfate. The desiccant was filtered off. The solvent was evaporated and the obtained residue was purified by silica gel column chromatography (petroleum ether/ethyl acetate) to give the title compound (24 g, 0.091 mol, 63%).
MS (ESI) m/z 265 (M+H)⁺

### (step 3) Synthesis of 2-[4-(aminomethyl)-2-pyridyl]-5-(trifluoromethyl)phenol (C-3)

To a solution of the compound obtained in step 2 (24 g, 91 mmol) in ethanol (1.0 L) was added 10% palladium/carbon (3.0 g), and the mixture was stirred under pressurized hydrogen atmosphere at 50psi at 70°C for 3 hr. The catalyst was filtered off, the filtrate was concentrated under reduced pressure and the obtained residue was purified by silica gel column chromatography (petroleum ether/ethyl acetate/dichloromethane) to give the title compound (15 g, 56 mmol, 62%).
MS (ESI) m/z 269 (M+H)⁺
¹H NMR (400 MHz, DMSO-d₆) δ 8.59 (d, J = 5.0 Hz, 1H), 8.30 (s, 1H), 8.27 (d, J = 8.2 Hz, 1H), 7.51 (d, J = 5.0 Hz, 1H), 7.25 (d, J = 8.2 Hz, 1H), 7.23 (s, 1H), 3.88 (s, 2H).

### Reference Example C-4: Synthesis of [4-[5-(trifluoromethyl)-2-pyridyl]-2-pyridyl]methylamine hydrochloride (C-4)

### (step 1) Synthesis of tert-butyl N-[(4-chloro-2-pyridyl)methyl]carbamate

To a solution of (4-chloro-2-pyridyl)methylamine (28 g, 0.20 mol) in dichloromethane (250 mL) were added triethylamine (56 mL, 0.40 mol) and di-tert-butyl dicarbonate (52 g, 0.24 mol), and the mixture was stirred at room temperature for 2 hr. The solvent was evaporated and the obtained residue was purified by silica gel column chromatography (petroleum ether/ethyl acetate) to give the title compound (45 g, 0.18 mol, 92%).
MS (ESI) m/z 243 (M+H)⁺

### (step 2) Synthesis of [4-[5-(trifluoromethyl)-2-pyridyl]-2-pyridyl]methylamine hydrochloride (C-4)

To the compound obtained in step 1 (21 g, 88 mmol), E-4 (36 g, 0.12 mol), 1,1'-bis(diphenylphosphino)ferrocene (5.6 g, 10 mmol), palladium acetate (1.1 g, 4.9 mmol), cesium carbonate (66 g, 0.20 mol) and copper(I) chloride (10 g, 0.10 mol) was added N,N-dimethylformamide (450 mL), and the mixture was stirred at 100°C for 4 hr. Insoluble material was filtered off, ethyl acetate was added to the filtrate and the mixture was washed successively with water and saturated brine. The organic layer was dried over sodium sulfate and the desiccant was filtered off. The solvent was evaporated and to the obtained residue was added 4 mol/L hydrogen chloride (dichloromethane solution, 10 mL, 40 mmol), and the mixture was concentrated under reduced pressure. To the obtained residue was added dichloromethane, and insoluble material was collected by filtration and dried to give the title compound (22 g, 74 mmol, 84%).
MS (ESI) m/z 254 (M+H)⁺
¹H NMR (400 MHz, CD₃OD) δ 9.14 (s, 1H), 8.95 (d, J = 5.4 Hz, 1H), 8.61 (s, 1H), 8.45 (d, J = 5.4 Hz, 1H), 8.44 - 8.40 (m, 2H), 4.56 (s, 2H).

### Reference Example C-5: Synthesis of [4-[2-(trifluoromethyl)pyrimidin-5-yl3-2-pyridyl]methylamine 2 hydrochloride (C-5)

### (step 1) Synthesis of tert-butyl N-[[4-(4,4,5,5-tetramethyl-1,3,2-dioxaborolan-2-yl)-2-pyridyl]methyl]carbamate

To the compound obtained in Reference Example C-4, step 1 (11 g, 47 mmol), bis(pinacolato)diboron (14 g, 56 mmol), potassium acetate (3.8 g, 14 mmol) and 1,1'-bis(diphenylphosphino)ferrocenedichloropalladium (II) (3.4 g, 4.2 mmol) was added N,N-dimethylformamide (200 mL), and the mixture was stirred at 100°C for 2 hr. Insoluble material was filtered off, ethyl acetate was added to the filtrate and the mixture was washed successively with water and saturated brine. The organic layer was dried over sodium sulfate and the desiccant was filtered off. The solvent was evaporated to give the title compound as a crude purified product (13 g).

### (step 2) Synthesis of [4-[2-(trifluoromethyl)pyrimidin-5-yl]-2-pyridyl]methylamine 2 hydrochloride (C-5)

To the crude purified product (0.60 g) obtained in step 1, 5-bromo-2-(trifluoromethyl)pyrimidine (0.50 g, 2.2 mmol), sodium carbonate (0.47 g, 4.4 mmol) and 1,1'-bis(diphenylphosphino)ferrocenedichloropalladium(II) (90 mg, 0.11 mmol) were added N,N-dimethylformamide (16 mL) and water (4 mL), and the mixture was stirred at 100°C for 2 hr. Insoluble material was filtered off, ethyl acetate was added to the filtrate and the mixture was washed successively with water and saturated brine, and dried over sodium sulfate. The desiccant was filtered off. The solvent was evaporated and the obtained residue was purified by silica gel column chromatography (petroleum ether/ethyl acetate). To the obtained compound were added dichloromethane (20 mL) and 6 mol/L hydrogen chloride (dichloromethane solution, 10 mL, 60 mmol), and the mixture was stirred at room temperature for 1 hr and concentrated under reduced pressure to give the title compound (0.36 g, 1.2 mmol, 57%).
MS (ESI) m/z 255 (M+H)⁺
¹H NMR (400 MHz, DMSO-d₆) δ 12.84 (br s, 1H), 9.61 (s, 2H), 8.93 - 8.78 (m, 4H), 8.36 (s, 1H), 8.06 (d, J = 5.2 Hz, 1H), 4.40 - 4.28 (m, 2H).

Using corresponding commercially available reagents, an operation similar to Reference Example C-5 was performed to synthesize Reference Examples C-6 and C-7 described in Table 4.

### Reference Example C-8: Synthesis of [4-[6-(trifluoromethyl)pyridazin-3-yl]-2-pyridyl]methylamine hydrochloride (C-8)

### (step 1) Synthesis of 3-chloro-6-(trifluoromethyl)pyridazine

To 3-(trifluoromethyl)-1H-pyridazin-6-one (1.1 g, 6.7 mmol) was added phosphorus oxychloride (10 mL) and the mixture was stirred at 100°C for 2.5 hr, and concentrated under reduced pressure. To the obtained residue were added dichloromethane and water, and the mixture was stirred at room temperature for 5 min. After stirring, the mixture was alkalified with potassium carbonate and partitioned. The organic layer was washed with saturated brine, dried over sodium sulfate and the desiccant was filtered off. The solvent was evaporated and the obtained residue was purified by silica gel column chromatography (petroleum ether/ethyl acetate) to give the title compound (0.77 g, 4.2 mmol, 63%).
MS (ESI) m/z 182 (M+H)⁺
¹H NMR (400 MHz, CDCl₃) δ 7.82 (d, J = 8.8 Hz, 1H), 7.74 (d, J = 8.8 Hz, 1H).

### (step 2) Synthesis of [4-[6-(trifluoromethyl)pyridazin-3-yl]-2-pyridyl]methylamine hydrochloride (C-8)

Using the compound obtained in step 1 instead of 5-bromo-2-(trifluoromethyl)pyrimidine, an operation similar to Reference Example C-5, step 2 was performed to give the title compound (yield 54%).
MS (ESI) m/z 255 (M+H)⁺
¹H NMR (400 MHz, CD₃OD) δ 8.92 (d, J = 5.3 Hz, 1H), 8.59 (d, J = 9.0 Hz, 1H), 8.37 (br s, 1H), 8.33 (d, J = 9.0 Hz, 1H), 8.24 (dd, J = 5.3, 1.4 Hz, 1H), 4.49 (s, 2H).

### Reference Example C-9: Synthesis of [5-[6-(trifluoromethyl)-3-pyridyl]-3-pyridyl]methylamine (C-9)

### (step 1) Synthesis of 3-[2-(trifluoromethyl)pyridin-5-yl]benzonitrile

To 5-bromo-2-trifluoromethylpyridine (4.2 g, 19 mmol), (5-cyano-3-pyridyl)boronic acid (3.3 g, 22 mmol), sodium carbonate (3.9 g, 37 mmol) and 1,1'-bis(diphenylphosphino)ferrocenedichloropalladium(II) (0.69 g, 0.94 mmol) were added N,N-dimethylformamide (160 mL) and water (40 mL), and the mixture was stirred at 110°C for 2 hr. Insoluble material was filtered off, ethyl acetate was added to the filtrate and the mixture was washed successively with water and saturated brine and dried over sodium sulfate. The desiccant was filtered off. The solvent was evaporated and the obtained residue was purified by silica gel column chromatography (petroleum ether/ethyl acetate) to give the title compound (4.3 g, 17 mmol, 93%).
¹H NMR (400 MHz, CDCl₃) δ 9.07 (s, 1H), 8.99 (s, 1H), 8.96 (s, 1H), 8.19 (s, 1H), 8.10 (d, J = 6.0 Hz, 1H), 7.88 (d, J = 8.4 Hz, 1H).

### (step 2) Synthesis of [5-[6-(trifluoromethyl)-3-pyridyl]-3-pyridyl]methylamine (C-9)

To the compound obtained in step 1 (4.3 g, 17 mmol) and cobalt chloride(II) hexahydrate (2.1 g, 17 mmol) were added tetrahydrofuran (40 mL) and water (20 mL). At 0°C, to the reaction mixture was added sodium tetrahydroborate (1.3 g, 34 mmol), and the mixture was stirred at room temperature for 2 hr. Then, 3 mol/L hydrochloric acid was added to adjust to pH 1. After stirring at room temperature for 1 hr, tetrahydrofuran was evaporated under reduced pressure from the reaction mixture, and aqueous ammonia was added to adjust to pH 8 - 9. The reaction mixture was extracted with ethyl acetate, and the organic layer was washed with saturated brine and dried over sodium sulfate. The desiccant was filtered off. The solvent was evaporated and the obtained residue was purified by high performance liquid chromatography (water-acetonitrile) to give the title compound (0.45 g, 1.7 mmol, 10%).
MS (ESI) m/z 254 (M+H)⁺
¹H NMR (400 MHz, CD₃OD) δ 9.10 (s, 1H), 8.87 (d, J = 1.6 Hz, 1H), 8.68 (s, 1H), 8.41 (dd, J = 8.0, 1.6 Hz, 1H), 8.27 (s, 1H), 7.99 (d, J = 8.0 Hz, 1H), 4.02 (s, 2H).

### Reference Example C-10: Synthesis of [5-[5-(trifluoromethyl)-2-pyridyl]-3-pyridyl]methylamine (C-10)

### (step 1) Synthesis of 5-[5-(trifluoromethyl)-2-pyridyl]pyridine-3-carbonitrile

To 2-bromo-5-(trifluoromethyl)pyridine (4.0 g, 18 mmol), 5-cyano-3-pyridylboronic acid (3.1 g, 21 mmol), sodium carbonate (3.8 g, 35 mmol) and 1,1'-bis(diphenylphosphino)ferrocenedichloropalladium(II) (0.66 g, 0.90 mmol) were added N,N-dimethylformamide (160 mL) and water (40 mL), and the mixture was stirred at 110°C for 2 hr. Insoluble material was filtered off, ethyl acetate was added to the filtrate and the mixture was washed successively with water and saturated brine and dried over sodium sulfate. The desiccant was filtered off. The solvent was evaporated and the obtained residue was purified by silica gel column chromatography (petroleum ether/ethyl acetate) to give the title compound (3.3 g, 13 mmol, 73%).
MS (ESI) m/z 250 (M+H)⁺
¹H NMR (400 MHz, CDCl₃) δ 9.44 (s, 1H), 9.03 (s, 1H), 8.98 (s, 1H), 8.71 (s, 1H), 8.12 (d, J = 8.4 Hz, 1H), 7.94 (d, J = 8.4 Hz, 1H).

### (step 2) Synthesis of [5-[5-(trifluoromethyl)-2-pyridyl]-3-pyridyl]methylamine (C-10)

To a solution of the compound obtained in step 1 (3.0 g, 12 mmol) in methanol (30 mL) was added nickel (50 mg), and the mixture was stirred under a hydrogen atmosphere at room temperature for 2 hr. The catalyst was filtered off, the solvent was evaporated and the obtained residue was purified by high performance liquid chromatography (water - acetonitrile) to give the title compound (0.42 g, 1.7 mmol, 14%).
MS (ESI) m/z 254 (M+H)⁺
¹H NMR (400 MHz, CD₃OD) δ 9.74 (s, 1H), 9.60 (s, 1H), 9.18 (s, 1H), 9.16 (s, 1H), 8.48 - 8.42 (m, 2H), 4.58 (s, 2H).

### Reference Example C-11: Synthesis of [6-[4-(trifluoromethoxy)phenyl]pyrimidin-4-yl]methylamine hydrochloride (C-11)

### (step 1) Synthesis of 4-chloro-6-[4-(trifluoromethoxy)phenyl]pyrimidine

To 4,6-dichloropyrimidine (131 g, 879 mmol), 4-(trifluoromethoxy)phenylboronic acid (200 g, 970 mol), potassium carbonate (244 g, 1.77 mol) and tetrakis(triphenylphosphine)palladium(0) (21.5 g, 18.6 mmol) were added 1,4-dioxane (3.0 L) and water (200 mL) and the mixture was stirred at 105°C for 6 hr. Insoluble material was filtered off, the filtrate was concentrated under reduced pressure and the obtained residue was purified by silica gel column chromatography (petroleum ether/ethyl acetate) to give the title compound (78.0 g, 284 mmol, 32%).
MS (ESI) m/z 275 (M+H)⁺
¹H NMR (400 MHz, CDCl₃) δ 9.04 (s, 1H), 8.13 (d, J = 8.6 Hz, 2H), 7.73 (s, 1H), 7.36 (d, J = 8.6 Hz, 2H).

### (step 2) Synthesis of 6-[4-(trifluoromethoxy)phenyl]pyrimidine-4-carbonitrile

To the compound obtained in step 1 (1.0 g, 3.6 mmol), sodium cyanide (0.22 g, 4.4 mmol) and 1,4-diazabicyclo[2.2.2]octane (41 mg, 0.37 mmol) were added water (2 mL) and dimethyl sulfoxide (6 mL), and the mixture was stirred at 38°C for 7 hr. The reaction mixture was added to water, and extracted with ethyl acetate. The organic layer was washed with saturated brine and dried over sodium sulfate. The desiccant was filtered off and the solvent was evaporated. To the obtained residue was added petroleum ether, and the insoluble material was collected by filtration and dried to give the title compound (0.80 g, 3.0 mmol, 83%).
MS (ESI) m/z 266 (M+H)⁺
¹H NMR (300 MHz, CDCl₃) δ 9.38 (s, 1H), 8.20 (d, J = 8.5 Hz, 2H), 8.04 (s, 1H), 7.42 (d, J = 8.5 Hz, 2H).

### (step 3) Synthesis of [6-[4-(trifluoromethoxy)phenyl]pyrimidin-4-yl]methylamine hydrochloride (C-11)

To a solution of the compound obtained in step 2 (10 g, 38 mmol) in acetic acid (150 mL) was added 10% palladium/carbon (0.15 g), and the mixture was stirred under a hydrogen atmosphere at room temperature for 1.5 hr. The catalyst was filtered off, to the filtrate was added dichloromethane and the mixture was washed with aqueous sodium hydrogen carbonate solution. The organic layer was dried over sodium sulfate. The desiccant was filtered off. The solvent was evaporated and to the obtained residue was added dichloromethane (120 mL) for dissolution. Di-tert-butyl dicarbonate (11 g, 49 mmol) and triethylamine (10 mL, 72 mmol) were added and the mixture was stirred at room temperature for 30 min. The solvent was evaporated from the reaction mixture and the obtained residue was purified by silica gel column chromatography (petroleum ether/ethyl acetate). To the obtained compound was added 4 mol/L hydrogen chloride (dichloromethane solution, 10 mL, 40 mmol), and the mixture was stirred at room temperature for 20 min and concentrated under reduced pressure to give the title compound (4.1 g, 13 mmol, 35%).
MS (ESI) m/z 270 (M+H)⁺
¹H NMR (400 MHz, DMSO-d₆) δ 9.31 (s, 1H), 8.73 (s, 3H), 8.37 - 8.34 (m, 3H), 7.61 (d, J = 8.4 Hz, 2H), 4.32 - 4.28 (m, 2H).

### Reference Example C-12: Synthesis of [6-[6-(trifluoromethyl)-3-pyridyl]pyrimidin-4-yl]methylamine hydrochloride (C-12)

### (step 1) Synthesis of 4-chloro-6-[6-(trifluoromethyl)-3-pyridyl]pyrimidine

To 2,4-dichloropyrimidine (6.0 g, 40 mmol), [(6-trifluoromethyl)-3-pyridyl]boronic acid (8.5 g, 44 mmol), potassium carbonate (11 g, 81 mmol) and tetrakis(triphenylphosphine)palladium(0) (1.2 g, 1.0 mmol) were added 1,4-dioxane (150 mL) and water (15 mL), and the mixture was stirred with heating at 110°C for 4 hr. The reaction mixture was filtered, and the filtrate was diluted with ethyl acetate and washed successively with water and saturated brine. The organic layer was dried over sodium sulfate and the desiccant was filtered off. The solvent was evaporated and the obtained residue was purified by silica gel column chromatography (petroleum ether/ethyl acetate) to give the title compound (3.5 g, 14 mmol, 34%).
¹H NMR (400 MHz, DMSO-d₆) δ 9.37 (s, 1H), 9.14 (s, 1H), 8.61 (dd, J =8.4, 1.6 Hz, 1H), 7.87 (d, J = 8.4 Hz, 1H), 7.85 (s, 1H).

### (step 2) Synthesis of 6-[6-(trifluoromethyl)-3-pyridyl]pyrimidine-4-carbonitrile

To the compound obtained in step 1 (3.5 g, 14 mmol), sodium cyanide (0.79 g, 16 mmol) and 1,4-diazabicyclo[2.2.2]octane (0.15 g, 1.2 mmol) were added water (9 mL) and dimethyl sulfoxide (25 mL), and the mixture was stirred at room temperature for 4 hr. The reaction mixture was added to water, and extracted with diethyl ether. The organic layer was washed with saturated brine and dried over sodium sulfate. The desiccant was filtered off and the solvent was evaporated. The obtained residue was purified by silica gel column chromatography (petroleum ether/ethyl acetate) to give the title compound (2.2 g, 8.8 mmol, 65%).
¹H NMR (400 MHz, CDCl₃) δ 9.49 (s, 1H), 9.44 (s, 1H), 8.67 (d, J = 8.4 Hz, 1H), 8.15 (s, 1H), 7.92 (d, J = 8.4 Hz, 1H).

### (step 3) Synthesis of [6-[6-(trifluoromethyl)-3-pyridyl]pyrimidin-4-yl]methylamine hydrochloride (C-12)

To a solution of the compound obtained in step 2 (2.2 g, 8.8 mmol) in acetic acid (120 mL) was added 10% palladium/carbon (660 mg), and the mixture was stirred under a hydrogen atmosphere at room temperature for 1.5 hr. The catalyst was filtered off, dichloromethane was added to the filtrate and the mixture was washed with aqueous sodium carbonate solution. The organic layer was dried over sodium sulfate, the desiccant was filtered off, and the solvent was evaporated. To the obtained residue was added 4 mol/L hydrogen chloride (dichloromethane solution, 10 mL, 40 mmol) and the mixture was concentrated under reduced pressure. To the obtained residue was added dichloromethane, and the insoluble material was collected by filtration and dried to give the title compound (1.0 g, 3.4 mmol, 40%).
MS (ESI) m/z 255 (M+H)⁺
¹H NMR (400 MHz, CD₃OD) δ 9.53 (s, 1H), 9.37 (s, 1H), 8.67 (d, J = 8.0 Hz, 1H), 8.30 (s, 1H), 8.04 (d, J = 8.0 Hz, 1H), 4.51 (s, 2H).

### Reference Example C-13: Synthesis of [6-[5-(trifluoromethyl)-2-pyridyl]pyrimidin-4-yl]methylamine hydrochloride (C-13)

### (step 1) Synthesis of 6-methyl-2-[5-trifluoromethyl]-2-pyridyl-1,3, 6,2-dioxaazaborocane-4, 8-dione

To 2-bromo-5-trifluoromethylpyridine (7.79 g, 34.4 mmol) and triisopropyl borate (9.6 mL, 40 mmol) was added tetrahydrofuran (100 mL), and the mixture was cooled to -78°C and 2.5 mol/L n-butyllithium (hexane solution, 13.8 mL, 34.4 mmol) was added dropwise. After stirring at -78°C for 1 hr, the mixture was heated to 23°C and further stirred for 3 hr. A solution of N-methyliminodiacetic acid (8.61 g, 58.5 mmol) in dimethyl sulfoxide (68 mL) was separately prepared, placed in a three-necked flask connected to a dropping funnel and a distillation apparatus, and heated to an inside temperature of 115°C. The above reaction mixture was transferred to the dropping funnel and added dropwise over about 1 hr while controlling the addition rate such that the inside temperature was 110 - 120°C. During this time, tetrahydrofuran was rapidly evaporated. After completion of the dropwise addition, the temperature was decreased to 50°C, dimethyl sulfoxide was evaporated under reduced pressure (250 mTorr). The residue was washed with diethyl ether and the obtained solid was dried under reduced pressure to give the title compound (3.43 g, 11.4 mmol, 33%).
MS (ESI) m/z 303 (M+H)⁺
¹H NMR (400 MHz, CD₃OD): δ 8.92 (s, 1H), 8.17 (dd, J = 8.0, 1.6 Hz, 1H), 7.65 (d, J = 8.0 Hz, 1H), 4.13 (s, 2H), 4.12 (s, 2H), 3.00 (s, 3H).

### (step 2) Synthesis of 4-chloro-6-[5-(trifluoromethyl)-2-pyridyl]pyrimidine

To 4-chloro-1H-pyrimidin-6-one (3.0 g, 23 mmol), the compound obtained in step 1 (9.0 g, 30 mmol), 1,1'-bis(diphenylphosphino)ferrocene (1.3 g, 2.3 mmol), palladium acetate (0.26 g, 1.2 mmol), cesium carbonate (15 g, 46 mmol) and copper(I) chloride (2.3 g, 23 mmol) was added N,N-dimethylformamide (100 mL), and the mixture was stirred at 100°C overnight. Insoluble material was filtered off, and the filtrate was concentrated under reduced pressure. To the obtained residue was added phosphorus oxychloride (40 mL), and the mixture was stirred at 105°C for 3 hr. The reaction mixture was concentrated under reduced pressure, dichloromethane was added to the obtained residue and the mixture was successively washed with water and saturated brine. The organic layer was dried over sodium sulfate and the desiccant was filtered off. The solvent was evaporated and the obtained residue was purified by silica gel column chromatography (petroleum ether/ethyl acetate) to give the title compound (0.55 g, 2.1 mmol, 9%).
MS (ESI) m/z 260 (M+H)⁺
¹H NMR (400 MHz, CDCl₃) δ 9.09 (s, 1H), 8.98 (s, 1H), 8.63 (d, J = 8.0 Hz, 1H), 8.49 (s, 1H), 8.13 (dd, J = 8.0, 1.6 Hz, 1H).

### (step 3) Synthesis of 6-[5-(trifluoromethyl)-2-pyridyl]pyrimidine-4-carbonitrile

To sodium cyanide (0.17 g, 3.4 mmol) and 1,4-diazabicyclo[2.2.2]octane (24 mg, 0.21 mmol) was added water (15 mL) for dissolution, a solution of the compound obtained in step 2 (0.55 g, 2.1 mmol) in dimethyl sulfoxide (50 mL) was added and the mixture was stirred at room temperature for 7 hr. The reaction mixture was added to water, and extracted with dichloromethane. The organic layer was dried over sodium sulfate and the desiccant was filtered off. The solvent was evaporated and the obtained residue was purified by silica gel column chromatography (petroleum ether/ethyl acetate) to give the title compound (0.35 g, 1.4 mmol, 66%).
MS (ESI) m/z 251 (M+H)⁺
¹H NMR (400 MHz, CDCl₃) δ 9.44 (s, 1H), 9.02 (s, 1H), 8.80 (s, 1H), 8.68 (d, J = 8.8 Hz, 1H), 8.17 (dd, J = 8.0, 2.0 Hz, 1H).

### (step 4) Synthesis of [6-[5-(trifluoromethyl)-2-pyridyl]pyrimidin-4-yl]methylamine hydrochloride (C-13)

To a solution of the compound obtained in step 3 (0.32 g, 1.3 mmol) in acetic acid (15 mL) was added 10% palladium/carbon (90 mg), and the mixture was stirred under a hydrogen atmosphere at room temperature for 30 min. The catalyst was filtered off, the filtrate was adjusted to pH8 by adding an aqueous sodium carbonate solution, and extracted with dichloromethane. The organic layer was dried over sodium sulfate, the desiccant was filtered off, and the solvent was evaporated. To the obtained residue was added dichloromethane (15 mL) for dissolution. Triethylamine (0.60 mL, 4.3 mmol) and di-tert-butyl dicarbonate (0.34 g, 1.5 mmol) were added, and the mixture was stirred at room temperature for 1 hr. The reaction mixture was washed with water and dried over sodium sulfate. The desiccant was filtered off. The solvent was evaporated and the obtained residue was purified by silica gel column chromatography (petroleum ether/ethyl acetate). To the obtained compound were added dichloromethane (2 mL) and 4 mol/L hydrogen chloride (dichloromethane solution, 10 mL, 40 mmol), and the mixture was stirred at room temperature for 30 min and concentrated under reduced pressure to give the title compound (0.10 g, 0.34 mmol, 27%).
MS (ESI) m/z 255 (M+H)⁺
¹H NMR (400 MHz, CD₃OD) δ 9.37 (s, 1H), 9.08 (s, 1H), 8.76 (d, J = 8.2 Hz, 1H), 8.59 (s, 1H), 8.36 (dd, J = 8.2, 1.5 Hz, 1H), 4.49 (s, 2H).

### Reference Example C-14: Synthesis of [6-[2-(trifluoromethyl)pyrimidin-5-yl]pyrimidin-4-yl]methylamine hydrochloride (C-14)

### (step 1) Synthesis of 4-chloro-6-[2-(trifluoromethyl)pyrimidin-5-yl]pyrimidine

To the compound obtained in Reference Example C-2, step 1 (2.5 g, 9.1 mmol), 4,6-dichloropyrimidine (2.4 g, 16 mmol), potassium carbonate (3.3 g, 24 mmol) and tetrakis(triphenylphosphine)palladium(0) (0.68 g, 0.60 mmol) were added 1,4-dioxane (150 mL) and water (15 mL), and the mixture was stirred at 110°C for 2 hr. Insoluble material was filtered off, ethyl acetate was added to the filtrate and the mixture was washed with saturated brine and dried over sodium sulfate. The desiccant was filtered off. The solvent was evaporated and the obtained residue was purified by silica gel column chromatography (petroleum ether/ethyl acetate) to give the title compound (0.87 g, 3.3 mmol, 37%).
MS (ESI) m/z 261 (M+H)⁺
¹H NMR (400 MHz, CDCl₃) δ 9.55 (s, 2H), 9.18 (s, 1H), 7.87 (s, 1H).

### (step 2) Synthesis of 6-[2-(trifluoromethyl)pyrimidin-5-yl]pyrimidine-4-carbonitrile

To a solution of sodium cyanide (0.19 g, 4.0 mmol) and 1,4-diazabicyclo[2.2.2]octane (41 mg, 0.37 mmol) in water (2 mL) was added a solution of the compound obtained in step 1 (0.85 g, 3.3 mmol) in dimethyl sulfoxide (6 mL) and the mixture was stirred at 38°C for 7 hr. The reaction mixture was added to water (30 mL) and the mixture was extracted with ethyl acetate. The organic layer was washed with saturated brine and dried over sodium sulfate. The desiccant was filtered off. The solvent was evaporated, petroleum ether was added to the obtained residue and insoluble material was collected by filtration and dried to give the title compound (0.55 g, 2.2 mmol, 67%).
MS (ESI) m/z 252 (M+H)⁺

### (step 3) Synthesis of [6-[2-(trifluoromethyl)pyrimidin-5-yl]pyrimidin-4-yl]methylamine hydrochloride (C-14)

To a solution of the compound obtained in step 2 (0.55 g, 2.2 mmol) in acetic acid (20 mL) was added 10% palladium/carbon (20 mg), and the mixture was stirred under a hydrogen atmosphere at room temperature for 1.5 hr. The catalyst was filtered off, dichloromethane was added to the filtrate and the mixture was washed with saturated aqueous sodium hydrogen carbonate. The organic layer was dried over sodium sulfate and the desiccant was filtered off. The solvent was evaporated, 4 mol/L hydrogen chloride (dichloromethane solution, 20 mL, 80 mmol) was added to the obtained residue, and the mixture was stirred at room temperature for 30 min and concentrated under reduced pressure. To the obtained residue was added dichloromethane, insoluble material was collected by filtration and dried to give the title compound (0.18 g, 0.62 mmol, 28%).
MS (ESI) m/z 256 (M+H)⁺
¹H NMR (400 MHz, DMSO-d₆) δ 9.78 (s, 2H), 9.46 (d, J = 0.8 Hz, 1H), 8.73 (brs, 3H), 8.60 (d, J = 0.8 Hz, 1H), 4.37 (q, J = 5.6 Hz, 2H)

### Reference Example C-15: Synthesis of [6-[4-(trifluoromethyl)phenyl]pyridazin-4-yl]methylamine hydrochloride (C-15)

### (step 1) Synthesis of tert-butyl N-[(3,6-dichloropyridazin-4-yl)methyl]carbamate

To N-Boc-glycine (10 g, 57 mmol), 3,6-dichloropyridazine (5.0 g, 34 mmol) and silver (I) nitrate (0.57 g, 3.4 mmol) were added water (60 mL) and trifluoroacetic acid (0.50 mL, 6.7 mmol). The reaction mixture was heated to 70°C, a solution of ammonium persulfate (14 g, 61 mmol) in water (20 mL) was gradually added over 20 min. After stirring for 30 min, isopropyl acetate (200 mL) was added to the reaction mixture. After cooling to 20°C, the mixture was adjusted to pH 9 with aqueous ammonia and partitioned. The aqueous layer was extracted with isopropyl acetate (50 mL), and the combined organic layer was washed with 1 mol/L aqueous sodium hydrogen carbonate solution. The organic layer was dried over magnesium sulfate, the desiccant was filtered off and hexane was added. The resulting insoluble material was collected by filtration and dried to give the title compound (2.0 g, 7.2 mmol, 21%).
MS (ESI) m/z 278 (M+H)⁺

### (step 2) Synthesis of tert-butyl N-[[3-chloro-6-[6-(trifluoromethyl)phenyl]pyridazin-4-yl]methyl]carbamate

To the compound obtained in step 1 (0.90 g, 3.2 mmol), 4-(trifluoromethyl)phenylboronic acid (0.58 g, 3.0 mmol), sodium carbonate (2.1 g, 20 mmol) and 1,1'-bis(diphenylphosphino)ferrocenedichloropalladium(II) (0.20 g, 0.28 mmol) were added 1,4-dioxane (20 mL) and water (5 mL), and the mixture was stirred with heating at 110°C for 2 hr. To the reaction mixture was added ethyl acetate, and the mixture was washed successively with water and saturated brine. The organic layer was dried over sodium sulfate, the desiccant was filtered off and the solvent was evaporated. The obtained residue was purified by silica gel column chromatography (petroleum ether/ethyl acetate) to give the title compound (0.60 g, 1.5 mmol, 51%).
MS (ESI) m/z 388 (M+H)⁺

### (step 3) Synthesis of [6-[4-(trifluoromethyl)phenyl]pyridazin-4-yl]methylamine hydrochloride (C-15)

To a solution of the compound obtained in step 2 (0.60 g, 1.5 mmol) in acetic acid (20 mL) was added 10% palladium/carbon (22 mg), and the mixture was stirred under a hydrogen atmosphere at room temperature for 3 hr. The catalyst was filtered off, ethyl acetate was added to the filtrate and the mixture was washed with saturated brine. The organic layer was dried over sodium sulfate, the desiccant was filtered off, and the solvent was evaporated. To the obtained residue was added 4 mol/L hydrogen chloride (dichloromethane solution, 5 mL, 20 mmol), and the mixture was concentrated under reduced pressure. The obtained residue was purified by high performance liquid chromatography (water-acetonitrile) to give the title compound (0.16 g, 0.49 mmol, 32%).
MS (ESI) m/z 254 (M+H)⁺
¹H NMR (400 MHz, DMSO-d₆) δ 9.44 (d, J = 1.8 Hz, 1H), 8.95 (s, 3H), 8.68 (d, J = 1.8 Hz, 1H), 8.40 (d, J = 8.4 Hz, 2H), 8.24 - 8.19 (m, 1H), 8.00 (d, J = 8.4 Hz, 2H), 4.27 - 4.23 (m, 2H).

Using corresponding commercially available reagents, an operation similar to Reference Example C-15 was performed to synthesize Reference Example C-16 described in Table 4.

### Reference Example C-17: Synthesis of [5-[5-(trifluoromethyl)-2-pyridyl]pyridazin-3-yl]methylamine hydrochloride (C-17)

### (step 1) Synthesis of 3-chloro-5-[5-(trifluoromethyl)-2-pyridyl]pyridazine

To 4-chloro-1H-pyridazin-6-one (4.5 g, 35 mmol), E-4 (13 g, 41 mmol), 1,1'-bis(diphenylphosphino)ferrocene (1.9 g, 3.5 mmol), palladium acetate (0.39 g, 1.7 mmol), cesium carbonate (23 g, 69 mmol) and copper(I) chloride (3.4 g, 35 mmol) was added N,N-dimethylformamide (100 mL), and the mixture was stirred at 105°C for 6 hr. Insoluble material was filtered off, and the filtrate was concentrated under reduced pressure. To the obtained residue was added phosphorus oxychloride (30 mL), and the mixture was stirred at 105°C for 2 hr. The reaction mixture was concentrated under reduced pressure, dichloromethane was added to the obtained residue and the mixture was successively washed with water and saturated brine. The organic layer was dried over sodium sulfate and the desiccant was filtered off. The solvent was evaporated and the obtained residue was purified by silica gel column chromatography (petroleum ether/ethyl acetate) to give the title compound (2.1 g, 8.1 mmol, 23%).
MS (ESI) m/z 260 (M+H)⁺
¹H NMR (400 MHz, DMSO-d₆) δ 9.96 (d, J = 1.6 Hz, 1H), 9.20 (s, 1H), 8.62 - 8.51 (m, 3H).

### (step 2) Synthesis of 5-[5-(trifluoromethyl)-2-pyridyl]pyridazine-3-carbonitrile

The compound obtained in step 1 (1.0 g, 3.9 mmol) was dissolved in N,N-dimethylformamide (20 mL). Zinc cyanide (0.27 g, 2.3 mmol), tris(dibenzylideneacetone)dipalladium(0) (0.14 g, 0.15 mmol) and 1,1'-bis(diphenylphosphino)ferrocene (0.16 g, 0.29 mmol) were added to the solution under a nitrogen atmosphere and the mixture was stirred with heating at 110°C for 4.5 hr. To the reaction mixture was added dichloromethane and the mixture was washed successively with water and saturated brine. The organic layer was dried over sodium sulfate and the desiccant was filtered off. The solvent was evaporated and the obtained residue was purified by silica gel column chromatography (petroleum ether/ethyl acetate) to give the title compound (0.30 g, 1.2 mmol, 31%).
MS (ESI) m/z 251 (M+H)⁺

### (step 3) Synthesis of [5-[5-(trifluoromethyl)-2-pyridyl]pyridazin-3-yl]methylamine hydrochloride (C-17)

To a solution of the compound obtained in step 2 (0.60 g, 2.4 mmol) in acetic acid (15 mL) was added 10% palladium/carbon (0.18 g), and the mixture was stirred under a hydrogen atmosphere at 25°C for 1.5 hr. The catalyst was filtered off, and the filtrate was concentrated under reduced pressure. Dichloromethane (30 mL), triethylamine (3.5 mL, 25 mmol) and di-tert-butyl dicarbonate (2.0 g, 9.2 mmol) were added to the obtained residue, and the mixture was stirred at room temperature for 1 hr. To the reaction mixture was added dichloromethane and the mixture was washed with water. The organic layer was dried over sodium sulfate and the desiccant was filtered off. The solvent was evaporated and the obtained residue was purified by silica gel column chromatography (petroleum ether/ethyl acetate). To the obtained compound was added 4 mol/L hydrogen chloride (dichloromethane solution, 25 mL, 0.10 mol), and the mixture was stirred at room temperature for 1 hr and concentrated under reduced pressure to give the title compound (0.50 g, 1.7 mmol, 71%).
MS (ESI) m/z 255 (M+H)⁺
¹H NMR (400 MHz, DMSO-d₆) δ 9.98 (d, J = 2.1 Hz, 1H), 9.22 (s, 1H), 8.75 (s, 3H), 8.59 - 8.52 (m, 3H), 4.54 - 4.50 (m, 2H).

### Reference Example C-18: Synthesis of 4-(aminomethyl)-2-[5-(trifluoromethyl)-2-pyridyl]phenol hydrochloride (C-18)

### (step 1) Synthesis of (5-cyano-2-methoxy-phenyl)boronic acid

To 3-Bromo-4-methoxybenzonitrile (7.7 g, 36 mmol) and triisopropyl borate (14 g, 73 mmol) was added tetrahydrofuran (150 mL) for dissolution, and 2.5 mol/L n-butyllithium (hexane solution, 22 mL, 55 mmol) was gradually added over 20 min at - 78°C. After stirring at -78°C for 2 hr, 7% phosphoric acid (100 mL) was added to the reaction mixture and the mixture was heated to room temperature. The reaction mixture was partitioned, dichloromethane was added to the organic layer, and the mixture was extracted with 5% aqueous sodium hydroxide solution (200 mL). The aqueous layer was washed with diethyl ether, adjusted to pH 2.5 by adding 85% phosphoric acid and insoluble material was collected by filtration. The obtained solid was washed with water and dried to give the title compound (5.1 g, 29 mmol, 79%).
MS (ESI) m/z 178 (M+H)⁺
¹H NMR (300 MHz, DMSO-d₆) δ 8.03 (s, 2H), 7.86 - 7.78 (m, 2H), 7.13 (d, J = 11.6 Hz, 1H), 3.85 (s, 3H).

### (step 2) Synthesis of 4-methoxy-3-[5-(trifluoromethyl)-2-pyridyl]benzonitrile

To the compound obtained in step 1 (1.1 g, 6.0 mmol), 2-bromo-5-(trifluoromethyl)pyridine (1.2 g, 5.5 mmol), sodium carbonate (1.2 g, 11 mmol) and 1,1'-bis(diphenylphosphino)ferrocenedichloropalladium(II) (0.20 g, 0.27 mmol) were added N,N-dimethylformamide (16 mL) and water (4 mL), and the mixture was stirred at 100°C for 2 hr. Insoluble material was filtered off, ethyl acetate was added to the filtrate and the mixture was washed successively with water and saturated brine, and dried over sodium sulfate. The desiccant was filtered off. The solvent was evaporated and the obtained residue was purified by silica gel column chromatography (petroleum ether/ethyl acetate) to give the title compound (1.4 g, 5.1 mmol, 92%).
MS (ESI) m/z 279 (M+H)⁺

### (step 3) Synthesis of tert-butyl N-[[4-hydroxy-3-[5-(trifluoromethyl)-2-pyridyl]phenyl]methyl]carbamate

To the compound obtained in step 2 (1.5 g, 5.4 mmol) and cobalt chloride(II) hexahydrate (0.70 g, 5.4 mmol) were added tetrahydrofuran (60 mL) and water (40 mL). To the reaction mixture was added sodium tetrahydroborate (0.51 g, 14 mmol) at 0°C. The mixture was stirred at room temperature for 3 hr, then 3 mol/L hydrochloric acid (150 mL) was added. Tetrahydrofuran was evaporated under reduced pressure from the reaction mixture, and the mixture was adjusted to pH 8 - 9 with aqueous ammonia. The reaction mixture was extracted with ethyl acetate, and the organic layer was washed with saturated brine and dried over sodium sulfate. The desiccant was filtered off. The solvent was evaporated and to the obtained residue was added dichloromethane (5 mL) for dissolution and 1 mol/L boron tribromide (dichloromethane solution, 10 mL, 10 mmol) was added at -70°C. After stirring at room temperature for 7 hr, the reaction mixture was adjusted to pH 8 with saturated aqueous sodium carbonate solution and extracted with ethyl acetate. The organic layer was washed with saturated brine and dried over sodium sulfate. The desiccant was filtered off. The solvent was evaporated and to the obtained residue was added dichloromethane (5 mL) for dissolution, di-tert-butyl dicarbonate (0.46 g, 2.1 mmol) was added and the mixture was stirred at room temperature for 30 min. The solvent was evaporated from the reaction mixture and the obtained residue was purified by silica gel column chromatography (dichloromethane) to give the title compound (0.28 g, 0.76 mmol, 14%).
MS (ESI) m/z 369 (M+H)⁺
¹H NMR (400 MHz, CDCl₃) δ 13.59 (s, 1H), 8.80 (s, 1H), 8.08 - 8.02 (m, 2H), 7.75 (s, 1H), 7.29 (dd, J = 8.8, 0.8 Hz, 1H), 7.02 (d, J = 8.8 Hz, 1H), 4.86 (s, 1H), 4.45-4.15 (m, 2H), 1.47 (s, 9H).

### (step 4) Synthesis of 4-(aminomethyl)-2-[5-(trifluoromethyl)-2-pyridyl]phenol hydrochloride (C-18)

To the compound obtained in step 3 (0.28 g, 0.76 mmol) were added dichloromethane (3 mL) and 4 mol/L hydrogen chloride (dichloromethane solution, 10 mL, 40 mmol), and the mixture was stirred at room temperature for 30 min and concentrated under reduced pressure to give the title compound (0.21 g, 0.67 mmol, 88%).
MS (ESI) m/z 269 (M+H)⁺
¹H NMR (400 MHz, DMSO-d₆) δ 9.08 (s, 1H), 8.46 - 8.41 (m, 5H), 8.30 (d, J = 2.0 Hz, 1H), 7.50 (dd, J = 8.4, 2.0 Hz, 1H), 7.05 (d, J = 8.4 Hz, 1H), 4.03 - 3.99 (m, 2H).

### Reference Example C-19: Synthesis of 4-(aminomethyl)-N,N-dimethyl-6-[4-(trifluoromethyl)phenyl]pyrimidine-2-amine hydrochloride (C-19)

### (step 1) Synthesis of tert-butyl N-[[2-benzylsulfanyl-6-[4-(trifluoromethyl)phenyl]pyrimidin-4-yl]methyl]carbamate

To a solution of tert-butyl N-prop-2-ynylcarbamate (5.0 g, 32 mmol) in tetrahydrofuran (100 mL) were added 4-trifluoromethylbenzoylchloride (4.3 mL, 29 mmol), dichlorobis(triphenylphosphine)palladium(II) (0.25 g, 0.36 mmol) and copper(I) iodide (0.25 g, 1.3 mmol) and the mixture was stirred at room temperature for 5 min. Triethylamine (5.5 mL, 39 mmol) was added and the mixture was stirred for 30 min. Using a small amount of silica gel, insoluble material was filtered off, and the filtrate was concentrated under reduced pressure. To the obtained residue was added acetonitrile (300 mL) for dissolution and S-benzylisothiourea hydrochloride (7.5 g, 37 mmol) and potassium carbonate (6.0 g, 43 mmol) were added. The reaction mixture was stirred at 70°C overnight, dichloromethane was added and the mixture was washed with water. The organic layer was dried over sodium sulfate and the desiccant was filtered off. The solvent was evaporated and the obtained residue was purified by silica gel column chromatography (hexane/ethyl acetate) to give the title compound (4.0 g, 8.4 mmol, 26%).
MS (ESI) m/z 476 (M+H)⁺

### (step 2) Synthesis of tert-butyl N-[[2-benzylsulfonyl-6-[4-(trifluoromethyl)phenyl]pyrimidin-4-yl]methyl]carbamate

To a solution of the compound obtained in step 1 (1.08 g, 2.27 mmol) in dichloromethane (20 mL) was added 3-chloroperbenzoic acid (1.56 g, 9.08 mmol) at 0°C, and the mixture was stirred at room temperature for 6 hr. To the reaction mixture was added saturated aqueous sodium hydrogen carbonate, and the mixture was extracted with dichloromethane. The organic layer was dried over sodium sulfate. The desiccant was filtered off and the solvent was evaporated to give the title compound (1.01 g, 1.99 mmol, 88%).
MS (ESI) m/z 508 (M+H)⁺

### (step 3) Synthesis of 4-(aminomethyl)-N,N-dimethyl-6-[4-(trifluoromethyl)phenyl]pyrimidine-2-amine hydrochloride (C-19)

To a solution of the compound obtained in step 2 (152 mg, 0.30 mmol) in N,N-dimethylformamide (2 mL) was added 2 mol/L dimethylamine (tetrahydrofuran solution, 0.30 mL, 0.60 mmol), and the mixture was stirred with heating at 100°C for 10 min in a microwave reactor. The reaction mixture was concentrated under reduced pressure, water was added and the mixture was extracted with dichloromethane. The organic layer was dried over sodium sulfate and the desiccant was filtered off. The solvent was evaporated, 4 mol/L hydrochloric acid (1,4-dioxane solution, 2 mL) was added to the obtained residue, and the mixture was stirred at room temperature for 1.5 hr. The reaction mixture was concentrated under reduced pressure to give the title compound (96 mg, 0.29 mmol, 96%).
MS (ESI) m/z 297 (M+H)⁺

### Reference Example C-20: Synthesis of 2-[6-(aminomethyl)pyrimidin-4-yl]-5-(trifluoromethyl)phenol hydrochloride (C-20)

### (step 1) Synthesis of 4-chloro-6-[2-methoxy-4-(trifluoromethyl)phenyl]pyrimidine

To 4,6-dichloropyrimidine (15.4 g, 103 mmol), 2-methoxy-4-(trifluoromethyl)phenylboronic acid (25.0 g, 114 mmol), potassium carbonate (28.5 g, 206 mmol) and tetrakistriphenylphosphinepalladium(0) (6 g) were added 1,4-dioxane (500 mL) and water (50 mL), and the mixture was stirred under a nitrogen atmosphere at 110°C for 4 hr. The insoluble material was filtered off, the filtrate was added to water and the mixture was extracted with ethyl acetate. The organic layer was washed with water and saturated brine and dried over sodium sulfate. The desiccant was filtered off, the filtrate was concentrated under reduced pressure and the obtained residue was purified by silica gel column chromatography (petroleum ether/ethyl acetate) to give the title compound (20 g, 69.4 mmol, 67%).
MS (ESI) m/z 289 (M+H)⁺

### (step 2) Synthesis of 6-[2-methoxy-4-(trifluoromethyl)phenyl]pyrimidine-4-carbonitrile

To the compound obtained in step 1 (20.0 g, 69.4 mmol), sodium cyanide (4.08 g, 83.3 mmol) and 1,4-diazabicyclo[2.2.2]octane (778 mg, 6.94 mmol) were added dimethylsulfoxide (140 mL) and water (45 mL), and the mixture was stirred at room temperature for 4 hr. The reaction mixture was added to water and the mixture was extracted with diethyl ether. The organic layer was washed with saturated brine and dried over sodium sulfate. The desiccant was filtered off, the filtrate was concentrated under reduced pressure and the obtained residue was purified by silica gel column chromatography (petroleum ether/ethyl acetate) to give the title compound (16.0 g, 83%).
¹H NMR (400 MHz, DMSO-d₆) δ 9.39 (d, J = 1.2 Hz, 1H), 8.40 (d, J = 1.2 Hz, 1H), 8.25 (d, J = 8.0 Hz, 1H), 7.40 (d, J = 8.0 Hz, 1H), 7.28 (s, 1H), 4.04 (s, 3H).

### (step 3) Synthesis of [6-[2-methoxy-4-(trifluoromethyl)phenyl]pyrimidin-4-yl]methylamine

To a solution of the compound obtained in step 2 (14.5 g, 51.9 mmol) in acetic acid (150 mL) was added 10% palladium/carbon (4.5 g), and the mixture was stirred under a hydrogen atmosphere at room temperature for 1.5 hr. The catalyst was filtered off, dichloromethane was added to the filtrate and the mixture was washed with aqueous sodium carbonate solution and dried over sodium sulfate. The desiccant was filtered off and the mixture was concentrated under reduced pressure to give the title compound (12.0 g, 42.4 mmol, 82%).

### (step 4) Synthesis of 2-[6-(aminomethyl)pyrimidin-4-yl]-5-(trifluoromethyl)phenol hydrochloride (C-20)

A solution of the compound obtained in step 3 (12.0 g, 42.4 mmol) in dichloromethane (250 mL) was cooled to -78°C, 1 mol/L boron tribromide (dichloromethane solution, 170 mL, 170 mmol) was added, and the mixture was stirred at room temperature for 4 hr. The reaction mixture was adjusted to pH 8 with saturated aqueous sodium carbonate solution and extracted with dichloromethane. The organic layer was dried over sodium sulfate. The desiccant was filtered off, and the filtrate was concentrated under reduced pressure. To the obtained residue (9.60 g) was added dichloromethane (100 mL) for dissolution, di-tert-butyldicarbonate (11.7 g, 53.5 g) and triethylamine (10 mL, 72 mmol) were added and the mixture was stirred at room temperature for 30 min. The reaction mixture was concentrated under reduced pressure and the obtained residue was purified by silica gel column chromatography (petroleum ether/ethyl acetate) to give the title compound in a protected form. To the obtained protected form was added 4 mol/L hydrochloric acid (dichloromethane solution, 100 mL), the mixture was stirred at room temperature for 20 min and concentrated under reduced pressure to give the title compound (3.7 g, 34%).
MS (ESI) m/z 270 (M+H)⁺
¹H NMR (400 MHz, DMSO-d₆) δ 9.35 (s, 1H), 8.75 (br s, 3H), 8.47 (s, 1H), 8.26 (d, J = 8.4 Hz, 1H), 7.48 (s, 1H), 7.34 (d, J = 8.4 Hz, 1H), 4.35-4.31 (m, 2H).

**Table 5**

| Ref. Ex. No | strucural formula | MS(ESI)*mz* (M+H)⁺ | NMR |
|---|---|---|---|
| D-1 | 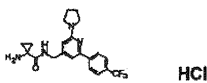 HCl | 405 | - |

### Reference Example D-1: Synthesis of 1-amino-N-[[2-pyrrolidin-1-yl-6-[4-(trifluoromethyl)phenyl]-4-pyridyl]methyl]cyclopropanecarboxamide hydrochloride (D-1)

### (step 1) Synthesis of 2-pyrrolidin-1-yl-6-[4-(trifluoromethyl)phenyl]pyridine-4-carbonitrile

To a solution of 2,6-dichloropyridine-4-carbonitrile (0.52 g, 3.0 mmol) in ethanol (30 mL) was added pyrrolidine (0.25 mL, 3.0 mmol) and the mixture was stirred at 70°C for 1 hr. The reaction mixture was concentrated under reduced pressure, 4-(trifluoromethyl)phenylboronic acid (0.68 g, 3.6 mmol), palladium acetate (33 mg, 0.15 mmol), XPhos (0.14 g, 0.30 mmol), cesium carbonate (3.9 g, 12 mmol) and 1,4-dioxane (30 mL) were added to the obtained residue, and the mixture was heated to 100°C and stirred for 1 hr. Insoluble material was filtered through celite, the filtrate was concentrated under reduced pressure and the obtained residue was purified by silica gel column chromatography (hexane/ethyl acetate) to give the title compound (0.45 g, 1.4 mmol, 47%).

### (step 2) Synthesis of [2-pyrrolidin-1-yl-6-[4-(trifluoromethyl)phenyl]pyridin-4-yl]methylamine (C-21)

A solution of the compound obtained in step 1 (0.50 g, 1.6 mmol) in diethyl ether (15 mL) was cooled to 0°C, lithium aluminum hydride (0.15 g, 3.1 mmol) was added and the mixture was stirred at room temperature for 30 min. To the reaction mixture was added water (0.38 mL) and the mixture was filtered through celite. The filtrate was concentrated under reduced pressure to give the title compound (0.47 g, 1.5 mmol, 93%).
MS (ESI) m/z 322 (M+H)⁺

### (step 3) Synthesis of tert-butyl N-[1-[[2-pyrrolidin-1-yl-6-[4-(trifluoromethyl)phenyl]-4-pyridyl]methylcarbamoyl]cyclopropyl]carbamate

To the compound obtained in step 2 (0.47 g, 1.5 mmol), 1-(tert-butoxycarbonylamino)cyclopropanecarboxylic acid (0.29 g, 1.5 mmol), WSC hydrochloride (0.56 g, 2.9 mmol) and 1-hydroxy-7-azabenzotriazole (0.40 g, 2.9 mmol) were added dichloromethane (15 mL) and triethylamine (0.60 mL, 4.4 mmol) and the mixture was stirred at room temperature for 1 hr. The reaction mixture was concentrated under reduced pressure and the obtained residue was purified by silica gel column chromatography (hexane/ethyl acetate) to give the title compound (0.23 g, 0.46 mmol, 32%).

### (step 4) Synthesis of 1-amino-N-[[2-pyrrolidin-1-yl-6-[4-(trifluoromethyl)phenyl]-4-pyridyl]methyl]cyclopropanecarboxamide hydrochloride (D-1)

To the compound obtained in step 3 (0.23 g, 0.46 mmol) was added 4 mol/L hydrochloric acid (1,4-dioxane solution, 5 mL, 20 mmol), and the mixture was stirred at room temperature for 1 hr. The reaction mixture was concentrated under reduced pressure to give the title compound (0.20 g, 0.46 mmol, 100%).
MS (ESI) m/z 405 (M+H)⁺

**[Table 6-1]**

| **Ex. No.** | **structural formula** | **compound name** |
|---|---|---|
| 1 | 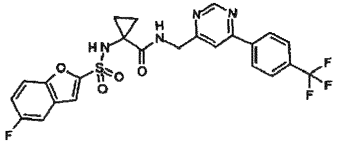 | 1-[(5-fluorobenzofuran-2-yl)sulfonylamino]-N-[[6-[4-(trifluoromethyl)phenyl]pyrimidin-4-yl]methyl]cyclopropanecarboxamide |
| 2 | 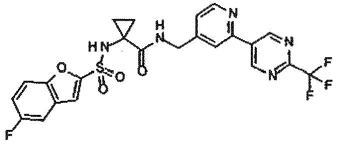 | 1-[(5-fluorobenzofuran-2-yl)sulfonylamino]-N-[[2-[2-(trifluoromethyl)pyrimidin-5-yl]-4-pyridyl]methyl]cyclopropanecarboxamide |
| 3 | 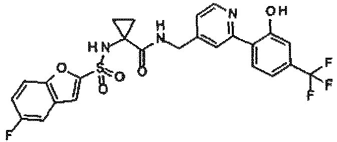 | 1-[(5-fluorobenzofuran-2-yl)sulfonylamino]-N-[[2-[2-hydroxy-4-(trifluoromethyl)phenyl]-4-pyridyl]methyl]cyclopropanecarboxamide |
| 4 | 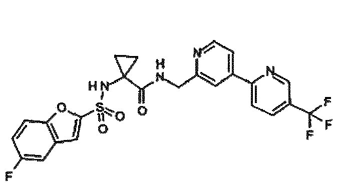 | 1-[(5-fluorobenzofuran-2-yl)sulfonylamino]-N-[[4-[5-(trifluoromethyl)-2-pyridyl]-2-pyridyl]methyl]cyclopropanecarboxamide |
| 5 | 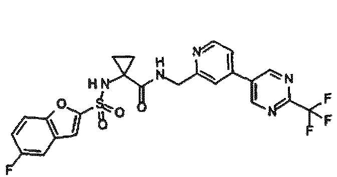 | 1-[(5-fluorobenzofuran-2-yl)sulfonylamino]-N-[[4-[2-(trifluoromethyl)pyrimidin-5-yl]-2-pyridyl]methyl]cyclopropanecarboxamide |
| 6 | 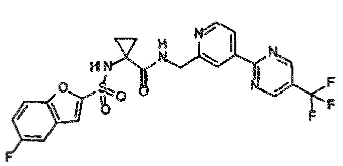 | 1-[(5-fluorobenzofuran-2-yl)sulfonylamino]-N-[[4-[5-(trifluoromethyl)pyrimidin-2-yl]-2-pyridyl]methyl]cyclopropanecarboxamide |
| 7 | 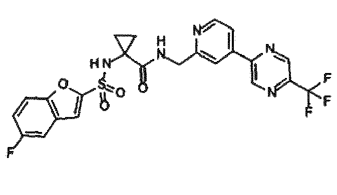 | 1-[(5-fluorobenzofuran-2-yl)sulfonylamino]-N-[[4-[5-(trifluoromethyl)pyrazin-2-yl]-2-pyridyl]methyl]cyclopropanecarboxamide |
| 8 | 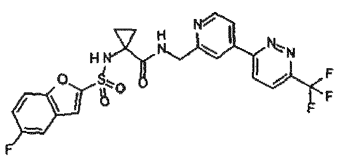 | 1-[(5-fluorobenzofuran-2-yl)sulfonylamino]-N-[[4-[6-(trifluoromethyl)pyridazin-3-yl]-2-pyridyl]methyl] cyclopropanecarboxamide |
| 9 | 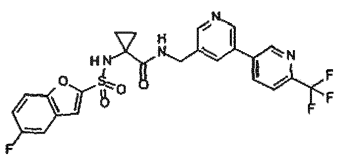 | 1-[(5-fluorobenzofuran-2-yl)sulfonylamino]-N-[[5-[6-(trifluoromethyl)-3-pyridyl]-3-pyridyl] methyl] cyclopropanecarboxamide |
| 10 | 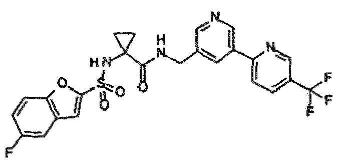 | 1-[(5-fluorobenzofuran-2-yl)sulfonylamino]-N-[[5-[5-(trifluoromethyl)-2-pyridyl]-3-pyridyl]methyl]cyclopropanecarboxamide |

**[Table 6-2]**

| **Ex. No.** | **structural formula** | **compound name** |
|---|---|---|
| 11 | 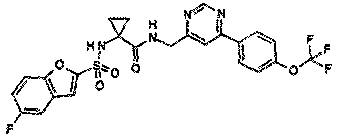 | 1-[(5-fluorobenzofuran-2-yl)sulfonylamino]-N-[[6-[4-(trifluoromethoxy)phenyl]pyrimidin-4-yl]methyl]cyclopropanecarboxamide |
| 12 | 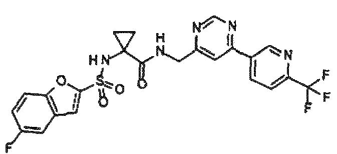 | 1-[(5-fluorobenzofuran-2-yl)sulfonylamino]-N-[[6-[6-(trifluoromethyl)-3-pyridyl]pyrimidin-4-yl]methyl]cyclopropanecarboxamide |
| 13 | 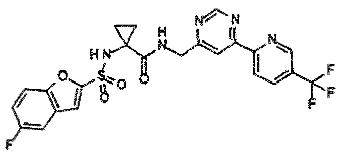 | 1-[(5-fluorobenzofuran-2-yl)sulfonylamino]-N-[[6-[5-(trifluoromethyl)-2-pyridyl]pyrimidin-4-yl]methyl]cyclopropanecarboxamide |
| 14 | 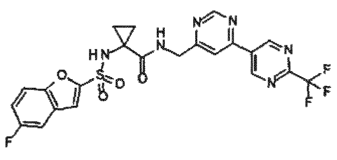 | 1-[(5-fluorobenzofuran-2-yl)sulfonylamino]-N-[[6-[2-(trifluoromethyl)pyrimidin-5-yl]pyrimidin-4-yl]methyl]cyclopropanecarboxamide |
| 15 | 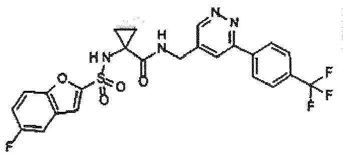 | 1-[(5-fluorobenzofuran-2-yl)sulfonylamino]-N-[[6-[4-(trifluoromethyl)phenyl]pyridazin-4-yl]methyl]cyclopropanecarboxamide |
| 16 | 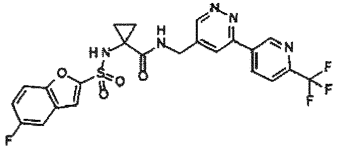 | 1-[(5-fluorobenzofuran-2-yl)sulfonylamino]-N-[[6-[6-(trifluoromethyl)-3-pyridyl]pyridazin-4-yl]methyl]cyclopropanecarboxamide |
| 17 | 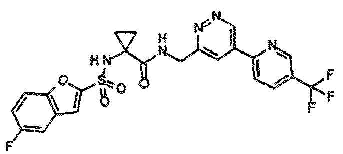 | 1-[(5-fluorobenzofuran-2-yl)sulfonylamino]-N-[[5-[5-(trifluoromethyl)-2-pyridyl]pyridazin-3-yl]methyl]cyclopropanecarboxamide |
| 18 | 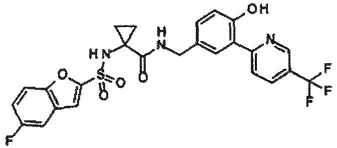 | 1-[(5-fluorobenzofuran-2-yl)sulfonylamino]-N-[[4-hydroxy-3-[5-(trifluoromethyl)-2-pyridyl]-phenyl]methyl]cyclopropanecarboxamide |
| 19 | 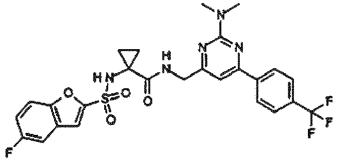 | N-[[2-(dimethylamino)-6-[4-(trifluoromethyl)phenyl]pyrimidin-4-yl]methyl]-1-[(5-fluorobenzofuran-2-yl)-sulfonylamino]cyclopropanecarboxamide |
| 20 | 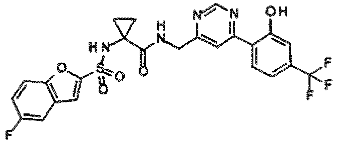 | 1-[(5-fluorobenzofuran-2-yl)sulfonylamino]-N-[[6-[2-hydroxy-4-(trifluoromethyl)-phenyl]pyrimidin-4-yl]methyl]cyclopropanecarboxamide |

**[Table 6-3]**

| **Ex. No.** | **structural formula** | **compound name** |
|---|---|---|
| 21 | 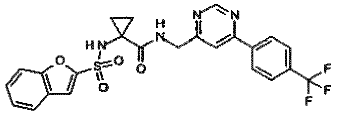 | 1-(benzofuran-2-ylsulfonylamino)-N-[[6-[4-(trifluoromethyl) phenyl] pyrimidin-4-yl]methyl]cyclopropanecarboxamide |
| 22 | 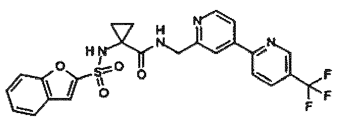 | 1-(benzofuran-2-ylsulfonylamino)-N-[[4-[5-(trifluoromethyl)-2-pyridyl]-2-pyridyl]-methyl]cyclopropanecarboxamide |
| 23 | 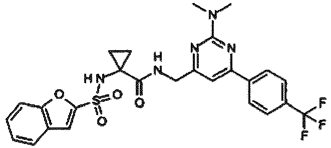 | 1-(benzofuran-2-ylsulfonylamino)-N-[[2-(dimethylamino)-6-[4-(trifluoromethyl)phenyl]pyrimidin-4-yl]methyl]cyclopropanecarboxamide |
| 24 | 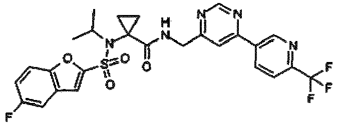 | 1-[(5-fluorobenzofuran-2-yl)sulfonyl-isopropyl-amino]-N-[[6-[6-(trifluoromethyl)-3-pyridyl]pyrimidin-4-yl]methyl]cyclopropanecarboxamide |
| 25 | 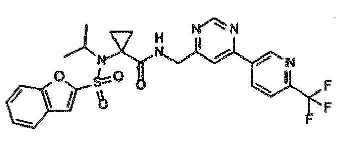 | 1-[benzofuran-2-ylsulfonyl(isopropyl)amino]-N-[[6-[6-(trifluoromethyl)-3-pyridyl]pyrimidin-4-yl]methyl]cyclopropanecarboxamide |
| 26 | 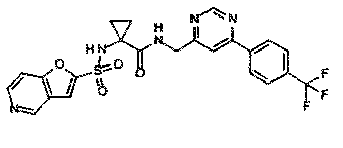 | 1-(furo[3,2-c]pyridin-2-ylsulfonylamino)-N-[[6-[4-(trifluoromethyl)phenyl]pyrimidin-4-yl]methyl]cyclopropanecarboxamide |
| 27 | 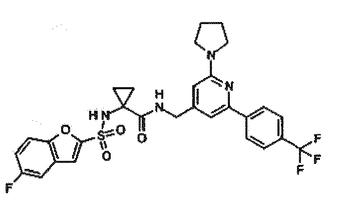 | 1-[(5-fluorobenzofuran-2-yl)sulfonylamino]-N-[[2-pyrrolidin-1-yl-6-[4-(trifluoromethyl)phenyl]-4-pyridyl]methyl]-cyclopropanecarboxamide |
| 28 | 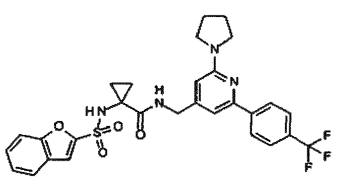 | 1-(benzofuran-2-ylsulfonylamino)-N-[[2-pyrrolidin-1-yl-6-[4-(trifluoromethyl)phenyl]-4-pyridyl]methyl]cyclopropanecarboxamide |
| 29 | 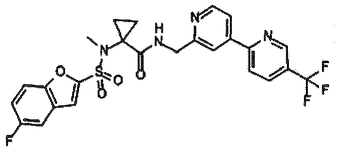 | 1-[(5-fluorobenzofuran-2-yl)sulfonyl-methyl-amino]-N-[[4-[5-(trifluoromethyl)-2-pyridyl]-2-pyridyl]methyl]cyclopropanecarboxamide |

### Example 1: Synthesis of 1-[(5-fluorobenzofuran-2-yl)sulfonylamino]-N-[[6-[4-(trifluoromethyl)phenyl]pyrimidin-4-yl]methyl]cyclopropanecarboxamide (1)

To B-1 (29 mg, 0.10 mmol), C-1 (28 mg, 0.10 mmol), WSC hydrochloride (38 mg, 0.20 mmol) and 1-hydroxy-7-azabenzotriazole (26 mg, 0.20 mmol) were added dichloromethane (1 mL) and triethylamine (54 µL, 0.40 mmol) and the mixture was stirred at room temperature for 30 min. The reaction mixture was concentrated under reduced pressure and the obtained residue was purified by high performance liquid chromatography (water-acetonitrile, each containing 0.1% trifluoroacetic acid) to give the title compound (24 mg, 0.045 mmol, 45%).
MS (ESI) m/z 535 (M+H)⁺
¹H NMR (400 MHz, DMSO-d₆) δ 9.53 (s, 1H), 9.22 (d, J = 1.3 Hz, 1H), 8.66 (t, J = 5.8 Hz, 1H), 8.40 (d, J = 8.2 Hz, 2H), 8.07 (d, J = 1.3 Hz, 1H), 7.97 (d, J = 8.2 Hz, 2H), 7.77 (dd, J = 9.1, 4.0 Hz, 1H), 7.67 (d, J = 0.9 Hz, 1H), 7.60 (dd, J = 8.5, 2.7 Hz, 1H), 7.39 (ddd, J = 9.2, 9.1, 2.7 Hz, 1H), 4.44 (d, J = 5.8 Hz, 2H), 1.30 - 1.25 (m, 2H), 0.97 - 0.93 (m, 2H).

Using corresponding commercially available reagents and compounds of Reference Examples, an operation similar to Example 1 was performed to synthesize Example 2 - Example 26 described in Table 6.

### Example 27: Synthesis of 1-[(5-fluorobenzofuran-2-yl)sulfonylamino]-N-[[2-pyrrolidin-1-yl-6-[4-(trifluoromethyl)phenyl]-4-pyridyl]methyl]cyclopropanecarboxamide (27)

To a solution of D-1 (40 mg, 0.085 mmol) in dichloromethane (1 mL) were added triethylamine (34 µL, 0.26 mmol) and A-1 (20 mg, 0.085 mmol) and the mixture was stirred at room temperature overnight. The reaction mixture was concentrated under reduced pressure and the obtained residue was purified by high performance liquid chromatography (water-acetonitrile, each containing 0.1% trifluoroacetic acid) to give the title compound (5.4 mg, 0.0090 mmol, 11%).
MS (ESI) m/z 603 (M+H)⁺
¹H NMR (400 MHz, DMSO-d₆) δ 9.38 (s, 1H), 8.43 (t, J = 6.0 Hz, 1H), 8.23 (d, J = 8.2 Hz, 2H), 7.83 (d, J = 8.2 Hz, 2H), 7.77 (dd, J = 9.1, 4.1 Hz, 1H), 7.62 - 7.57 (m, 2H), 7.40 (ddd, J = 9.2, 9.1, 2.7 Hz, 1H), 7.14 (s, 1H), 6.47 (s, 1H), 4.28 (d, J = 6.0 Hz, 2H), 3.54 - 3.44 (m, 4H), 2.02 - 1.94 (m, 4H), 1.28 - 1.20 (m, 2H), 0.97 - 0.89 (m, 2H).

### Example 28: Synthesis of 1-(benzofuran-2-ylsulfonylamino)-N-[[2-pyrrolidin-1-yl-6-[4-(trifluoromethyl)phenyl]-4-pyridyl]methyl]cyclopropanecarboxamide (28)

Using benzofuran-2-ylsulfonyl chloride instead of A-1, an operation similar to Example 27 was performed to give the title compound (yield 11%).
MS (ESI) m/z 585 (M+H)⁺
¹H NMR (400 MHz, DMSO-d₆) δ 9.32 (s, 1H), 8.44 (t, J = 5.9 Hz, 1H), 8.24 (d, J = 8.1 Hz, 2H), 7.86 - 7.79 (m, 3H), 7.72 (dd, J = 8.4, 1.0 Hz, 1H), 7.63 (d, J = 0.9 Hz, 1H), 7.54 (ddd, J = 8.5, 7.2, 1.3 Hz, 1H), 7.44 - 7.39 (m, 1H), 7.15 (s, 1H), 6.48 (s, 1H), 4.28 (d, J = 5.9 Hz, 2H), 3.52 - 3.42 (m, 4H), 2.03 - 1.95 (m, 4H), 1.27 - 1.18 (m, 2H), 0.96 - 0.88 (m, 2H).

### Example 29: Synthesis of 1-[(5-fluorobenzofuran-2-yl)sulfonylmethyl-amino]-N-[[4-[5-(trifluoromethyl)-2-pyridyl]-2-pyridyl]methyl]cyclopropanecarboxamide (29)

To a solution of 1-[(5-fluorobenzofuran-2-yl)sulfonylamino]-N-[[4-[5-(trifluoromethyl)-2-pyridyl]-2-pyridyl]methyl]cyclopropanecarboxamide(4) (45 mg, 0.085 mmol) in N,N-dimethylformamide (1 mL) were added potassium carbonate (39 mg, 0.30 mmol) and methyl iodide (8.3 µL, 0.13 mmol) and the mixture was stirred at room temperature for 2 hr. The reaction mixture was concentrated under reduced pressure and water/acetonitrile (1/1, containing 0.1% trifluoroacetic acid) was added to the obtained residue. Insoluble material was collected by filtration and dried under reduced pressure to give trifluoroacetate of the title compound (17.7 mg, 0.032 mmol, 38%).
MS (ESI) m/z 549 (M+H)⁺
¹H NMR (400 MHz, DMSO-d₆) δ 9.15 - 9.12 (m, 1H), 8.68 (dd, J = 5.1, 0.9 Hz, 1H), 8.43 (m, 1H), 8.38 (t, J = 5.8 Hz, 1H), 8.32 - 8.27 (m, 1H), 8.02 - 7.98 (m, 2H), 7.75 (dd, J = 9.1, 4.2 Hz, 1H), 7.63 (d, J = 0.9 Hz, 1H), 7.56 (dd, J = 8.6, 2.7 Hz, 1H), 7.38 (ddd, J = 9.3, 9.2, 2.7 Hz, 1H), 4.48 (d, J = 5.8 Hz, 2H), 3.11 (s, 3H), 1.57 - 1.34 (m, 4H).

The property data (MS, NMR) of respective Example compounds are shown in Table 7.

**Table 7-1**

| Ex. No. | salt | MS(ESI) *m*/*z* (M+H)⁺ | NMR |
|---|---|---|---|
| 1 | - | 535 | ¹ NMR (400 MHz, DMSO-*d*₆) δ 9.53 (s, 1H), 9.22 (d, *J* = 1.3 Hz, 1H), 8.66 (t, *J* = 5.8 Hz, 1H), 8.40 (d, *J* = 8.2 Hz, 2H), 8.07 (d, *J* = 1.3 Hz, 7.97 (d, *J* = 8.2 Hz, 2H), 7.77 (dd, *J* = 9.1, 4.0 Hz, 1H), 7.67 (d, *J* = 0.9 Hz, 7.60 (dd, *J* = 8.5, 2.7 Hz, 1H), 7.39 (ddd, *J* = 9.2, 9.1, 2.7 Hz, 1H), 4.44 (d, *J* = 5.8 Hz, 2H), 1.30 -1.25 (m, 2H), 0.29 - 0.93 (m, 2H). |
| 2 | TFA | 536 | ¹H NMR (400 MHz, DMSO-*d*₆) δ 9.65 (s, 2H), 9.43 (s, 2H), 9.43 (s, 1H), 7.71 (dd, *J* = 5.1, 0.8 Hz, 1H), 8.63 (t, *J* = 6.1 Hz, 1H), 8.12 (dd, *J* = 1.6, 0.8 Hz, 1H), 7.78 (dd, *J* =9.1, 4.1 Hz, 1H), 7.64 - 7.59 (m, 2H), 7.44 - 7.37 (m, 2H), 4.42 (d, *J* = 6.1 Hz, 2H), 1.27 - 1.23 (m, 2H), 0.96 - 0.92 m(, 2H). |
| 3 | - | 550 | ¹ NMR (400 MHz, DMSO-*d*₆) δ 9.44 (s, 1H), 8.66 (t, *J* = 6.0 Hz, 1H), 8.62 (d, *J* = 5.3 Hz, 1H), 8.23 (d, *J* = 8.2 Hz, 1H), 8.18 (s, 1H), 7.78 (dd, *J* = 9.2, 4.0 Hz, 1H), 7.64 (d, *J* = 0.9 Hz, 1H), 7.61 (dd, *J* = 8.5, 2.7 Hz, 1H), 7.44 - 7.37 (m, 2H), 7.30 - 7.23 (m, 2H), 4.45 (d, *J* = 6.0 Hz, 2H), 1.27 -1.23 (m, 2H), 0.96- 0.92 (m, 2H). |
| 4 | TFA | 535 | ¹ NMR (400 MHz, DMSO-*d*₆) δ 9.46 (s, 1H), 9.17 - 9.14 (m, 1H), d, *J* = 5.2 Hz, 1H, 8.49 - 8.44 (m, 2H), 8.32 (d, *J* = 8.4 Hz, 1H, 8.06 (dd, *J* = 5.2, 1.7 Hz, 1H), 8.02 (d, *J* = 1.7 Hz, 1H), 7.73 (dd, *J* = 9.2, 4.1 Hz, 1H), 7.61 (d, *J* =0.9 Hz, 1H), 7.61 (d, *J* =0.9 Hz, 1H), 7.56 (dd, *J* =8.5, 2.7 Hz, 1H), 7.36 (ddd, *J* =9.2, 9.1,2.7 Hz, 1H), 4.40 (d, *J* = 5.6 Hz, 2H), 1.30 -1.25 (m, 2H), 1.02 -0.97 (m, 2H). |
| 5 | TFA | 536 | ¹ NMR (400 MHz, DMSO-*d*₆) δ 9.51 - 9.46 (m, 3H), 8.73 (d, *J* = 5.1 Hz, 1H), 8.53 (t, *J* =5.8 Hz, 1H), 7.88 (dd, *J* = 5.1, 1.8 Hz, 1H), 7.85 (d, *J* = 1.8 Hz, 1H), 7.74 (dd, *J* = 9.1, 4.0 Hz, 1H), 7.61 - 7.56 (m, 2H), 7.38 (ddd, *J* = 9.2, 2.8 Hz, 1H, 4.44 (d, *J* = 5.8 Hz, 2H), 1.30 - 1.25 (m, 2H, 1.00 - 0.95 (m, 2H). |
| 6 | TFA | 36 | ¹ NMR (400 MHz, DMSO-*d*₆) δ 9.46 (d, *J* = 0.9 Hz, 2H), 9.41 (s, 1H), 8.72 (dd, *J* = 5.1, 0.9 Hz, 1H), 8.34 (*t*, *J* = 5.6 Hz, 1H), 8.17 (dd, *J* = 5.1, 1.6 Hz, 1H), 8.12 (dd, *J* = 1.6, 0.9 Hz, 1H), 7.69 (dd, *J* = 9.0, 4.1 Hz, 1H), 7.56 (d, *J* = 0.9 Hz, 1H), 7.51 (dd, *J* = 8.5, 2.7 Hz, 1H), 7.33 (ddd, *J* = 9.2, 9.0, 2.7 Hz, 1H), 4.34 (d, *J* = 5.6 Hz, 2H), 1.30 - 1.26 (m, 2H), 1.08 -1.04 (m, 2H) |
| 7 | TFA | 36 | ¹H NMR (400 MHz, DMSO-*d*₆) δ 9.52 (d, *J* = 1.4 Hz, 1H), 9.47 (s, 1H), d, *J* =1.4 Hz, 1H), 7.74 (dd, *J* = 5.2, 0.8 Hz, 1H), 8.47 (t, *J* = 5.6 Hz, 1H), 8.07 (dd, *J* = 5.2, 1.7 Hz, 1H), 8.04 (dd, *J* = 1.7, 0.8 Hz, 1H), 7.72 (dd, *J* = 9.3, 4.2 Hz, 1H), 7.58 (d, *J* = 0.9 Hz, 1H), 7.56 (dd, *J* = 8.5, 2.7 Hz, 1H), 7.36 (ddd, *J* = 9.3, 9.2, 2.7 Hz, 1H), 4.41 (d, *J* = 5.6 Hz, 2H), 1.29 - 1.25 (m, 2H), 1.03 - 0.99 (m, 2H). |
| 8 | TFA | 536 | ¹NMR (400 MHz, DMSO-*d*₆) δ 9.47 (s, 1H), 8.77 (d, *J* = 5.2 Hz, 1H), 8.62 (d, *J* = 8.9 Hz, 1H), 8.55 - 8.48 (m, 2H), 8.14 (dd, *J* = 5.2, 1.7 Hz, 1H), 8.10 (d, *J* = 1.7 Hz, 1H), 7.73 (dd, *J* = 9.1, 4.0 Hz, 1H), 7.62 (d, *J* = 0.8 Hz, 1H), 7.56 (dd, *J* = 8.5, 2.7 Hz, 1H), 7.36 (ddd, *J* = 9.2, 9.1, 2.7 Hz, 1H), 4.44 (d, *J* = 5.7 Hz, 2H), 1.30 - 1.26 (m, 2H), 1.01 -0.97 (m, 2H). |
| 9 | TFA | 535 | ¹H NMR (400 MHz, DMSO-*d*₆) δ 9.37 (s, 1H), 9.16 (d, *J* = 2.2 Hz, 1H, 8.97 (d, *J* = 2.2 Hz, 1H), 8.62 (d, *J* = 2.2 Hz, 1H), 8.58 (t, *J* = 6.0 Hz, 1H), 8.45 (dd, *J* = 8.2, 2.2 Hz, 1H), 8.20 (dd, *J* = 2.2, 2.2 Hz, 1H), 8.09 (d, *J* = 8.2 Hz, 1H), 7.77 (dd, *J* = 9.2, 3.9 Hz, 7.64 - 7.56 (m, 2H), 7.40 (ddd, *J* =9.2, 2.7 Hz, 1H), 4.41 (d, *J* = 6.0 Hz, 2H), 1.26 - 1.21 (m, 2H), 0.93 - 089 (m, 2H). |
| 10 | TFA | 535 | ¹H NMR(400 MHz, DMSO-*d*₆) δ 9.35 (s, 1H), 9.25 (d, *J* = 2.1 Hz, 1H), 9.14 - 9.11 (m, 1H), 8.63 (d, *J* = 2.1 Hz, 1H, 8.56 (t, *J* =5.9 Hz, 1H), 8.50 (dd, *J* = 2.1, 2.1 Hz, 1H), 8.41 (dd, *J* = 8.6, 2.6 Hz, 1H), 8.30 (d, *J* = 8.6 Hz, 1H), 7.76 (dd, *J* = 9.1, 4.1 Hz, 1H), 7.61 - 7.55 (m, 2H), 7.39 (ddd, *J* = 9.2, 9.1, 2.7 Hz, 1H), 4.39 (d, *J* = 5.9 Hz, 2H), 1.25 - 1.21 (m, 2H), 0.95 - 0.91 (m, 2H). |

∘

**[Table 7-2]**

| ex. No. | salt | MS(ESI) *m*/*z* (M+H)⁺ | NMR |
|---|---|---|---|
| 11 | - | 551 | ¹H NMR (400 MHz, DMSO-*d*₆) δ 9.53 (s, 1H), 9.17 (d, *J* = 1.3 Hz, 1H), 8.64 (t, *J* = 5.9 Hz, 1H), 8.35 - 8.29 (m, 2H), 7.99 (d, *J* = 1.3 Hz, 1H), 7.76 (dd, *J* = 9.1, 4.0 Hz, 1H), 7.65 (d, *J* = 0.9 Hz, 1H), 7.62 - 7.57 (m, 3H), 7.39 (ddd, *J* = 9.2, 9.1, 2.8 Hz, 1H), 4.41 (d, *J* = 5.9 Hz, 2H), 1.29 - 1.25 (m, 2H), 0.97 - 0.93 (m, 2H) |
| 12 | - | 536 | ¹NMR (400 MHz, DMSO-*d*₆) δ 9.53 (s, 1H), 9.49 (d, *J* = 1.8 Hz, 1H), 9.27 (d, *J* = 1.3 Hz, 1H), 8.79 (dd, *J* = 8.2, 1.8 Hz, 1H), 8.68 (t, *J* = 5.9 Hz, 1H), 8.17 (d, *J* = 8.2 Hz, 8.15 (d, *J* = 1.3 Hz, 1H), 7.77 (dd, *J* = 8.8, 4.2 Hz, 1H), 7.64 (d, *J* = 0.9 Hz, 1H), 7.59 (dd, *J* = 8.5, 2.7 Hz, 1H), 7.39 (ddd, *J* = 9.2, 8.8, 2.7 Hz, 1H), 4.45 (d, *J* = 5.9 Hz, 2H), 1.29 - 1.24 (m, 2H), 0.98 - 0.93 (m, 2H). |
| 13 | - | 536 | ¹H NMR (400 MHz, DMSO-*d*₆) δ 9.44 (s, 1H), 9.24 (d, *J* = 1.3 Hz, 1H), 9.19 - 916 (m, 1H), 8.63 (d, *J* = 8.3 Hz, 1H), 8.52 - 8.44 (m, 2H), 8.23 (d, *J* = 1.3 Hz, 1H), 7.72 (dd, *J* = 8.9, 4.0 Hz, 1H), 7.58 (d, *J* = 0.9 Hz, 1H), 7.51 (dd, *J* = 8.5, 2.7 Hz, 1H), 7.34 (ddd, *J* = 9.2, 8.9, 2.7 Hz, 1H), 4.36 (d, *J* = 5.7 Hz, 2H), 1.29 - 1.25 (m, 2H), 1.07 - 1.03 (m, 2H). |
| 14 | - | 537 | ¹H NMR (400 MHz, DMSO-*d*₆) δ 9.70 (s, 2H), 9.53 (s, 1H), 9.32 (d, *J* = 1.3 Hz, 1H), 8.68 (t, *J* = 5.8 Hz, 1H), 8.20 (d, *J* = 1.3 Hz, 1H), 7.77 (dd, *J* = 9.1, 4.1 Hz, 1H), 7.62 (d, *J* = 0.9 Hz, 1H), 7.60 (dd, *J* = 8.5, 2.8 Hz, 1H), 7.39 (ddd, *J* = 9.2, 9.1, 2.8 Hz, 1H), 4.46 (d, *J* = 5.8 Hz, 2H), 1.30 - 1.26 (m, 2H), 0.99 - 0.94 (m, 2H). |
| 15 | TFA | 535 | ¹H NMR (400 MHz, DMSO-*d*₆) δ 9.44 (s, 1H), 9.20 (d, *J* = *J* = 1.9 Hz, 1H), 8.71 (t,*J* = 6.0 Hz, 1H), 8.37 (d, *J* = 8.2 Hz, 2H), 8.17 (d, *J* = 1.9 Hz, 1H), 7.97 (d, *J* = 8.2 Hz, 2H), 7.78 (dd, *J* = 9.0, 4.0 Hz, 1H), 7.64 (d, *J* = 0.9 Hz, 1H), 7.60 (dd, *J* = 8.5, 2.7 Hz, 1H), 7.41 (ddd, *J* = 9.2, 9.0, 2.7 Hz, 1H), 4.45 (d, *J* = 6.0 Hz, 2H), 1.26 - 1.22 (m, 2H), 0.89 (m, 2H). |
| 16 | TFA | 536 | ¹H NMR (400 MHz, DMSO-*d*₆) δ 9.48 (d, *J* = 2.2 Hz, 1Hz, 1H), 9.45 (s, 1H), 9.25 (d, *J* = 2.0 Hz, 1H), 8.79 (dd, *J* = 8.2, 2.2 Hz, 1H), 8.73 (t, *J* = 6.0 Hz, 1H), 8.26 (d, *J* = 2.0 Hz, 1H), 8.16 (d, *J* = 8.2 Hz, 1H), 7.78 (dd, *J* = 9.2, 4.0 Hz, 1H), 7.63 (d, *J* = 0.8 Hz, 1H), 7.60 (dd, *J* = 8.5, 2.8 Hz, 1H), 7.41 (ddd, *J* = 9.2, 9.0, 2.8 Hz, 1H), 4.47 (d, *J* = 6.0 Hz, 2H), 1.27 - 1.23 (m, 2H), 0.93 - 0.90 (m, 2H) |
| 17 | TFA | 536 | ¹H NMR (400, MHz, DMSO-*d*₆) δ 9.85 (d, *J* = 2.1 Hz, 1H), 9.46 (s, 1H), 9.22 - 9.19 (m, 1H), 8.65 (t, *J* = 5.8 Hz, 1H), 8.52 (dd, *J* = 8.4, 2.3 Hz, 1H), 8.42 (d, *J* = 8.4 Hz, 1H), 8.19 (d, *J* = 2.1 Hz, 1H), 7.72 (dd, *J* = 8.7, 4.0 Hz, 1H), 7.60 (d, *J* = 0.9 Hz, 1H), 7.55 (dd, *J* = 8.5, 2.7 Hz, 1H), 7.36 (ddd, *J* = 9.2, 8.7, 2.7 Hz, 1H), 4.61 (d, *J* = 5.8 Hz, 2H), 1.29 -1.25 (m, 2H), 1.01 - 0.97 (m, 2H). |
| 18 | - | 550 | ¹H NMR (400 MHz, DMSO-*d*₆) δ 29 (s, 1H), 9.09 - 9.04 (m, 1H), 7.40(dd, *J* = 8.8, 2.4 Hz, 1H), 8.34 (d, *J* = 8.8 Hz, 1H), 8.18 (t, *J* = 5.9 Hz, 1H), 7.90 (d, *J* = 2.1 Hz, 1H, 7.75 (dd, *J* = 9.1, 4.0 Hz, 1H), 7.61 - 7.56 (m, 2H), 7.38 (dd, *J* = 9.2, 9.1, 2.8 Hz, 1H), 7.21 (dd, *J* = 8.4, 2.1 Hz, 1H), 6.92 (d, *J* = 8.4 Hz, 1H), 4.18 (d, *J* = 5.9 Hz, 2H), 1.27 - 1.21 (m, 2H), 0.98 - 0.93 (m, 2H). |
| 19 | - | 578 | ¹H NMR (400 MHz, DMSO-*d*₆) δ 9.51 (s, 1H), 8.42 (t, *J* = 5.5 Hz, 1H), 8.33 (d, *J* = 8.2 Hz, 2H), 7.90 (d, *J* = 8.2 Hz, 2H), 7.73 (dd, *J* = 9.3, 4.0 Hz, 1H), 7.63 (d, *J* = 0.9 Hz, 1H), 7.58 (dd, *J* = 8.4, 2.8 Hz, 1H), 7.37 (ddd, *J* = 9.3, 9.2, 2.8 Hz, 1H), 7.20 (s, 1H), 4.25 (d, *J* = 5.5 Hz, 2H), 3.24 (s, 6H), 1.29 - 1.24 (m, 2H), 1.00 - 0.94 (m, 2H). |
| 20 | - | 551 | ¹H NMR (400 MHz, DMSO-*d*₆) δ 13.07 (s,1H), 9.49 (s, 1H), 9.21 (d, *J* = 1.3 Hz, 1H), 8.50 (t, *J* = 5.8 Hz, 1H), 8.25 (d, *J* = 7.6 Hz, 1H), 8.12 (d, *J* = 1.3 Hz, 1H), 7.76 (dd, *J* = 9.0, 4.2 Hz, 1H), 7.63 (d, *J* = 0.9 Hz, 1H), 7.58 (dd, *J* = 8.4, 2.7 Hz, 1H), 7.42 - 7.32 (m, 3H), 4.40 (d, *J* = 5.8 Hz, 2H), 1.29 - 1.25 (m, 2H), 1.00 - 0.94 (m, 2H). |

**Table 7-3**

| ex. No. | salt | MS(ESI) *m*/*z* (M+H)⁺ | NMR |
|---|---|---|---|
| 21 | - | 517 | ¹H NMR (400 MHz, DMSO-*d*₆) δ 9.46 (s, 1H), 9.22 (d, *J* = 1.2 Hz, 1H), 8.68 (t, *J* = 5.9 Hz, 1H), 8.41 (d, *J* = 8.2 Hz, 2H), 8.08 (d, *J* = 1.2 Hz, 1H), 7.98 (d, *J* = 8.2 Hz, 2H), 7.81 (dd, *J* = 8.0, 1.2 Hz, 1H), 7.73 - 7.69 (m, 2H), 7.53 (ddd, *J* = 8.6, 7.3, 1.4 Hz, 1H), 7.43 - 7.38 (m, 1H), 4.44 (d, *J* = 5.9 Hz, 2H), 1.28 - 1.22 (m, 2H), 0.97 - 0.90 (m, 2H). |
| 22 | TFA | 517 | ¹H NMR (400 MHz, DMSO-*d*₆) δ 9.40 (s, 1H), 9.18 -9.14 (m, 1H), 8.72 (d, *J* = 5.2 Hz, 1H), 8.51 (t, *J* = 5.7 Hz, 1H), 8.47 (dd, *J* = 8.5, 2.4 Hz, 1H), 8.34 (*d*, *J* = 8.5 Hz, 1H), 8.10 - 8.04 (m 2H), 7.78 (dd, *J* = 7.9, 1.2 Hz, 1H, 7.68 (dd, *J* = 8.4, 0.9 Hz, 1H), 7.64 (d, *J* = 0.9 Hz, 1H), 7.51 (ddd, *J* = 8.4, 7.2, 1.2 Hz, 1H), 7.41 - 7.35 (m, 1H), 4.42 (d, *J* = 5.7 Hz, 2H), 1.29 - 1.23 (m, 2H), 1.00 - 0.95 (m, 2H). |
| 23 | - | 560 | ¹ NMR (400 MHz, DMSO-*d*₆) δ 9.45 (s, 1H), 8.46 (t, *J* = 5.6 Hz, 1H), 8.34 (d, *J* = 8.1 Hz, 2H), 7.91 (d, *J* = 8.1 Hz, 2H), 7.82 - 7.78 (m, 1H), 7.69 (dd, *J* = 8.4, 1.0 Hz, 1H), 7.66 (d, *J* = 0.9 Hz, 1H), 7.52 (ddd, *J* = 8.4, 7.2, 1.3 Hz, 1H), 7.42 - 7.36 (m, 1H), 7.22 (s, 1H), 4.26 (d, *J* = 5.6 Hz, 2H), 3.24 (s, 6H), 1.28 - 1.22 (m, 2H), 0.98 -0.92 (m, 2H). |
| 24 | - | 578 | ¹ NMR (400 MHz, DMSO-*d*₆) δ 9.46 (*d J* = 2.1 Hz, 1H), 9.28 (d, *J* = 1.3 Hz, 1H), 8.77 (dd, *J* = 8.4, 2.1 Hz, 1H), 8.32 (t, *J* = 5.8 Hz, 1H), 8.15 (d, *J* = 8.4 Hz, 1H), 8.11 (d, *J* = 1.3 Hz, 1H), 7.78 (dd, *J* = 9.1, 4.0 Hz, 1H), 7.69 (d, *J* = 0.7 Hz, 1H), 7.60 (dd, *J* = 8.5, 2.7 Hz, 1H), 7.40 (ddd, *J* = 9.2, 9.1, 2.7 Hz, 1H), 4.65 - 4.38 (m, 2H), 4.30 (hept, *J* = 6.9 Hz, 1.68 - 1.48 (m, 4H), 1.47 - 1.10 (m, 6H). |
| 25 | - | 560 | ¹H NMR (400, MHz, DMSO-*d*₆) δ 9.46 (d, *J* = 2.1 Hz, 1H), 9.28 (d, J = 1.3 Hz, 1H), 8.77 (dd, J = 8.3, 2.1 Hz, 1H), 8.34 (t, J = 5.8 Hz, 1H), 8.15 (d, J = 8.3 Hz, 1H), 8.12 (d, J = 1.3 1H), 7.80 (dd, J = 7.9, 1.3 Hz, 1Hz, 1H), 7.75 - 7.69 (m, 2H), 7.54 (ddd, J = 8.5, 7.2, 1.3 Hz; 1H), 7.44 - 7.38 (m, 1H), 4.66 - 4.40 (m, 2H), 4.32 (hept, J = 6.9. Hz, 1H), 1.71 - 1.45 (m, 4H), 1.45 - 1.07 (m, 6H). |
| 26 | TFA | 518 | ¹H NMR (400 MHz, DMSO-*d*₆) δ 9.68 (s, 1H), 9.22 (d, *J* = 1.3 Hz, 1H), 9.17 (s, 1H), 8.69 (d, *J* = 6.0 Hz, 1H), 8.66 (t, *J* = 5.9 Hz, 1H), 8.40 (d, *J* = 8.2 Hz, 2H), 8.06 (d, *J* = 1.3) Hz, 1H), 7.98 (d, *J* = 8.2 Hz, 2H), 7.93 (d, *J* = 6.0 Hz, 1H), 7.86 (s, 1H), 1 4.43 (d, *J* = 5.9 Hzn 2H), 1.31 - 1.27 (m, 2H), 1.01 - 0.96 (m. 2H). |
| 27 | - | 603 | ¹ NMR (400 MHz, DMSO-*d*₆) δ 9.38 (s, 1H), 8.43 (t, *J* = 6.0 Hz, 1H), 8.23 (d, *J* = 8.2 Hz, 2H), 7.83 (d, *J* = 8.2 Hz, 2H), 7.77 (dd, *J* = 9.1, 4.1 Hz, 1H), 7.62 - 7.57 (m, 2H), 7.40 (ddd, *J* = 9.2, 9.1, 2.7 Hz, 1H), 7.14 (s, 1H), 6.47 (s, 1H), 4.28 (d, *J* = 6.0 Hz, 2H), 3.54 - 3.44 (m, 4H), 2.02 - 1.94 (m, 4H), 1.28 - 1.20 (m, 2H), 0.97 - 0.89 (m, 2H). |
| 28 | - | 585 | ¹H NMR (400 MHz, DMSO-*d*₆) δ 9.32 (s, 1H), 8.44 (t, *J* = 5.9 Hz, 1H), 8.24 (d, *J* = 8.1 Hz, 2H), 7.86 - 7.79 (m, 3H), 7.72 (dd, *J* = 8.4, 1.0 Hz, 1H), 7.63 (d, *J* = 0.9 Hz, 1H), 7.54 (ddd, *J* = 8.5, 7.2, 1.3 Hz, 1H), 7.44 - 7.39 (m, 1H), 7.15 (s, 1H), 6.48 (s, 1H), 4.28 (d, *J* = 5.9 Hz, 2H), 3.52 - 3.42 (m, 4H), 2.03 - 1.95 (m, 4H), 1.27 - 1.18 (m, 2H), 0.96 - 0.88 (m, 2H). |
| 29 | TFA | 549 | ¹H NMR (400 MHz, DMSO-*d*₆) δ 9.15 - 9.12 (m, 1H), 8.68 (dd, *J* = 5.1, 0.9 Hz, 1H), 8.43 (m, 1H) 8.38 (t, *J* = 5.8 Hz, 1H), 8.32 - 8.27 (m, 1H), 8.02 - 7.98 (m, 2H), 7.75 (dd, *J* = 9.1, 4.2 Hz, 1H), 7.63 (d, *J* = 0.9 Hz, 1H), 7.56 (dd, *J* = 8.6, 2,7 Hz, 1H), 7.38 (ddd, *J* = 9.3, 9.2, 2.7 Hz, 1H), 4.48 (d, *J* = 5.8 Hz, 2H), 3.11 (s, 3H), 1.57 - 1.34 (m, 4H). |

For reference, Comparative Examples are shown.

### Comparative Example 1: Synthesis of 1-[(4-fluorophenyl)sulfonyl-isopropyl-amino]-N-[[6-[6-(trifluoromethyl)-3-pyridyl]pyrimidin-4-yl]methyl]cyclopropanecarboxamide

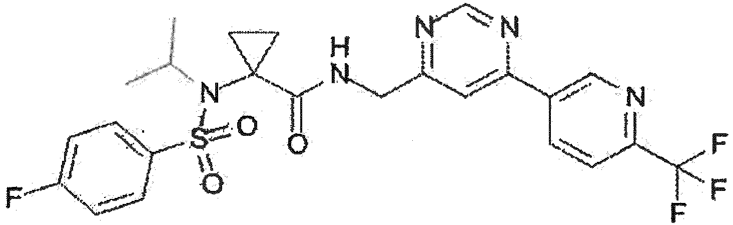

According to the method described in Example 84 of patent document 3 (WO 2014/049047), the title compound was synthesized. MS (ESI) m/z 538 (M+H)⁺

### Comparative Example 2: Synthesis of 1-[(4-fluorophenyl)sulfonylamino]-N-[[2-pyrrolidin-1-yl-6-[4-(trifluoromethyl)phenyl]-4-pyridyl]methyl]cyclopropanecarboxamide

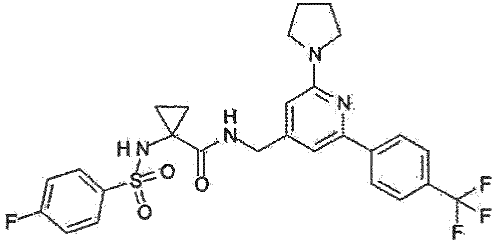

Using 4-fluorobenzenesulfonyl chloride instead of A-1, an operation similar to Example 27 was performed to give the title compound (yield 46%).
MS (ESI) m/z 563 (M+H)⁺
¹H NMR (400 MHz, DMSO-d₆) δ 8.73 (s, 1H), 8.45 (t, J = 6.0 Hz, 1H), 8.25 (d, J = 8.1 Hz, 2H), 7.90 - 7.80 (m, 4H), 7.49 - 7.40 (m, 2H), 7.18 (s, 1H), 6.52 (s, 1H), 4.31 (d, J = 6.0 Hz, 2H), 3.55 - 3.50 (m, 4H), 2.03 - 1.94 (m, 4H), 1.16 - 1.08 (m, 2H), 0.75 - 0.67 (m, 2H).

### Comparative Example 3: Synthesis of 1-(2-furylsulfonylamino)-N-[[2-pyrrolidin-1-yl-6-[4-(trifluoromethyl)phenyl]-4-pyridyl]methyl]cyclopropanecarboxamide

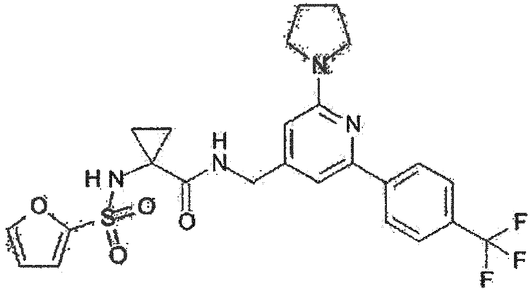

Using 2-furansulfonyl chloride instead of A-1, an operation similar to Example 27 was performed to give the title compound (yield 16%).
MS (ESI) m/z 535 (M+H)⁺
¹H NMR (400 MHz, DMSO-d₆) δ 9.02 (s, 1H), 8.42 (t, J = 6.1 Hz, 1H), 8.23 (d, J = 8.1 Hz, 2H), 7.99 (dd, J = 1.8, 0.9 Hz, 1H), 7.83 (d, J = 8.1 Hz, 2H), 7.16 (s, 1H), 7.14 (dd, J = 3.4, 0.9 Hz, 1H), 6.70 (dd, J = 3.4, 1.8 Hz, 1H), 6.52 (s, 1H), 4.33 (d, J = 6.1 Hz, 2H), 3.54 - 3.44 (m, 4H), 2.02 - 1.95 (m, 4H), 1.22 - 1.14 (m, 2H), 0.83 - 0.75 (m, 2H).

### Comparative Example 4: Synthesis of 1-[(5-chloro-2-thienyl)sulfonylamino]-N-[[2-pyrrolidin-1-yl-6-[4-(trifluoromethyl)phenyl]-4-pyridyl]methyl]cyclopropanecarboxamide

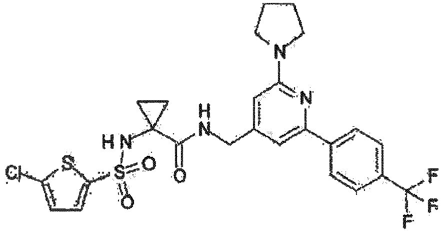

Using 5-chlorothiophene-2-sulfonyl chloride instead of A-1, an operation similar to Example 27 was performed to give the title compound (yield 16%).
MS (ESI) m/z 585 (M+H)⁺
¹H NMR (400 MHz, DMSO-d₆) δ 9.00 (s, 1H), 8.43 (t, J = 6.0 Hz, 1H), 8.23 (d, J = 8.1 Hz, 2H), 7.82 (d, J = 8.1 Hz, 2H), 7.49 (d, J = 4.1 Hz, 1H), 7.25 (d, J = 4.1 Hz, 1H), 7.15 (s, 1H), 6.50 (s, 1H), 4.31 (d, J = 6.0 Hz, 2H), 3.51 - 3.42 (m, 4H), 2.01 - 1.96 (m, 4H), 1.23 - 1.17 (m, 2H), 0.93 - 0.87 (m, 2H).

### Experimental Example 1: Measurement of TRPA1 antagonist activity

### human TRPA1 expression plasmid

Using cDNA encoding human TRPA1 (GenBank accession No. NM_0078332) (manufactured by Kazusa DNA Research Institute, item No.: FHC07217) as a template, primer 1 (SEQ ID NO: 1) and primer 2 (SEQ ID NO: 2), PCR by PfuUltra High-Fidelity DNA Polymerase (Stratagene) was performed, and full-length human TRPA1 gene was amplified.

**primer 1 :5' -AAGTTTAGTAAGCTTCGATCGCCATGAAG-3' (SEQ ID NO: 1)**
**primer 2 : 5'-GTACCGATCTAGAATTCGTTTACTAAGGCTCAAG-3(SEQ ID NO: 2)**

A recognition site (underlined) of restriction enzyme HindIII was added to the 5' end, and XbaI site (underlined) was added to the 3' end, and GTT of the template sequence was changed to termination codon TAG (bold). The obtained double stranded DNA was enzyme-digested with HindIII and XbaI, and introduced into a multicloning site of expression plasmid pcDNA3.1/zeo(+) (manufactured by Invitrogen) to give a human TRPA1 expression plasmid.

### Cell preparation

Human embryonic kidney-derived 293T cells were cultured in Dulbecco's Modified Eagle Medium containing 10% fetal bovine serum, 10 unit penicillin, and 10 µg streptomycin. One day before assay, 3×10⁶ of 293T cells were plated on a petri dish having a diameter of 10 cm, and cultured in a CO₂ incubator for 24 hr. OPTI-MEM I Reduced Serum Media (Invitrogen) (600 µL), Mirus TransIT-293 (Mirus Bio) (18 µL), and human TRPA1 expression plasmid (6 µg) were mixed, the total amount of the mixture was added to the cells on the petri dish to allow for gene transfer. The cells were recovered about for 8 hr later, plated on a poly-D-lysine coated 384 well black/clear bottom plate at 12,000 cells/well, and cultured overnight.

### Measurement of intracellular calcium increase

The medium was removed from the 384 well plate, calcium indicator (Molecular Device, trade name: FLIPR Calcium4 Assay Kit) dissolved in HBSS (Thermo Fisher Scientific) (pH 7.2) containing 20 mM HEPES was added (38 µL/well), and the cells were stained in a CO₂ incubator for 1 hr. The 384 well plate was stood at room temperature for not less than 15 min, set on FDSS7000 (Hamamatsu Photonics K.K.), and a test substance solution was added at 10 µL/well. After 10 min, allylisothiocyanate solution (12 µL/well) was added, the relative fluorescence intensity was measured for 5 min after addition of the allylisothiocyanate solution.

### Preparation of test substance solution and allylisothiocyanate solution

A test substance solution was prepared to have a composition of HBSS (Thermo Fisher Scientific) (pH 7.2) containing 0.48% dimethyl sulfoxide, a test substance at 4.8-fold concentration of the evaluation concentration, 0.1% bovine serum albumin and 20 mM HEPES. An allylisothiocyanate solution was prepared to have a composition of HBSS (Thermo Fisher Scientific) (pH 7.2) containing 0.1% dimethyl sulfoxide, 100 µM allylisothiocyanate, 0.1% bovine serum albumin and 20 mM HEPES.

### Calculation of antagonist activity

The activity rate of a test substance at each concentration was calculated, wherein the relative fluorescence intensity change of a well free of a test substance and containing allylisothiocyanate is 100% activity rate, and the relative fluorescence intensity change of a well free of a test substance and allylisothiocyanate is 0% activity rate. The inhibitory rate of a test substance at each concentration was calculated by subtracting the activity rate of the test substance from 100% activity rate, and the concentration of a test substance showing 50% inhibitory rate was calculated as IC50 from the sigmoid approximate curve by XLFit (idbs).

The results are shown in Table 8. The activity values of Examples are shown in Table 8-1 and, for reference, the activity values of Comparative Examples are shown in Table 8-2. As shown therein, the compound of the present invention showed a superior TRPA1 antagonist activity.

**[Table 8-1]**

| Ex. No | hTRPA1 IC50 (µM) | Ex. No. | hTRPA1 IC50 (µM) | Ex. No | hTRPA1 IC50 (µM |
|---|---|---|---|---|---|
| 1 | 0.033 | 11 | 0.040 | 21 | 0.077 |
| 2 | 0.12 | 12 | 0.030 | 22 | 0.13 |
| 3 | 0.072 | 13 | 0.11 | 23 | 0.10 |
| 4 | 0.022 | 14 | 0.047 | 24 | 0.0033 |
| 5 | 0.26 | 15 | 0.31 | 25 | 0.0081 |
| 6 | 0.23 | 16 | 0.99 | 26 | 0.045 |
| 7 | 1.17 | 17 | 0.24 | 27 | 0.086 |
| 8 | 0.074 | 18 | 0.026 | 28 | 0.26 |
| 9 | 0.17 | 19 | 0.038 | 29 | 0.0012 |
| 10 | 0.11 | 20 | 0.023 | | |

**[Table 8-2]**

| **Comp. Ex. No.** | **hTRPA1 IC50 (µM)** |
|---|---|
| **1** | 0.18 |
| **2** | >1 |
| **3** | >1 |
| **4** | >1 |

### Experimental Example 2: AITC-induced pain behavior evaluation test

To evaluate the effectiveness of the test substance in vivo, allylisothiocyanate (AITC)-induced pain behavior evaluation test was performed using mice.

AITC is a selective agonist of the TRPA1 channel, and causes a pain behavior by TRPA1 activation when administered to animal. Therefore, the intensity of the TRPA1 antagonist action of the test substance in the living body can be evaluated by measuring the pain behavior after AITC administration.

### 1. Administration of test substance to animal

As the animal, male ICR mice (6- to 8-week-old) are used. The mice are fasted on the previous day of the test. The test substance is intraperitoneally or orally administered for evaluation. In the case of intraperitoneal administration, the substance is administered 30 min before the AITC administration. In the case of oral administration, the substance is administered 60 min before the AITC administration.

### 2. AITC-induced pain behavior evaluation

AITC (0.1%) is subcutaneously administered to the sole of the left leg of mouse, and the time when the mouse shows a behavior of licking the sole of the leg (Licking time) in 5 min immediately after the AITC administration is measured.

### 3. Calculation of inhibitory rate

The licking time of the vehicle administration group in each test is taken as 100%, and the activity rate by administration of each test substance (Licking time of test substance administration/Licking time of vehicle administration group x 100) is determined, and the numerical value obtained by subtracting the activity rate from 100 is calculated as an inhibitory rate.

By the above-mentioned method, it can be confirmed that the compound of the present invention has a superior TRPA1 antagonist activity, is superior pharmacokinetics, and shows superior efficacy in animal model.

The above-mentioned evaluation tests confirm the effectiveness of the compound of the present invention.

### [Sequence Listing Free Text]

SEQ ID NO: 1: primer
SEQ ID NO: 2: primer

### [Industrial Applicability]

The compound of the present invention has a superior TRPA1 antagonist activity, and therefore, is utilizable for the prophylaxis/or treatment of diseases involving TRPA1 (e.g., pain associated diseases, digestive tract diseases, lung diseases, bladder diseases, inflammatory diseases, dermatic diseases, and neurological diseases).

In view of this object, the compound of the present invention shows a certain level of blood concentration or bioavailability by oral administration, shows sustainability of the blood concentration, and is possibly utilizable as an oral preparation.

In addition, the compound of the present invention shows a certain level of stability in acidic or alkaline solutions and can be applied to various dosage forms.

Furthermore, the compound of the present invention specifically inhibits TRPA1. That is, the compound of the present invention has high selectivity to molecule targets, is free of a fear of interactions with drugs, is superior in safety and is useful.

This application is based on a patent application No. 2015-150058 filed in Japan (filing date: July 29, 2015), the contents of which are incorporated in full herein.

## Claims

1. A compound represented by the formula (I): 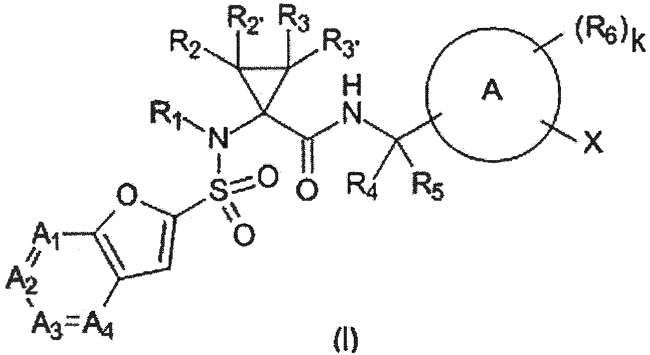 wherein
ring A is a 5-membered or 6-membered monocyclic aromatic ring or heteroaromatic ring, or bicyclic aromatic ring or heteroaromatic ring;
A₁ is -C(Ra)= or -N=;
A₂ is -C(Rb)= or -N=;
A₃ is -C(Rc)= or -N=;
A₄ is -C(Rd)= or -N=;
Ra, Rb, Rc and Rd are the same or different and each is hydrogen, a halogeno group, a cyano group, a hydroxy group, a C₁₋₆ alkyl group, a C₁₋₆ alkoxy group, a halogeno C₁₋₆ alkyl group or a halogeno C₁₋₆ alkoxy group;
at least two of A₁ - A₄ are not -N=;
R₁ is hydrogen or a C₁₋₆ alkyl group optionally having substituent(s) ;
R₂, R₂', R₃ and R₃' are the same or different and each is hydrogen or a C₁₋₆ alkyl group optionally having substituent(s);
R₄ is hydrogen or a C₁₋₆ alkyl group;
R₅ is hydrogen or a C₁₋₆ alkyl group;
R₄ and R₅ are optionally joined to form cycloalkane;
X is hydrogen,
-Cy,
-C(Rₓ₁Rₓ₂)-Cy,
-C(Rₓ₁Rₓ₂)-C(Rₓ₃Rₓ₄)-Cy,
-C(Rₓ₁)=C(Rₓ₂)-Cy,
-O-Cy,
-O-C(Rₓ₁Rₓ₂)-Cy,
-C(Rₓ₁Rₓ₂)-O-Cy,
-S(O)n-Cy,
-S(O)n-C(Rₓ₁Rₓ₂)-Cy,
-C(Rₓ₁Rₓ₂)-S(O)n-Cy,
-N(Rₓ₅)-Cy,
-N(Rₓ₅)-C(Rₓ₁Rₓ₂)-Cy,
-C(Rₓ₁Rₓ₂)-N(Rₓ₅)-Cy,
-N (Rₓ₅)-N(Rₓ₆)-Cy,
-O-N(Rₓ₅)-Cy,
-N(Rₓ₅)-O-Cy,
-C(O)-N(Rₓ₅)-Cy,
-N(Rₓ₅)-C(O)-Cy,
-S(O)m-N(Rₓ₅)-Cy,
-N(Rₓ₅)-S(O)m-Cy,
-O-S(O)m-Cy, or
-S(O)m-O-Cy;
n is an integer of 0 to 2;
m is 1 or 2;
Cy is a saturated or unsaturated cyclic group optionally having substituent(s) (optionally including heteroatom(s));
Rₓ₁, Rₓ₂, Rₓ₃, Rₓ₄, Rₓ₅ and Rₓ₆ are the same or different and each is hydrogen, a C₁₋₆ alkyl group optionally having substituent(s) or a C₁₋₆ alkoxycarbonyl group optionally having substituent(s);
R₆ is a C₁₋₆ alkyl group optionally having substituent (s), a C₂₋₆ alkenyl group, a cyclic C₃₋₆ alkyl group (optionally containing heteroatom(s)), a halogeno group, a hydroxy group, a C₁₋₆ alkoxy group optionally having substituent(s), a halogeno C₁₋₆ alkyl group, a halogeno C₁₋₆ alkoxy group, an amino group, an amino group mono- or di-substituted by a C₁₋₆ alkyl group optionally having substituent(s), a cyano group, a C₁₋₆ alkylthio group, a carboxyl group, a C₁₋₆ alkoxycarbonyl group optionally having substituent(s), a carbamoyl group, a carbamoyl group mono- or di-substituted by a C₁₋₆ alkyl group optionally having substituent(s) or an amino group substituted by an acyl group optionally having substituent(s);
when R₆ is present in plurality, they may be the same or different; and
k is an integer of 0 to 3, or a pharmaceutically acceptable salt thereof.

2. The compound or pharmaceutically acceptable salt according to claim 1, wherein ring A is a 6-membered monocyclic aromatic ring or heteroaromatic ring, or bicyclic aromatic ring or heteroaromatic ring.

3. The compound or pharmaceutically acceptable salt according to claim 1 or 2, wherein R₁ is a C₁₋₆ alkyl group optionally having substituent(s).

4. The compound or pharmaceutically acceptable salt according to claim 1 or 2, wherein R₁ is hydrogen.

5. The compound or pharmaceutically acceptable salt according to any one of claims 1 to 4, wherein R₂, R₂', R₃, and R₃' are each hydrogen.

6. The compound or pharmaceutically acceptable salt according to any one of claims 1 to 5, wherein R₄ and R₅ are each hydrogen.

7. The compound or pharmaceutically acceptable salt according to any one of claims 1 to 6, wherein ring A is a 6-membered monocyclic aromatic ring or heteroaromatic ring.

8. The compound or pharmaceutically acceptable salt according to any one of claims 1 to 7, wherein ring A is benzene, pyridine or pyrimidine.

9. The compound or pharmaceutically acceptable salt according to any one of claims 1 to 8, wherein partial structure (b) containing ring A 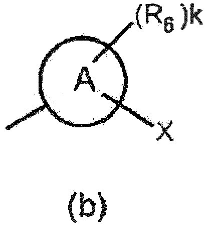 is a group of any of the following illustrations

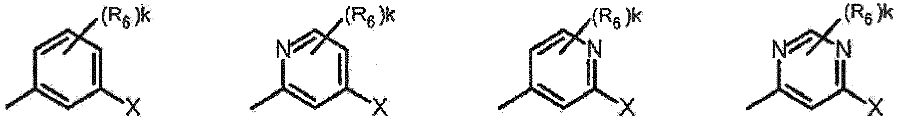

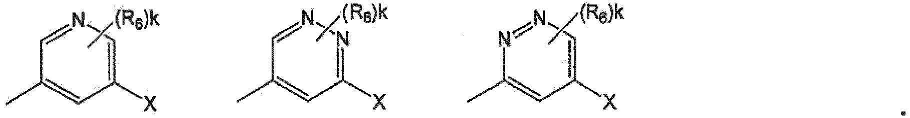

10. The compound or pharmaceutically acceptable salt according to any one of claims 1 to 9, wherein k is an integer of 0 to 2, and R₆ is a C₁₋₆ alkyl group, a cyclic C₃₋₆ alkyl group (optionally containing heteroatom(s)), a halogeno group, a hydroxy group, a C₁₋₆ alkoxy group optionally having substituent(s), an amino group, a C₁₋₆ alkoxycarbonyl group, or an amino group mono- or di-substituted by a C₁₋₆ alkyl group.

11. The compound or pharmaceutically acceptable salt according to any one of claims 1 to 9, wherein k is 0.

12. The compound or pharmaceutically acceptable salt according to any one of claims 1 to 9, wherein partial structure (b) containing ring A 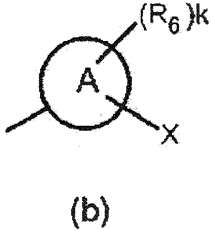 is a group of any of the following illustrations 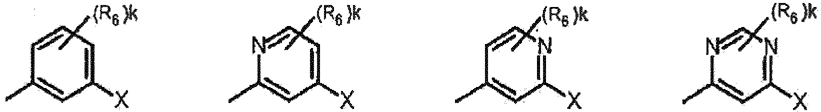 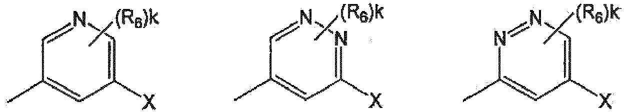 k is 0 or 1, R₆ is a cyclic C₃₋₆ alkyl group (optionally containing heteroatom(s)), a halogeno group, a C₁₋₆ alkoxycarbonyl group, an amino group, an amino group mono- or di-substituted by a C₁₋₆ alkyl group or a hydroxy group.

13. The compound or pharmaceutically acceptable salt according to any one of claims 1 to 12, wherein partial structure (b) containing ring A 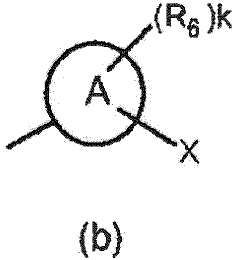 is a group of any of the following illustrations

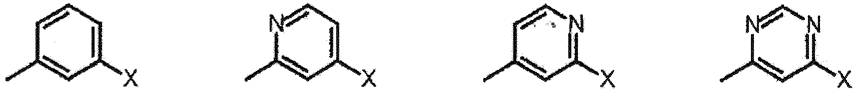

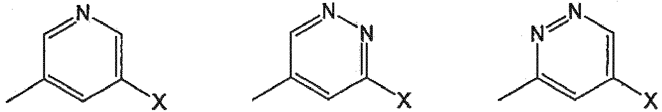

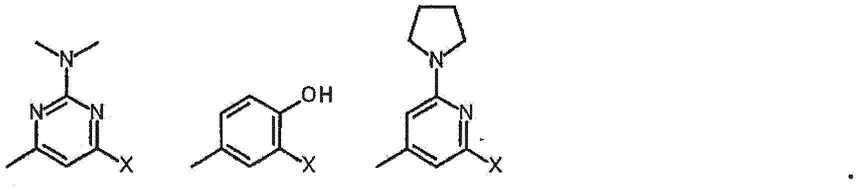

14. The compound or pharmaceutically acceptable salt according to any one of claims 1 to 13, wherein A₁ is -C(Ra)=, A₂ is - C(Rb)=, A₃ is -C(Rc)=, and A₄ is -C(Rd)=.

15. The compound or pharmaceutically acceptable salt according to any one of claims 1 to 14, wherein A₁ is -C(Ra)=, A₂ is - C(Rb)=, A₃ is -C(Rc)=, and A₄ is -C(Rd)=;
Ra, Rb, Rc, and Rd are all hydrogen or any one of them is a halogeno group.

16. The compound or pharmaceutically acceptable salt according to any one of claims 1 to 15, wherein partial structure (a) 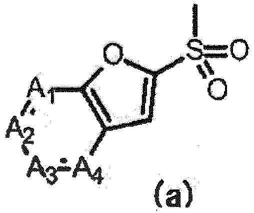 is a group of any of the following illustrations

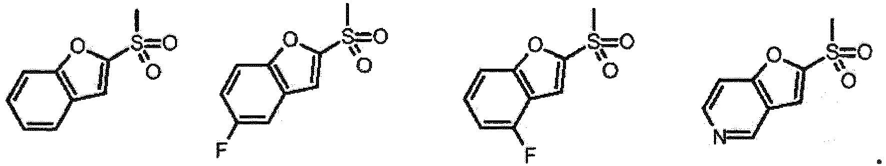

17. The compound or pharmaceutically acceptable salt according to any one of claims 1 to 16, wherein X is hydrogen, -Cy, -O-Cy or -O-CH₂-Cy.

18. The compound or pharmaceutically acceptable salt according to any one of claims 1 to 17, wherein X is -Cy.

19. The compound or pharmaceutically acceptable salt according to any one of claims 1 to 18, wherein Cy is benzene optionally having substituent(s), pyridine optionally having substituent(s), pyrimidine optionally having substituent(s), pyridazine optionally having substituent(s), or pyrazine optionally having substituent(s).

20. The compound or pharmaceutically acceptable salt according to any one of claims 1 to 19, wherein Cy is a group of any of the following illustrations

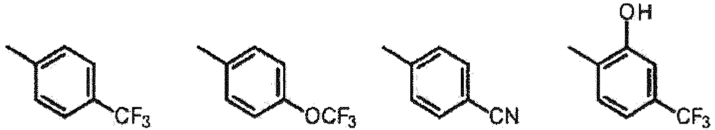

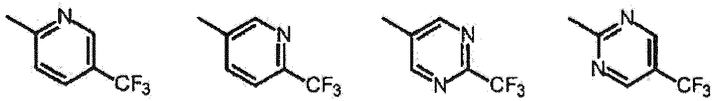

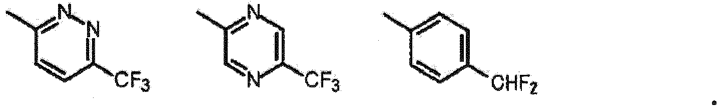

21. The compound or pharmaceutically acceptable salt according to any one of claims 1 to 20, which is a TRPA1 antagonist.

22. A medicament comprising the compound or pharmaceutically acceptable salt according to any one of claims 1 to 21 as an active ingredient.

23. The medicament according to claim 22 for the prophylaxis and/or treatment of a disease involving TRPA1.

24. The medicament according to claim 23, wherein said disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.

25. The medicament according to claim 23, wherein said disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.

26. A method for the prophylaxis and/or treatment of a disease involving TRPA1, said method comprising administering an effective amount of a compound or pharmaceutically acceptable salt according to any one of claims 1 to 20 to a subject in need thereof.

27. The method according to claim 26, wherein said disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.

28. The method according to claim 26, wherein said disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.

29. The compound or pharmaceutically acceptable salt according to any one of claims 1 to 20 for use in the prophylaxis and/or treatment of a disease involving TRPA1.

30. The compound or pharmaceutically acceptable salt according to claim 29, wherein said disease involving TRPA1 is selected from the group consisting of chronic pain, acute pain, diabetic neuropathy, osteoarthritis, asthma, chronic cough, chronic obstructive pulmonary diseases, functional gastrointestinal disorder, erosive esophagitis, irritable bowel syndrome, inflammatory bowel disease, pancreatitis, anticancer agent-induced neuropathy, pruritus, and allergic dermatitis.

31. The compound or pharmaceutically acceptable salt according to claim 29, wherein said disease involving TRPA1 is selected from the group consisting of chronic pain, acute pain, asthma, chronic obstructive pulmonary diseases, functional gastrointestinal disorder, erosive esophagitis, inflammatory bowel disease, anticancer agent-induced neuropathy, and pruritus.
